# EUROPEAN PATENT APPLICATION

(11) **EP 4 310 081 A1**
(43) Date of publication of application: **24.01.2024**
(21) Application number: 22770601.7
(22) Date of filing: 17.03.2022
(51) Int. Cl.: C07D 401/14, C07D 401/04, C07D 487/04, C07D 471/04, C07D 498/04, C07D 239/42, A61K 31/506, A61P 35/00

(54) **CTLA-4 SMALL MOLECULE DEGRADATION AGENT AND APPLICATION THEREOF**

(30) Priority: 18.03.2021 CN 202110292038; 08.03.2022 CN 202210220548
(71) Applicant: Suzhou Guokuang Pharmtech. Co., Ltd., Suzhou, Jiangsu 215125 (CN)
(72) Inventor: MEI, Desheng, Suzhou, Jiangsu 215125 (CN); HE, Baokun, Suzhou, Jiangsu 215125 (CN); LV, Shiming, Suzhou, Jiangsu 215125 (CN); SUN, Gaorui, Suzhou, Jiangsu 215125 (CN); WANG, Kui, Suzhou, Jiangsu 215125 (CN); XIAO, Cheng, Suzhou, Jiangsu 215125 (CN); LIANG, Min, Suzhou, Jiangsu 215125 (CN); LING, Xin, Suzhou, Jiangsu 215125 (CN); LIU, Shuaishuai, Suzhou, Jiangsu 215125 (CN)
(74) Representative: karo IP
(86) International application number: PCT/CN2022/081458
(87) International publication number: WO 2022/194248

(57) **Abstract**

A CTLA-4 small molecule degradation agent and an application thereof. The CTLA-4 small molecule degradation agent comprises a compound having the structure represented by formula I or a pharmaceutically acceptable salt, an ester, a deuterated product, an isomer, a solvate, a prodrug, or an isotopic label thereof: 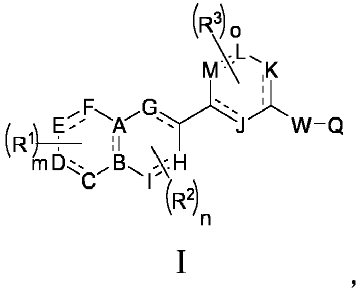 a new class of small molecule compounds having high degradation activity on CTLA-4. The compounds show a good degradation activity on CTLA-4 at the nanomolar (nM) level in in vitro studies.

## Description

### TECHNICAL FIELD

The present invention belongs to the field of pharmaceutical chemistry, and in particular, to a type of small molecule degradation agents, which can cause the degradation of CTLA-4 protein by inhibiting the interaction between LRBA protein and CTLA-4 protein, and preparation methods, pharmaceutical compositions and uses in medicine thereof.

### BACKGROUND

PD-1/PD-L1 inhibitors are currently one of the most important tumor immunity drugs, but the clinical response rate is relatively low (10-30%), and they are ineffective for most tumor patients. Thus, new clinical solutions are needed. The combined administration of PD-1/PD-L1 inhibitors with other tumor therapeutic drugs may be a better strategy.

CTLA-4 (cytotoxic T lymphocyte-associated protein-4) is a protein receptor that acts as an immune checkpoint and downregulates immune responses. It is constitutively highly expressed in T cells, especially regulatory T cells. CTLA-4 acts as an "off" switch when it binds to CD80 or CD86 on the surface of antigen-presenting cells. CTLA-4 inhibitors can activate anti-tumor immune responses and be used in tumor immunotherapy. The CTLA-4 inhibitor Ipilimumab (Yervoy, "Y" drug) has been used in the treatment of various tumors including melanoma.

Considering that CTLA-4 and PD-1/PD-L1 co-regulate the immune response at different stages of tumor immune response: CTLA-4, early and middle stage of tumor immune response; PD-1, late stage of tumor immune response. Therefore, the combined administration of CTLA-4 and PD-1/PD-L1 inhibitors is feasible, and this combined administration strategy has been fully verified in clinical trials: FDA has approved six clinical indications, including hepatocellular carcinoma and non-small cell lung cancer. For example, compared with the single "O" drug, the objective response rate (ORR) of the combination of Ipilimumab (Yervoy, "Y" drug) and PD-1 inhibitor Nivolumab (OPDIVO, "O" drug) was significantly improved. The double immunotherapy combination of Y drug and O drug is the first and the only dual immunotherapy approved by FDA.

| Tumor | ORR data |
|---|---|
| Colorectal cancer (MSI-H/dMMR) | single O drug group ORR=36% |
| | O+Y drug group ORR=54.6% |
| Non-small cell lung cancer | single O drug group ORR=14.5% |
| | O+Y drug group ORR=36% |
| Hepatocellular carcinoma | single O drug group ORR=15% |
| | O+Y drug group ORR=31% |

However, the current CTLA-4 inhibitors, such as "Y" drugs, are antibody drugs, which have some inherent limitations: 1) Serious side effects: in clinical trials, about 54% of patients with combined administration will have 3-4 grade immune-related adverse events (irAEs), which greatly limits the clinical use of such drugs, and at the same time, the side effects are related to the ADCC effect of antibodies and immunogenicity. 2) Difficult Intratumoral diffusion: as macromolecular drugs, CTLA-4 antibody drugs are difficult to infiltrate into solid tumors, which limits the therapeutic effect of single or combined drugs. 3) Poor compliance: CTLA-4 antibody drugs are mainly administered intravenously, subcutaneously, or intramuscularly, and cannot be administered orally, which leads to poor patient compliance.

Compared with CTLA-4 monoclonal antibody drugs, CTLA-4 small molecule degradation agents have unique advantages: 1) Significantly reduced toxic and side effects: CTLA-4 small molecule degradation agents achieve the purpose of relieving immunosuppression by degrading CTLA-4 protein, they do not affect other activities of T cells, and small molecules do not have ADCC effect and the immunogenicity of antibodies, so the side effects will be considerably reduced. 2) Effortless Intratumoral diffusion: small molecule drugs are easy to infiltrate into solid tumors, significantly improving the therapeutic effect of single or combined drugs. 3) Good compliance: small molecule drugs can be taken orally. 4) Price advantage: the production cost of small molecule drugs is low.

However, at present, there is no development or report of CTLA-4 small molecule degradation agent or small molecule inhibitor in the world.

### SUMMARY

### Problems that the present invention aims to solve

To solve the above technical problems, the present invention provides a new type of small molecule compounds with high degradation activity and high in vivo activity to CTLA-4 for the first time in the world.

### Solutions for solving these problems

To solve the above technical problems, the present invention provides the following technical solutions:
a compound with the structure of formula I or a pharmaceutically acceptable salt, an ester, a deuterated analog, an isomer, a solvate, a prodrug, or an isotopic label thereof: 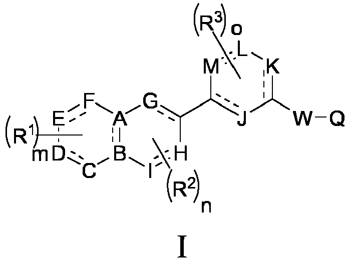 wherein,
A, B, C, D, E, F, G, H, I, J, K, L and M are independently selected from a direct bond, CR⁶, CR⁶R¹⁴, N, NR⁷, O, S and C=O;
each of R¹, R², R³, R⁶, R⁷ and R¹⁴ is independently selected from hydrogen, deuterium, an unsubstituted or substituted alkyl group, an unsubstituted or substituted alkenyl group, an unsubstituted or substituted alkynyl group, an unsubstituted or substituted cycloalkyl group, an unsubstituted or substituted heterocycloalkyl group, halogen, -OH, an unsubstituted or substituted alkoxy group, an unsubstituted or substituted arylalkyl group, an unsubstituted or substituted heteroaryl alkyl group, an unsubstituted or substituted aryl ether group, an unsubstituted or substituted heteroaryl ether group, - CN, -N(R⁴R⁵), -NO₂, -N₃, a boronic acid group, an unsubstituted or substituted boronic ester group, a carboxyl group, an ester group, an unsubstituted or substituted carbamoyl group, an unsubstituted or substituted aryl group, an unsubstituted or substituted heteroaryl group, an unsubstituted or substituted thioether group, an unsubstituted or substituted sulfoxide group, an unsubstituted or substituted sulfone group, an unsubstituted or substituted sulfonamide group, 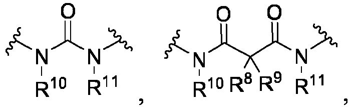 an unsubstituted or substituted phosphate ester group, wherein R⁴, R⁵, R⁸, R⁹, R¹⁰ and R¹¹ is independently selected from hydrogen, deuterium, an unsubstituted or substituted C₁₋₆ alkyl group, a C₃₋₇ cycloalkyl group, an unsubstituted or substituted aryl group, an unsubstituted or substituted heteroaryl group, R⁴ and R⁵, R⁸ and R⁹ can be connected with adjacent nitrogen atoms or carbon atoms to form a ring; or two adjacent R¹ and/or two adjacent R³ can also be connected to form a cycloalkyl group, a heterocycloalkyl group, a aryl group or a heteroaryl group;
m, n and o are independently selected from integers from 0 to 4;
W is selected from the direct bond, an unsubstituted or substituted aryl group, an unsubstituted or substituted heteroaryl group, an unsubstituted or substituted cycloalkyl group, an unsubstituted or substituted heterocycloalkyl group, an unsubstituted or substituted bridged cycloalkyl group, an unsubstituted or substituted bridged heterocycloalkyl group, an unsubstituted or substituted spiro cycloalkyl group, an unsubstituted or substituted spiro heterocycloalkyl group, an unsubstituted or substituted alkyl group, an unsubstituted or substituted heteroalkyl group, an unsubstituted or substituted alkenyl group, an unsubstituted or substituted heteroalkenyl group, an unsubstituted or substituted alkynyl group, an unsubstituted or substituted heteroalkynyl group, an unsubstituted or substituted -N(R¹²R¹³), an unsubstituted or substituted aminoalkyl group, an unsubstituted or substituted aminoalkylamino group,
an unsubstituted or substituted 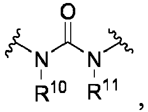 an unsubstituted or substituted 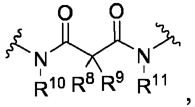 wherein R¹² and R¹³ are independently selected from hydrogen, deuterium, a C₁₋₆ alkyl group, a C₃₋₇ cycloalkyl group, an unsubstituted or substituted alkylamino group, an unsubstituted or substituted aryl group, an unsubstituted or substituted heteroaryl group, or R¹² and R¹³ can be connected to form a ring; Q is -H, -NH₂, -OH, -alkyl-NHC(=O)H, a cycloalkyl group, an unsubstituted or substituted alkylacyl group, an unsubstituted or substituted alkylhydroxy group, an unsubstituted or substituted alkenylhydroxy group, an unsubstituted or substituted alkynylhydroxy group, an unsubstituted or substituted alkylamino group, an unsubstituted or substituted sulfonamide group, an unsubstituted or substituted alkyl sulfonamide group, an amino acid residue, 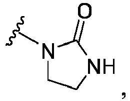 a sulfonamide group, a sulfonyl hydrazine group, an unsubstituted or substituted aryl group, an unsubstituted or substituted heteroaryl group, an unsubstituted or substituted 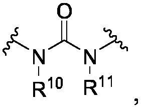 an unsubstituted or substituted 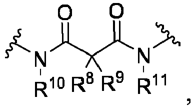 an unsubstituted or substituted -(CH₂ )ₙ₁ -(M)ₙ₂ -(CH₂ )ₙ₃ -(M)ₙ₄ -(CH₂)ₙ₅ -(M)ₙ₆, wherein each M is independently selected from O, OH, S, SO, SO₂ and an unsubstituted or substituted amino group, each n1, n2, n3, n4, n5, and n6 are independently selected from integers from 0 to 6;
or W and Q can be connected or fused to form a substituted or unsubstituted cycloalkyl group, a heterocycloalkyl group, an aryl group, or a heteroaryl group.

Preferably, at least one of C, G, and I is an N atom.

Preferably, at least one of J, K, and M is an N atom.

Preferably, I, J and K are all N atoms, or I, M and K are all N atoms.

Preferably, each of R¹, R², R³, R⁶, R⁷ and R¹⁴ is independently selected from hydrogen, deuterium, an unsubstituted or substituted C₁₋₆ alkyl group, an unsubstituted or substituted C₂₋₆ alkenyl group, an unsubstituted or substituted C₂₋₆ alkynyl group, an unsubstituted or substituted C₃₋₇ cycloalkyl group, an unsubstituted or substituted 3-7 membered heterocycloalkyl group, halogen, -OH, an unsubstituted or substituted C₁₋₆ alkoxyl group, an unsubstituted or substituted C₆₋₁₀ arylethyl group, an unsubstituted or substituted 5-10 membered heteroaryl ethyl group, an unsubstituted or substituted C₆₋₁₀ aryl ether group, an unsubstituted or substituted 5-10 membered heteroaryl ether group, -CN, -NH₂, -NO₂, -N₃, a boronic acid group, an unsubstituted or substituted boronic ester group, a carboxyl group, an ester group, an unsubstituted or substituted carbamoyl group, an unsubstituted or substituted C₆₋₁₀ aryl group, an unsubstituted or substituted 5-10 membered heteroaryl group, an unsubstituted or substituted thioether group, an unsubstituted or substituted sulfoxide group, an unsubstituted or substituted sulfone group, an unsubstituted or substituted sulfonamide group, 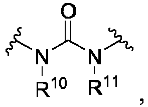 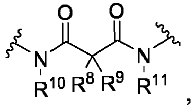 an unsubstituted or substituted phosphate ester group. The substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

Preferably, the W is selected from the direct bond, an unsubstituted or substituted aryl group, a heteroaryl group, a cycloalkyl group, a heterocycloalkyl group, a bridged alkyl group, a bridged heterocycloalkyl group, a spiro cycloalkyl group, a spiro heterocycloalkyl group, an alkyl group, a heteroalkyl group, an alkenyl group, a heteroalkenyl group, an alkynyl group, a heteroalkynyl group, -N(R¹²R¹³), an aminoalkyl group, an aminoalkylamino group, an unsubstituted or substituted 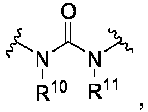 an unsubstituted or substituted 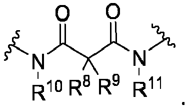 The substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

Preferably, the W is selected from an unsubstituted or substituted 5-7 membered heterocycloalkyl group, an unsubstituted or substituted amino-C₁₋₆ alkyl group. The substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

Preferably, the atom in W connected to the ring containing J and K is N.

Preferably, W is selected from the substituted or unsubstituted 5-7 membered heterocycloalkyl group. The 5-7 membered heterocycloalkyl group contains at least one nitrogen atom. More preferably, the 5-7 membered heterocycloalkyl group is piperidinyl or piperazinyl.

Preferably, Q is -H, -NH₂, -OH, -C₁₋₆ alkyl-HNC(=O)H, an unsubstituted or substituted C₁₋₆ alkylhydroxy group, an unsubstituted or substituted C₂₋₆ alkenylhydroxy group, an unsubstituted or substituted C₂₋₆ alkynylhydroxy group, an unsubstituted or substituted alkylamino group, a sulfonamide group, and a sulfonyl hydrazine group. The substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

Preferably, W and Q can be connected or fused to form a ring. The ring is a substituted or unsubstituted 5-7 membered cycloalkyl group, a substituted or unsubstituted 5-7 membered heterocycloalkyl group, a substituted or unsubstituted C₆₋₁₀ aryl group, and a substituted or unsubstituted 5-10 membered heteroaryl group.

More preferably, the substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

Preferably, the compound with the structure of formula I is: 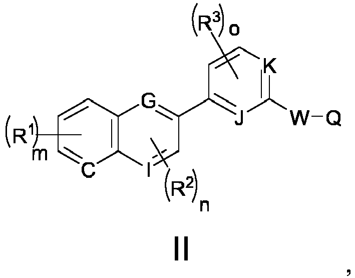 wherein, C, G, I, J, and K are independently selected from CR⁶ and N, and at least one of C, G, and I is N, and at least one of J and K is N. The definitions of R¹, R², R³, R⁶, o, m, n, W and Q are the same as above.

Preferably, the compound with the structure of formula I is: 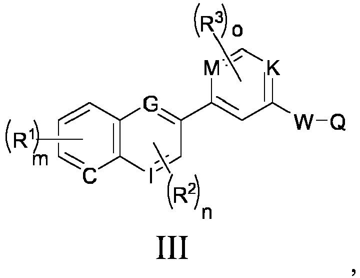 wherein, C, G, I, K, and M are independently selected from CR⁶ and N, and at least one of C, G, and I is N, and at least one of K and M is N; the definitions of R¹, R², R³, R⁶, o, m, n, W and Q are the same as above.

### Effects of the invention

1. The present invention reports a new kind of small molecule compounds with strong degradation activity to CTLA-4 for the first time in the world.
2. The compounds of the present invention show good activity to CTLA-4 at the level of nanomolar (nM) in vitro.
3. The compounds of the present invention, such as compound 18, have excellent anti-tumor effects in the study of tumor transplantation models in vivo, such as the commonly used MC-38 model for tumor immunity.

### DETAILED DESCRIPTION

To describe the content of the present invention more clearly, all terms involved are defined as follows:
The term "direct bond" used herein refers to that two atoms or groups connected to the direct bond are directly connected by a chemical bond, preferably, the chemical bond includes a single bond and a double bond.

Unless otherwise defined, the term "substitution" used herein refers to being replaced by the following substituents: alkyl, cycloalkyl, aryl, heterocyclo, halogen, hydroxy, alkoxy, boronic acid, boronic ester, ester, oxo, alkanoyl, aryloxy, alkanoyloxy, amino, alkylamino, arylamino, arylalkylamino, disubstituted amines in which the 2 amino substituents are selected from alkyl, aryl or arylalkyl; alkanoylamino, aroylamino, aralkanoylamino, substituted alkanoylamino, substituted arylamino, substituted aralkanoylamino, thiol, alkylthio, arylthiol, arylalkylthio, arylthiono, arylalkylthiono, alkylsulfonyl, arylsulfonyl, arylalkylsulfonyl, sulfonamide, e.g. - SO₂NH₂, substituted sulfonamide, nitro, cyano, carboxy, carbamoyl, e.g. -CONH₂, substituted carbamoyl, e.g. -CONHalkyl, -CONHaryl, -CONHarylalkyl or cases where there are two substituents on the nitrogen selected from alkyl, aryl or arylalkyl; alkoxycarbonyl, aryl, substituted aryl, guanidino, heterocyclyl, e.g. indolyl, imidazolyl, furyl, thienyl, thiazolyl, pyrrolidyl, pyridyl, pyrimidyl, pyrrolidinyl, piperidinyl, morpholinyl, piperazinyl, homopiperazinyl and the like, and substituted heterocyclyl.

As used herein, the term "alkyl" or "alkylene" is intended to include both branched and straight-chain saturated aliphatic hydrocarbon groups having the specified number of carbon atoms. For example, "C₁₋₆ alkyl" denotes alkyl having 1 to 6 carbon atoms. Example alkyl groups include, but are not limited to, methyl (Me), ethyl (Et), propyl (e.g., n-propyl and isopropyl), butyl (e.g., n-butyl, isobutyl, tert-butyl), and pentyl (e.g., n-pentyl, isopentyl, neopentyl).

The term "heteroalkyl" or "alkylhetero" refers to those 1 - 4 carbon atoms on alkyl are replaced by heteroatoms, preferably, 1-3 carbon atoms on alkyl are replaced by heteroatoms, more preferably, 1-2 carbon atoms on alkyl are replaced by heteroatoms. Preferably, the alkyl before being substituted by heteroatoms is a C₂₋₁₀ alkyl, more preferably, the alkyl before being substituted by heteroatoms is a C₂₋₆ alkyl. The position that was substituted by heteroatoms can be either the terminal position of alkyl or the middle position of alkyl, and these heteroatoms are independently selected from N, O, S, P, etc.

The term "alkenyl" denotes a straight- or a branch-chained hydrocarbon radical containing one or more double bonds and typically from 2 to 20 carbon atoms in length. For example, "C₂₋₆ alkenyl" contains two to six carbon atoms. Alkenyl groups include, but are not limited to, for example, ethenyl, propenyl, butenyl, 1-methyl-2-buten-1-yl, hexenyl, and the like.

The term "alkynyl" denotes a straight- or a branch-chained hydrocarbon radical containing one or more triple bonds and typically from 2 to 20 carbon atoms in length. For example, "C₂₋₆ alkenyl" contains two to six carbon atoms. Representative alkynyl groups include, but are not limited to, for example, ethynyl, 1-propynyl, 1-butynyl, pentynyl, hexynyl, and the like.

The term "heteroalkenyl" means that one or more carbon atoms in the "alkenyl" as defined above are replaced by heteroatoms selected from N, O, S, or by groups containing heteroatoms selected from N, O, S.

The term "heteroalkynyl" means that one or more carbon atoms in the "alkynyl" as defined above are replaced by heteroatoms selected from N, O, S, or by groups containing heteroatoms selected from N, O, S.

The term "alkoxy" or "alkyloxy" refers to an -O-alkyl group. "C₁₋₁₀ alkoxy" (or alkyloxy), is intended to include C₁-C₁₀ alkoxy groups. Example alkoxy groups include, but are not limited to, methoxy, ethoxy, propoxy (e.g., n-propoxy and isopropoxy), and tert-butoxy, while alkoxy groups can have multiple oxygen atoms, such as 1-10 oxygen atoms. Similarly, "alkylthio" or "thioalkoxy" represents an alkyl group as defined above with the indicated number of carbon atoms attached through a sulfur bridge, for example, methyl-S- and ethyl-S-.

The term "carbonyl" or "acyl" refers to organic functional groups (C=O) formed by the double bond connection of carbon and oxygen atoms.

The term "ester" includes carboxylic acid ester, phosphate ester, phosphite ester, silicate ester, boronic ester, etc. For example, -COOR, B(OR)₂, wherein R is alkyl.

The term "cycloalkyl" refers to a monocyclic or bicyclic alkyl group. Monocyclic alkyl refers to C₃₋₈ cyclic alkyl including, but not limited to, cyclopropyl, cyclobutyl, cyclopentyl, cyclohexyl, and norbornyl. Branched cycloalkyl such as 1-methylcyclopropyl and 2-methylcyclopropyl are included in the definition of "cycloalkyl". Bicyclic alkyl includes bridged, spiro, or fused cycloalkyl.

The term "cycloalkenyl" refers to a monocyclic or bicyclic alkenyl group. Monocyclic alkenyl refers to C₃-C₈ cyclic alkenyl including, but not limited to, cyclopropenyl, cyclobutenyl, cyclopentenyl, cyclohexenyl, and norbornenyl. Branched cycloalkenyl such as 1-methylcyclopropenyl and 2-methylcyclopropenyl are included in the definition of "cycloalkenyl", Bicyclic alkenyl includes bridged, spiro, or fused cyclic alkenyl.

"Halo" or "halogen" includes fluoro, chloro, bromo, and iodo. "Haloalkyl" is intended to include both branched and straight-chain saturated aliphatic hydrocarbon groups having the specified number of carbon atoms and substituted with one or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl, and heptachloropropyl. Examples of haloalkyl also include "fluoroalkyl" groups intended to include branched and straight chain saturated aliphatic hydrocarbon groups having the specified number of carbon atoms and substituted with one or more fluorine atoms.

"Haloalkoxy" or "haloalkyloxy" denotes a haloalkyl group, as defined above, having the indicated number of carbon atoms linked via an oxygen bridge. For example, "C₁₋₆ haloalkoxy" is intended to include C1, C2, C3, C4, C5, and C6 haloalkoxy. Examples of haloalkoxy include, but are not limited to, trifluoromethoxy, 2,2,2-trifluoroethoxy, and pentafluoroethoxy. Similarly, "haloalkylthio" or "thiohaloalkoxy" denotes a haloalkyl group, as defined above, having the indicated number of carbon atoms linked via a sulfur bridge; for example, trifluoromethyl-S- and pentafluoroethyl-S-.

The term "aryl"/"arylene", either alone or as part of a larger moiety such as "aralkyl", "aralkoxy", or aryloxyalkyl", refers to monocyclic, bicyclic, and tricyclic ring systems having a total of 6 to 10 ring members, wherein at least one ring in the system is aromatic and wherein each ring in the system contains three to seven ring members. In certain embodiments of the invention, "aryl" refers to an aromatic ring system which includes, but is not limited to phenyl, indanyl, 1-naphthyl, 2-naphthyl, and terahydronaphthyl. The fused aryls may be connected to another group either at a suitable position on the cycloalkyl ring or the aromatic ring. For example, arrowed lines drawn from the ring system indicate that the bond may be attached to any of the suitable ring atoms.

The terms "heteroary/heteroarylene", "arylheterocyclo", "heteroarylene", "heteroary", "arylheterocyclo" or "arylheterocyclo group" are intended to mean a stable 3-, 4-, 5-, 6-, or 7-membered monocyclic or bicyclic or 7-, 8-, 9-, 10-, 11-, 12-, 13-, or 14-membered polycyclic heterocyclic ring that is partially unsaturated or fully unsaturated, and that contains carbon atoms and 1, 2, 3 or 4 heteroatoms independently selected from the group consisting of N, O and S; and including any polycyclic group in which any of the above-defined heterocyclic rings is fused to a benzene ring. The nitrogen and sulfur heteroatoms may optionally be oxidized. The nitrogen atom may be substituted or unsubstituted (i.e., N or NR wherein R is H or another substituent, if defined). The heterocyclic ring may be attached to its pendant group at any heteroatom or carbon atom that results in a stable structure. The heterocyclic rings described herein may be substituted on carbon or on a nitrogen atom if the resulting compound is stable. Nitrogen in the heterocycle may optionally be quaternized. Preferably, when the total number of S and O atoms in the heterocycle exceeds 1, then these heteroatoms are not adjacent to one another. Preferably, the total number of S and O atoms in the heterocycle is not more than 1. Examples of heteroaryl include, but are not limited to, acridinyl, azetidinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolinyl, 2H,6H-l,5,2-dithiazinyl, dihydrofuro[2,3-b]tetrahydrofuran, furanyl, furazanyl, imidazolidinyl, imidazolinyl, imidazolyl, lH-indazolyl, imidazolopyridinyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridinyl, isoxazolyl, isoxazolopyridinyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinylperimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridooxazolyl, pyridoimidazolyl, pyridothiazolyl, pyridinyl, pyrimidinyl, pyrrolidinyl, pyrrolinyl, 2-pyrrolidonyl, 2H-pyrrolyl, pyrrolyl, quinazolinyl, quinolinyl, 4H-quinolizinyl, quinoxalinyl, quinuclidinyl, tetrazolyl, tetrahydrofuranyl, tetrahydroisoquinolinyl, tetrahydroquinolinyl, 6H-l,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridinyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, xanthenyl, quinolinyl, isoquinolinyl, phthalazinyl, quinazolinyl, indolyl, isoindolyl, indolinyl, lH-indazolyl, benzimidazolyl, 1,2.3,4-tetrahydroquinolinyl, 1,2.3,4-tetrahydroisoquinolinyl, 5,6,7,8-tetrahydro-quinolinyl, 2,3-dihydro-benzofuranyl, chromanyl, 1,2,3,4-tetrahydro-quinoxalinyl and 1,2,3,4-tetrahydro-quinazolinyl. Also included are fused ring and spiro compounds containing, for example, the above heterocycles.

As used herein, the term "heterocycloalkyl" refers to a monocyclic heterocycloalkyl system, or a bicyclic heterocycloalkyl system. The monocyclic heterocycloalkyl refers to a saturated or unsaturated but not aromatic 3 to 12-membered (preferably, 3 to 8-membered, more preferably, 5 to 7-membered) cyclic alkyl system containing at least one heteroatom selected from O, N, S, or P. The bicyclic heterocycloalkyl system refers to a heterocycloalkyl fused with a phenyl, or a cycloalkyl, or a cycloalkenyl, or a heterocycloalkyl, or a heteroaryl. The heterocyclic alkyl group includes, but is not limited to, aziridinyl, azacyclobutyl, oxyheterocyclicbutyl, pyrrolidyl, tetrahydrofuranyl, tetrahydrothienyl, piperidinyl, morpholinyl, piperazinyl, thiomorpholinyl, tetrahydropyranyl, 1,1-dioxythiomorpholinyl, 1,4-diazoalkyl, etc.

As used herein, the term "bridged cycloalkyl" refers to a 5 to 20-membered full-carbon polycyclic group, wherein every two rings in the system share two disconnected atoms, wherein the rings may have one or more double bonds, preferably 6 to 14-membered, and more preferably 7 to 10-membered. According to the number of membered rings, bridged cycloalkyl may be divided into bicyclic, tricyclic, tetracyclic, or polycyclic bridged cycloalkyl, and preferably bicyclic, tricyclic or tetracyclic, and more preferably bicyclic or tricyclic.

As used herein, the term "bridged heterocycloalkyl", "hetero bridged cycloalkyl" and "bridged cycloheteroalkyl" refers to polycyclic compounds that share two or more carbon atoms. The "bridged heterocycloalkyl", "hetero bridged cycloalkyl" and "bridged cycloheteroalkyl" at least have one heteroatom selected from O, N, S, P, etc. including bicyclic bridged cyclic hydrocarbons and polycyclic bridged cyclic hydrocarbons. The former is composed of two alicyclic rings sharing more than two carbon atoms; the latter is bridged cyclic hydrocarbons consisting of more than three rings.

As used herein, the term "spirocyclic hydrocarbon" and "spirocycloalkyl" refers to polycyclic hydrocarbons and polycyclicalkyl that share one carbon atom (referred to as a Spiro atom) between single rings.

The term "bridged cycloalkyl" used herein refers to 5 to 20-membered polycyclic heterocyclyl with rings connected through one common atom (called a spiro atom), wherein said rings have one or more heteroatoms selected from the group consisting of nitrogen, and oxygen. The sulfur can be optionally oxidized (i.e., forming sulfoxide or sulfone) and sulfur as ring atoms and the remaining ring atoms being carbon atoms, wherein one or more rings may contain one or more double bonds; preferably 6 to 14 membered, and more preferably 7 to 10-membered (e.g., 7, 8, 9 or 10-membered). According to the number of common spiro atoms, spiro heterocyclyl may be divided into mono-spiro heterocyclyl, di-spiro heterocyclyl, or poly-spiro heterocyclyl, preferably mono-spiro heterocyclyl or di-spiro heterocyclyl, and more preferably 3-membered/5-membered, 3-membered/6-membered, 4-membered/4-membered, 4-membered/5-membered, 4-membered/6-membered, 5-membered/5-membered or 5-membered/6-membered mono-spiro heterocyclyl.

As used herein, the term "isomer" includes "tautomer", "stereoisomer", etc. "Tautomer" refers to structural isomers of different energies which are interconvertible via a low energy barrier. Where tautomerization is possible (e.g., in solution), a chemical equilibrium of tautomers can be reached. For example, proton tautomers (also known as prototropic tautomers) include, but are not limited to, interconversions via migration of a proton, such as keto-enol isomerization, imine-enamine isomerization, and amide iminol isomerization. "Stereoisomers" refers to compounds which have identical chemical constitutions, but differ with regard to the arrangement of the atoms or groups in space. Stereoisomers include enantiomers, diastereomers, conformer (rotamer), geometric (cis/trans) isomers, atropisomers, etc. Unless otherwise stated, all tautomeric forms of the compounds disclosed herein are within the scope of the invention.

As referred to herein, the term "substituted" means that at least one hydrogen atom is replaced with a non-hydrogen group, provided that normal valencies are maintained and that the substitution results in a stable compound. Ring double bonds, as used herein, are double bonds that are formed between two adjacent ring atoms (e.g., C=C, C=N, or N=N).

In cases wherein there are nitrogen atoms (e.g., amines) on compounds of the present invention, these may be converted to N-oxides by treatment with an oxidizing agent (e.g., mCPBA and/or hydrogen peroxides) to afford other compounds of this invention. Thus, shown and claimed nitrogen atoms are considered to cover both the shown nitrogen and its N-oxide (N→O) derivative.

When any variable occurs more than one time in any constituent or formula for a compound, its definition at each occurrence is independent of its definition at every other occurrence. Thus, for example, if a group is shown to be substituted with 0-3 R, then said the group may optionally be substituted with up to three R groups, and at each occurrence, R is selected independently from the definition of R. Also, combinations of substituents and/or variables are permissible only if such combinations result in stable compounds.

When a bond to a substituent is shown to cross a bond connecting two atoms in a ring, then such substituent may be bonded to any atom on the ring. When a substituent is listed without indicating the atom in which such substituent is bonded to the rest of the compound of a given formula, then such substituent may be bonded via any atom in such substituent. Combinations of substituents and/or variables are permissible only if such combinations result in stable compounds.

The terms "amino", individually or in combination, denote primary amino (-NH₂), secondary amino (-NH-), or tertiary amino ( 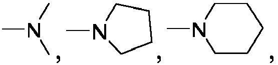 etc.) and the like.

The term "C₁₋₆ alkylamino" represents an amino group as defined above alone or in combination, wherein the hydrogen atom of the amino group is replaced by at least one C₁₋₆ alkyl group, wherein "alkyl" represents as defined as above. Accordingly, "C₁₋₆ alkylamino" includes methylamino, ethylamino, propylamino, isopropylamino, n-butylamino, isobutylamino, 2-butylamino, tert-butylamino, n-pentylamino, 2-pentylamino, 3-pentylamino, 2-methyl-2-butylamino, 3-methyl-2-butylamino, 3-methyl-1-butylamino, 2-methyl-1-butylamino, n-hexylamino, 2-hexylamino, 3-hexylamino, 2-methyl-2-pentylamino, 3-methyl-2-pentylamino, 4-methyl-2-pentylamino, 3-methyl-3-pentylamino, 2-methyl-3-pentylamino, 2,3-dimethyl-2-butylamino, 3,3-dimethyl-2-butylamino, etc. In particular, the "C₁₋₆ alkylamino" is methylamino, ethylamino, isopropylamino, tert-butylamino, etc.

The term "(C₁₋₆ alkyl)₂amine" represents the amine group as defined above alone or in combination, wherein the hydrogen atom of the amino group is replaced by two C₁₋₆ alkyl groups, wherein "alkyl" represents as defined as above. Accordingly, "(C1-6 alkyl)₂amine " includes dimethylamino, diethylamino, methylethylamino, etc.

As used herein, the term "amino acid residue" refers to that carboxyl or amine group at the carbon-terminal of an amino acid participates in the formation of a bond and loses a molecule of water. The amino acid unit is called an amino acid residue.

As used herein, the term "[Cu]" refers to reagents containing monovalent copper (Cu⁺) or bivalent copper (Cu²⁺), such as CuI, CuBr, CuCl, CuI₂, CuBr₂, CuCl₂, etc.

The term "isomer" includes all isomeric forms including enantiomers, diastereomers, tautomers and geometric isomers (including cis-trans isomers). Therefore, the individual stereochemical isomer of the compounds devised in the present invention or the mixture of an enantiomer, a diastereomer, a tautomer or a geometric isomer (or a cis-trans isomer) thereof all belong to the scope of the present invention.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic groups such as amines; and alkali or organic salts of acidic groups such as carboxylic acids. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, and nitric; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, and isethionic, and the like.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington: The Science and Practice of Pharmacy, 22nd Edition, Allen, L. V. Jr., Ed.; Pharmaceutical Press, London, UK (2012), the disclosure of which is hereby incorporated by reference.

The term "solvate" means a physical association of a compound of this invention with one or more solvent molecules, whether organic or inorganic. This physical association includes hydrogen bonding. In certain instances, the solvate will be capable of isolation, for example, when one or more solvent molecules are incorporated in the crystal lattice of the crystalline solid. The solvent molecules in the solvate may be present in a regular arrangement and/or a non-ordered arrangement. The solvate may comprise either a stoichiometric or nonstoichiometric amount of the solvent molecules. "Solvate" encompasses both solution-phase and isolable solvates. Exemplary solvates include, but are not limited to, hydrates, ethanolates, methanolates, and isopropanolates.

### Methods of solvation are generally known in the art.

The term "ester" refers to organic esters, including monoesters, diesters, triesters, and more generally polyesters.

The term "isotopic derivative" refers to the isotopic derivatives obtained by replacing the hydrogen atom with 1-6 deuterium atoms (D) in the general formula (I), the isotopic derivatives obtained by replacing the carbon atom with 1-3 carbon-14 atoms (¹⁴C) in the general formula (I).

As used herein, the term "treating" includes any effect, e.g., lessening, reducing, modulating, ameliorating, or eliminating, that results in the improvement of the condition, disease, disorder, and the like, or ameliorating a symptom thereof.

As used herein, the term "pharmaceutical composition" refers to the combination of an active agent with a carrier, inert or active, making the composition especially suitable for diagnostic or therapeutic use in vivo or ex vivo. Examples of bases include, but are not limited to, alkali metals (e.g., sodium) hydroxides, alkaline earth metals (e.g., magnesium), hydroxides, ammonia, and the like. For therapeutic use, salts of the compounds of the present invention are contemplated as being pharmaceutically acceptable. However, salts of acids and bases that are non-pharmaceutically acceptable may also find use, for example, in the preparation or purification of a pharmaceutically acceptable compound.

### SPECIFIC PHARMACEUTICAL AND MEDICAL TERMS

The term "cancer", as used herein, refers to an uncontrolled abnormal growth of cells and is capable of metastasis (transmission) under certain conditions. This type of cancer includes, but is not limited to, solid tumors (e.g., bladder, bowel, brain, chest, uterus, heart, kidney, lung, lymphoid tissue (lymphoma), ovary, pancreas, or other endocrine organs (e.g., thyroid), prostate, skin (melanoma), or hematological tumors (e.g., aleukemic leukemia).

### PHARMACEUTICAL COMPOSITIONS AND DOSAGES

The pharmaceutical compositions of the present invention may include a therapeutically effective amount of one or more compounds of formula I together with optionally one or more pharmaceutically acceptable carriers (additives) and/or diluents, and optionally one or more of the other therapeutic agents described above. The compounds of the present invention may be administered for any of the above-mentioned uses by any suitable means, for example by orally, such as in the form of tablets, capsules (each of which includes sustained release or timed release formulations), pills, powders, granules, elixirs, tinctures, suspensions (including nanosuspensions, micro suspensions, spray-dried dispersions), syrups and emulsions; by sublingually; by buccally; by parenterally, such as by subcutaneous, intravenous, intramuscular or intrasternal injection or infusion techniques (e.g., in the form of sterile injectable aqueous or nonaqueous solutions or suspensions); by nasally, including administration to the nasal mask, such as by inhalation spray; by topically, such as in the form of a cream or ointment; or by rectally, such as in the form of suppositories. They may be administered alone, but are generally administered using pharmaceutical acceptable carriers selected based on the chosen route of administration and standard pharmaceutical practice.

Pharmaceutically acceptable carriers are formulated according to a number of factors well within the purview of those of ordinary skill in the art. These factors include, but not limited to: the type and nature of the active agent being formulated; the subject to which the agent-containing composition is to be administered; the intended route of administration of the composition; and the therapeutic indication being targeted. Pharmaceutically acceptable carriers include both aqueous and non-aqueous liquid media, as well as a variety of solid and semi-solid dosage forms.

The above carriers can include a number of different ingredients and additives in addition to the active agent, the above additional ingredients being included in the formulation for a variety of reasons, e.g., stabilization of the active agent, binders, etc., well-known to those of ordinary skill in the art. Descriptions of suitable pharmaceutically acceptable carriers, and factors involved in their selection, are found in a variety of readily available sources such as, for example, Allen, L. V. Jr. et al. Remington: The Science and Practice of Pharmacy (2 Volumes), 22nd Edition (2012), Pharmaceutical Press.

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. By way of general guidance, the daily oral dosage of each active ingredient, when used for the indicated effects, will range between about 0.001 to about 10-5000 mg per day, preferably between about 0.01 to about 1000 mg per day, and most preferably between about 0.1 to about 250 mg per day. Intravenously, the most preferred doses will range from about 0.01 to about 10 mg/kg/minute during a constant rate infusion. Compounds of this invention may be administered in a single daily dose, or the total daily dosage may be administered in divided doses of two, three, or four times daily.

The compounds are typically administered in admixture with suitable pharmaceutical diluents, excipients, or carriers (collectively referred to herein as pharmaceutical carriers) suitably selected with respect to the intended form of administration, e.g., oral tablets, capsules, elixirs, and syrups, and consistent with conventional pharmaceutical practices.

Dosage forms (pharmaceutical compositions) suitable for administration may contain from about 0.1 milligram to about 2000 milligrams of active ingredient per dosage unit. In these pharmaceutical compositions, the active ingredient will be ordinarily present in an amount of about 0.1 %-95% by weight based on the total weight of the composition.

A typical injectable preparation is produced by aseptically placing at least one of the compounds of the present invention (250 mg) into a vial, aseptically freeze-drying and sealing. For use, the contents of the vial are mixed with 2 mL of physiological saline, to produce an injectable preparation.

The present invention includes within its scope pharmaceutical compositions comprising, as an active ingredient, a therapeutically effective amount of at least one of the compounds of the present invention, alone or in combination with a pharmaceutical carrier. Optionally, compounds of the present invention can be used alone, in combination with other compounds of the invention, or in combination with one or more other therapeutic agent(s), e.g., an anticancer agent or other pharmaceutically active material.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved. In general, a suitable daily dose of a compound of the invention will be that amount of the compound which is the lowest dose effective to produce a therapeutic effect. Such an effective dose will generally depend upon the factors described above. Generally, oral, intravenous, intracerebroventricular and subcutaneous doses of the compounds of this invention for a patient will range from about 0.01 to about 1000 mg per kilogram of body weight per day. If desired, the effective daily dose of the active compound may be administered as two, three, four, five, six or more sub-doses administered separately at appropriate intervals throughout the day, optionally, in unit dosage forms. In certain aspects of the invention, dosing is one administration per day.

While it is possible for a compound of the present invention to be administered alone, it is preferable to administer the compound as a pharmaceutical formulation (composition).

The features mentioned above, or the features mentioned in the embodiments mentioned herein, may be combined in any combination. All of the features disclosed in this specification may be used in combination with any composition, and each feature disclosed in this specification may be replaced by any alternative feature serving the same, equivalent or similar purpose. Thus, unless otherwise specified, the features disclosed are only general examples of equivalent or similar features.

First, the present invention provides a compound with the structure of formula I or a pharmaceutically acceptable salt, an ester, a deuterated analog, an isomer, a solvate, a prodrug, or an isotopic label thereof: 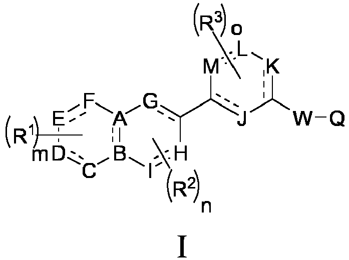 wherein,
A, B, C, D, E, F, G, H, I, J, K, L, and M are independently selected from the direct bond, CR⁶, CR⁶R¹⁴, N, NR⁷, O, S and C=O;
each of R¹, R², R³, R⁶, R⁷ and R¹⁴ is independently selected from hydrogen, deuterium, an unsubstituted or substituted alkyl group, an unsubstituted or substituted alkenyl group, an unsubstituted or substituted alkynyl group, an unsubstituted or substituted cycloalkyl group, an unsubstituted or substituted heterocycloalkyl group, halogen, -OH, an unsubstituted or substituted alkoxy group, an unsubstituted or substituted arylalkyl group, an unsubstituted or substituted heteroaryl alkyl group, an unsubstituted or substituted aryl ether group, an unsubstituted or substituted heteroaryl ether group, - CN, -N(R⁴R⁵), -NO₂, -N₃, a boronic acid group, an unsubstituted or substituted boronic ester group, a carboxyl group, an ester group, an unsubstituted or substituted carbamoyl group, an unsubstituted or substituted aryl group, an unsubstituted or substituted heteroaryl group, an unsubstituted or substituted thioether group, an unsubstituted or substituted sulfoxide group, an unsubstituted or substituted sulfone group, an unsubstituted or substituted sulfonamide group, 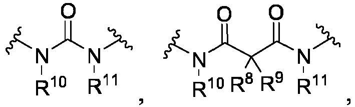 an unsubstituted or substituted phosphate ester group, wherein R⁴, R⁵, R⁸, R⁹, R¹⁰ and R¹¹ are independently selected from hydrogen, deuterium, an unsubstituted or substituted C₁₋₆ alkyl group, C₃₋₇ cycloalkyl group, an unsubstituted or substituted aryl group, an unsubstituted or substituted heteroaryl group, R⁴ and R⁵, R⁸ and R⁹ can be connected with adjacent nitrogen atoms or carbon atoms to form a ring;
or two adjacent R¹ and/or two adjacent R³ can also be connected to form cycloalkyl group, a heterocycloalkyl group, an aryl group or a heteroaryl group;
m, n and o are independently selected from integers from 0 to 4;
W is selected from the direct bond, an unsubstituted or substituted aryl group, an unsubstituted or substituted heteroaryl group, an unsubstituted or substituted cycloalkyl group, an unsubstituted or substituted heterocycloalkyl group, an unsubstituted or substituted bridged cycloalkyl group, an unsubstituted or substituted bridged heterocycloalkyl group, an unsubstituted or substituted spiro cycloalkyl group, an unsubstituted or substituted spiro heterocycloalkyl group, an unsubstituted or substituted alkyl group, an unsubstituted or substituted heteroalkyl group, an unsubstituted or substituted alkenyl group, an unsubstituted or substituted heteroalkenyl group, an unsubstituted or substituted alkynyl group, an unsubstituted or substituted heteroalkynyl group, an unsubstituted or substituted -N(R¹²R¹³), an unsubstituted or substituted aminoalkyl group, an unsubstituted or substituted aminoalkylamino group,
an unsubstituted or substituted 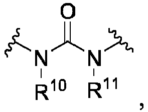 an unsubstituted or substituted 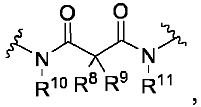 wherein R¹² and R¹³ are independently selected from hydrogen, deuterium, C₁₋₆ alkyl group, C₃₋₇ cycloalkyl group, an unsubstituted or substituted alkylamino group, an unsubstituted or substituted aryl group, an unsubstituted or substituted heteroaryl, or R¹² and R¹³ can be connected to form a ring;
Q is -H, -NH₂, -OH, -alkyl-NHC(=O)H, cycloalkyl group, an unsubstituted or substituted alkylacyl group, an unsubstituted or substituted alkylhydroxy group, an unsubstituted or substituted alkenylhydroxy group, an unsubstituted or substituted alkynylhydroxy group, an unsubstituted or substituted alkylamino group, an unsubstituted or substituted sulfonamide group, an unsubstituted or substituted alkyl sulfonamide group, amino acid residues, 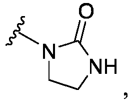 a sulfonamide group, a sulfonyl hydrazine group, an unsubstituted or substituted aryl group, an unsubstituted or substituted heteroaryl group, an unsubstituted or substituted 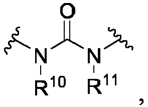 an unsubstituted or substituted 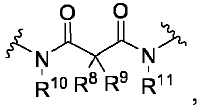 an unsubstituted or substituted -(CH₂ )ₙ₁ -(M)ₙ₂ -(CH₂ )ₙ₃ -(M)ₙ₄ -(CH₂ )ₙ₅ -(M)ₙ₆, wherein each M is independently selected from O, OH, S, SO, SO₂ and an unsubstituted or substituted amino group, each n1, n2, n3, n4, n5, and n6 are independently selected from integers from 0 to 6;
or W and Q can be connected or fused to form a substituted or unsubstituted cycloalkyl, heterocycloalkyl, aryl, or heteroaryl.

Preferably, at least one of C, G, and I is an N atom, for example, any one of C, G and I is an N atom, or any two of C, G and I are N atoms (i.e., C and G are N atoms, or C and I are N atoms, or G and I are N atoms), or C, G and I are all N atoms.

Preferably, at least one of J, K, and M is an N atom, for example, J is an N atom or K is an N atom, or both J and K are N atoms.

Preferably, I, J, and K are N atoms, or I, M and K are N atoms.

Preferably, each of R¹, R², R³, R⁶, R⁷ and R¹⁴ is independently selected from hydrogen, deuterium, an unsubstituted or substituted C₁₋₆ alkyl group, an unsubstituted or substituted C₂₋₆ alkenyl group, an unsubstituted or substituted C₂₋₆ alkynyl group, an unsubstituted or substituted C₃₋₇ cycloalkyl group, an unsubstituted or substituted 3-7 membered heterocycloalkyl group, halogen, -OH, an unsubstituted or substituted C₁₋₆ alkoxyl group, an unsubstituted or substituted C₆₋₁₀ arylethyl group, an unsubstituted or substituted 5-10 membered heteroaryl ethyl group, an unsubstituted or substituted C₆₋₁₀ aryl ether group, an unsubstituted or substituted 5-10 membered heteroaryl ether group, -CN, -NH₂, -NO₂, -N₃, a boronic acid group, an unsubstituted or substituted boronic ester group, a carboxyl group, an ester group, an unsubstituted or substituted carbamoyl group, an unsubstituted or substituted C₆₋₁₀ aryl group, an unsubstituted or substituted 5-10 membered heteroaryl group, an unsubstituted or substituted thioether group, an unsubstituted or substituted sulfoxide group, an unsubstituted or substituted sulfone group, an unsubstituted or substituted sulfonamide group, 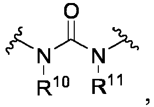 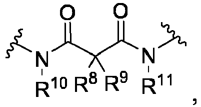 an unsubstituted or substituted phosphate ester group. The substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

Preferably, the W is selected from the direct bond, an unsubstituted or substituted aryl group, a heteroaryl group, a cycloalkyl group, a heterocycloalkyl group, a bridged cycloalkyl group, a bridged heterocycloalkyl group, a spiro cycloalkyl group, a spiro heterocycloalkyl group, an alkyl group, a heteroalkyl group, an alkenyl group, a heteroalkenyl group, an alkynyl group, a heteroalkynyl group, -N(R¹²R¹³), an aminoalkyl group, an aminoalkyl group, an aminoalkylamino group, an unsubstituted or substituted 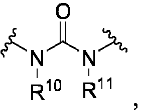 an unsubstituted or substituted 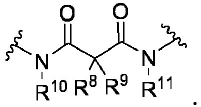 The substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

Preferably, W is selected from an unsubstituted or substituted 5-7 membered heterocycloalkyl group, an unsubstituted or substituted amino-C₁₋₆ alkyl group. The substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

Preferably, the atom in W connected to the ring containing J and K is N.

Preferably, W is selected from a substituted or unsubstituted 5-7 membered heterocycloalkyl group. The 5-7 membered heterocycloalkyl group contains at least one nitrogen atom. More preferably, the 5-7 membered heterocycloalkyl group is piperidinyl or piperazinyl.

Preferably, Q is -H, -NH₂, -OH, -C₁₋₆ alkyl-HNC(=O)H, an unsubstituted or substituted C₁₋₆ alkylhydroxy group, an unsubstituted or substituted C₂₋₆ alkenylhydroxy group, an unsubstituted or substituted C₂₋₆ alkynylhydroxy group, an unsubstituted or substituted alkylamino, a sulfonamide group, and a sulfonyl hydrazine group. The substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

Preferably, W and Q can be connected or fused to form a ring. The ring is a substituted or unsubstituted 5-7 membered cycloalkyl group, a substituted or unsubstituted 5-7 membered heterocycloalkyl group, a substituted or unsubstituted C₆₋₁₀ aryl group, and a substituted or unsubstituted 5-10 membered heteroaryl group.

More preferably, the substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

Preferably, the compound with the structure of formula I is: 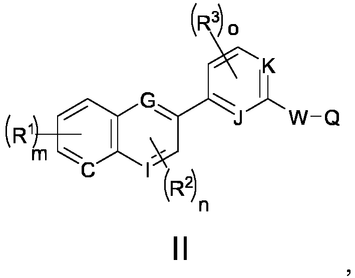 wherein, C, G, I, J, and K are independently selected from CR⁶ and N, and at least one of C, G, and I is N, and at least one of J and K is N. The definitions of R¹, R², R³, R⁶, o, m, n, W and Q are the same as above.

Preferably, the compound with the structure of formula I is: 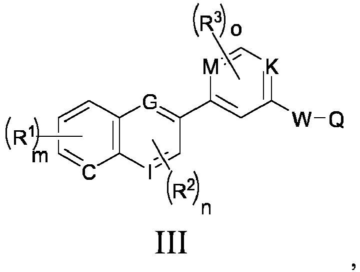 wherein, C, G, I, K, and M are independently selected from CR⁶ and N, and at least one of C, G, and I is N, and at least one of K and M is N. The definitions of R¹, R², R³, R⁶, o, m, n, W and Q are the same as above.

In a preferred embodiment, the present invention also provides the following compounds or a pharmaceutically acceptable salt, an ester, a deuterated analog, an isomer, a solvate, a prodrug, or an isotopic label thereof, compounds are selected from:

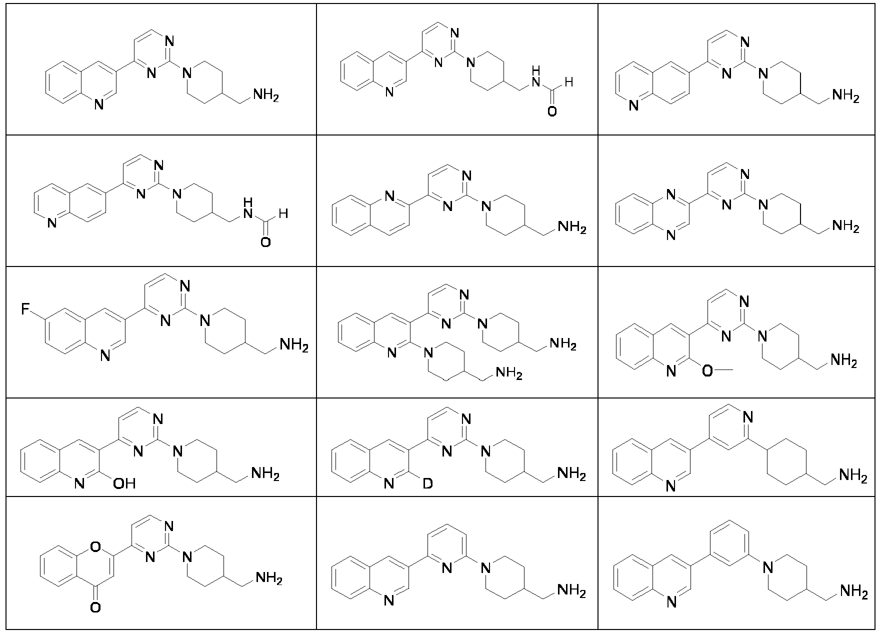

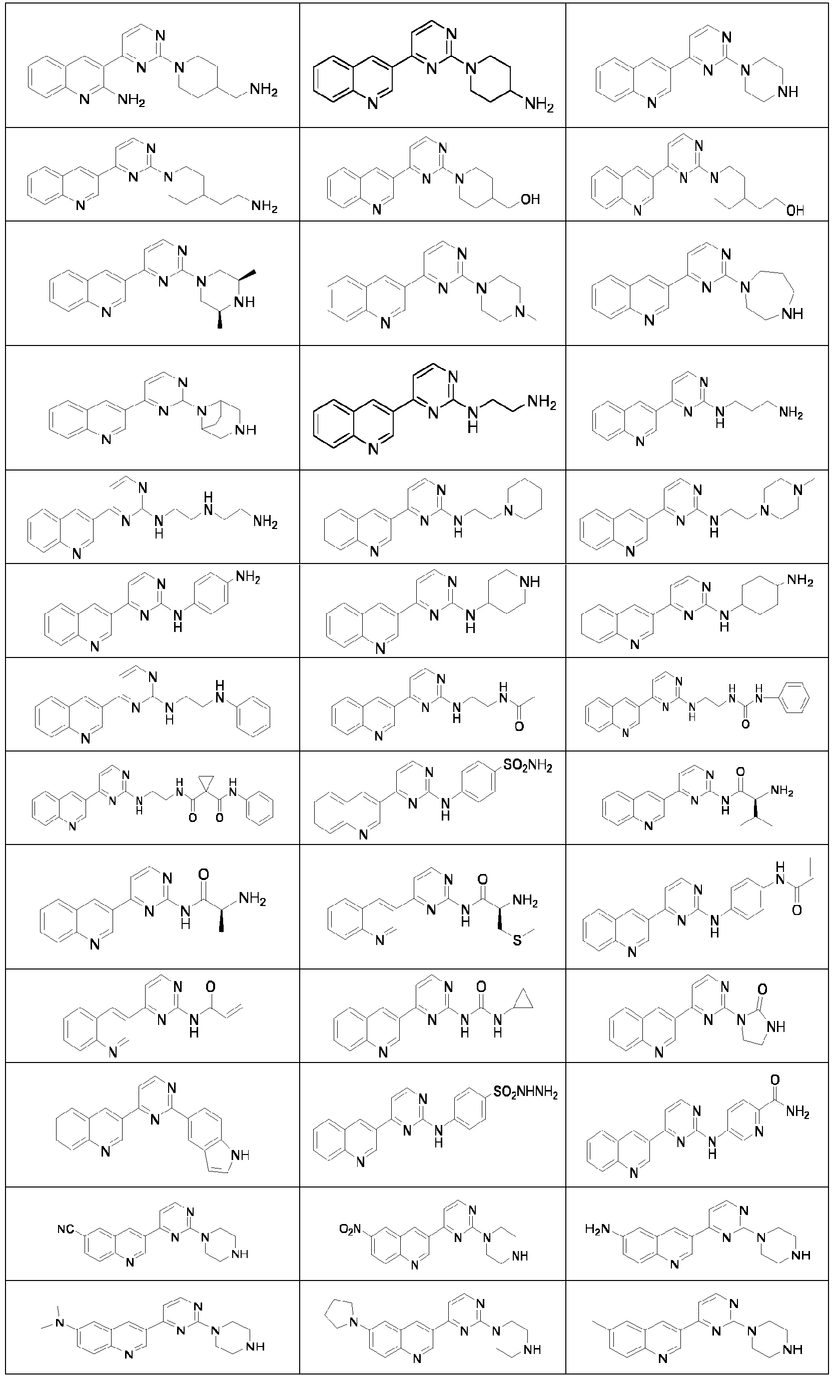

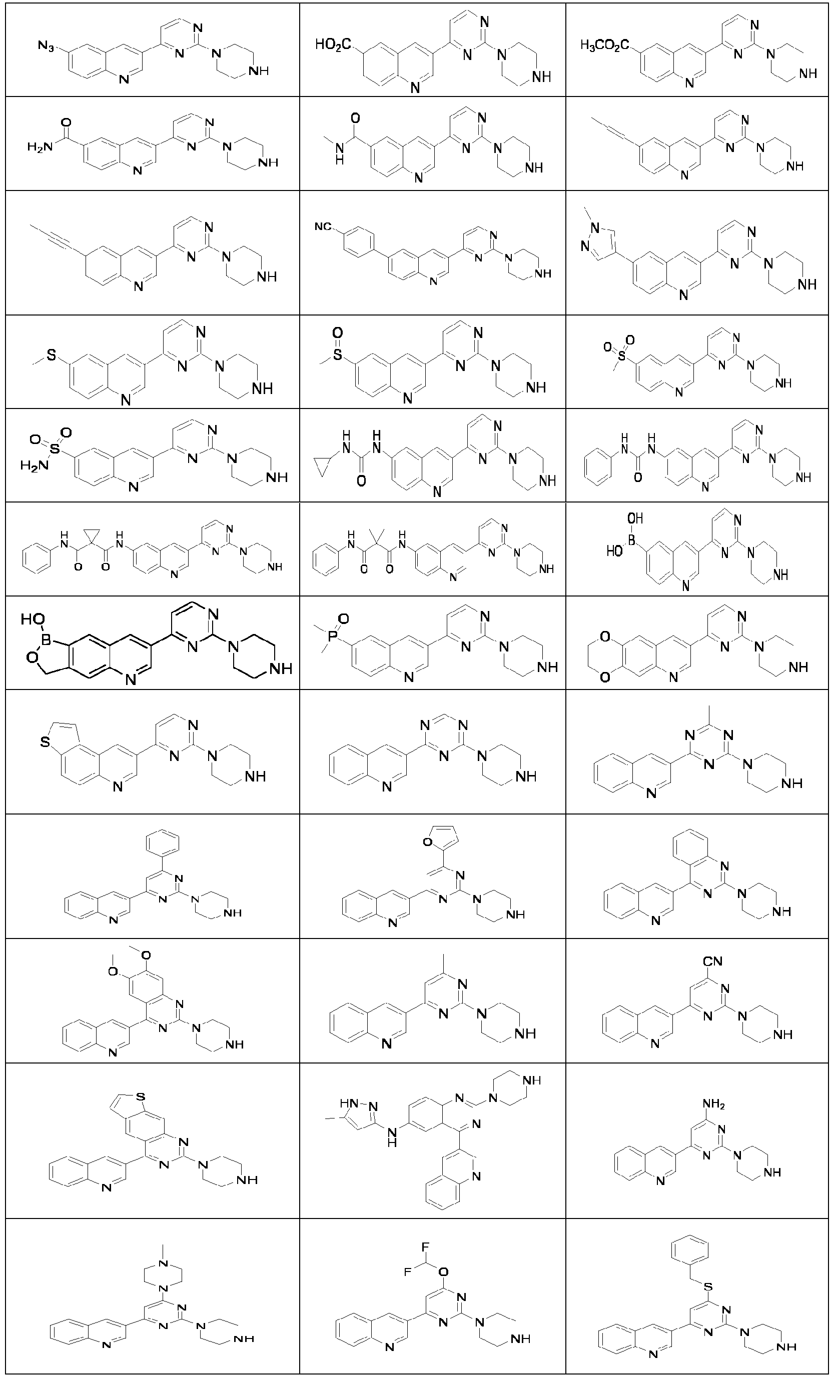

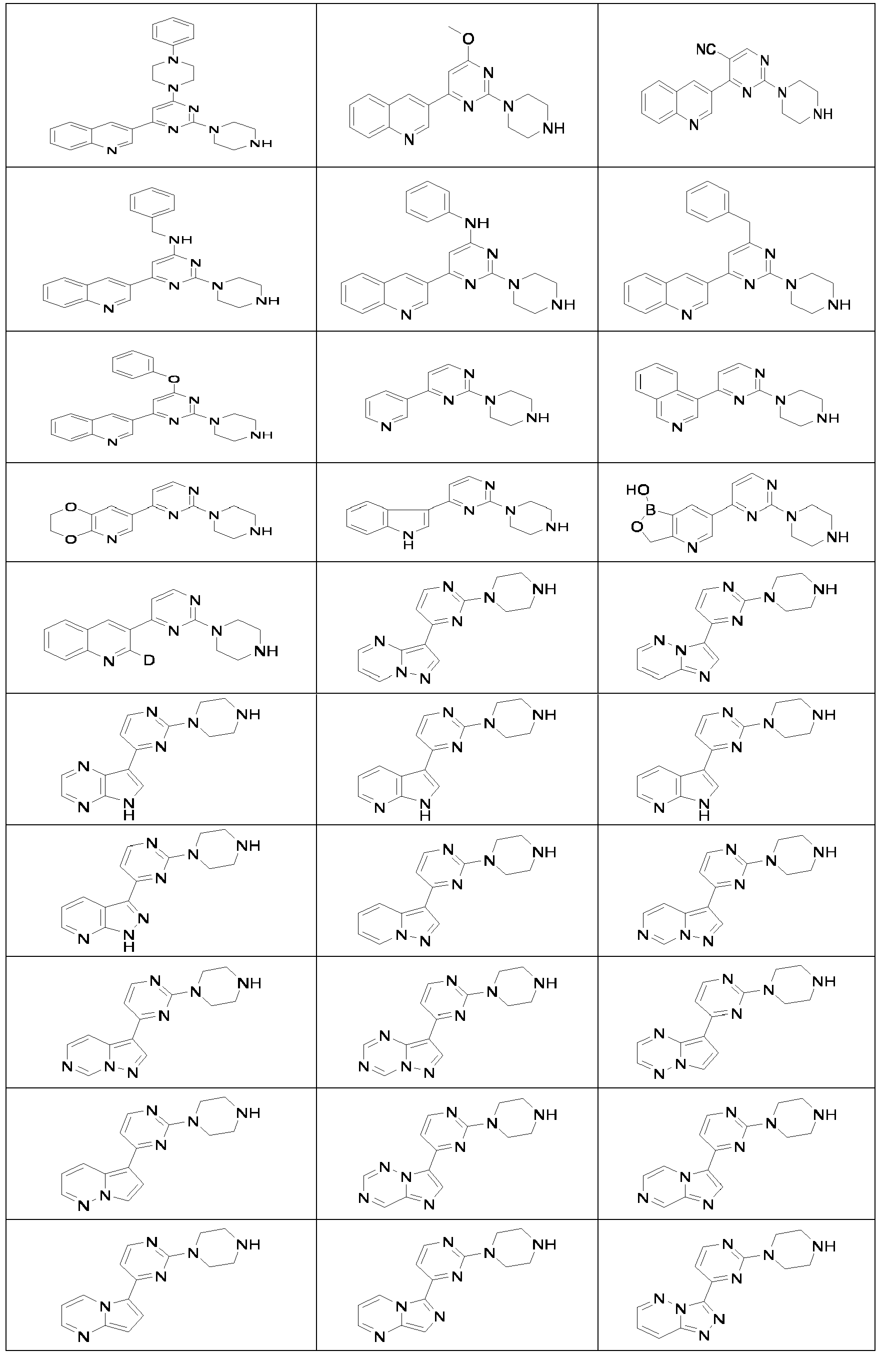

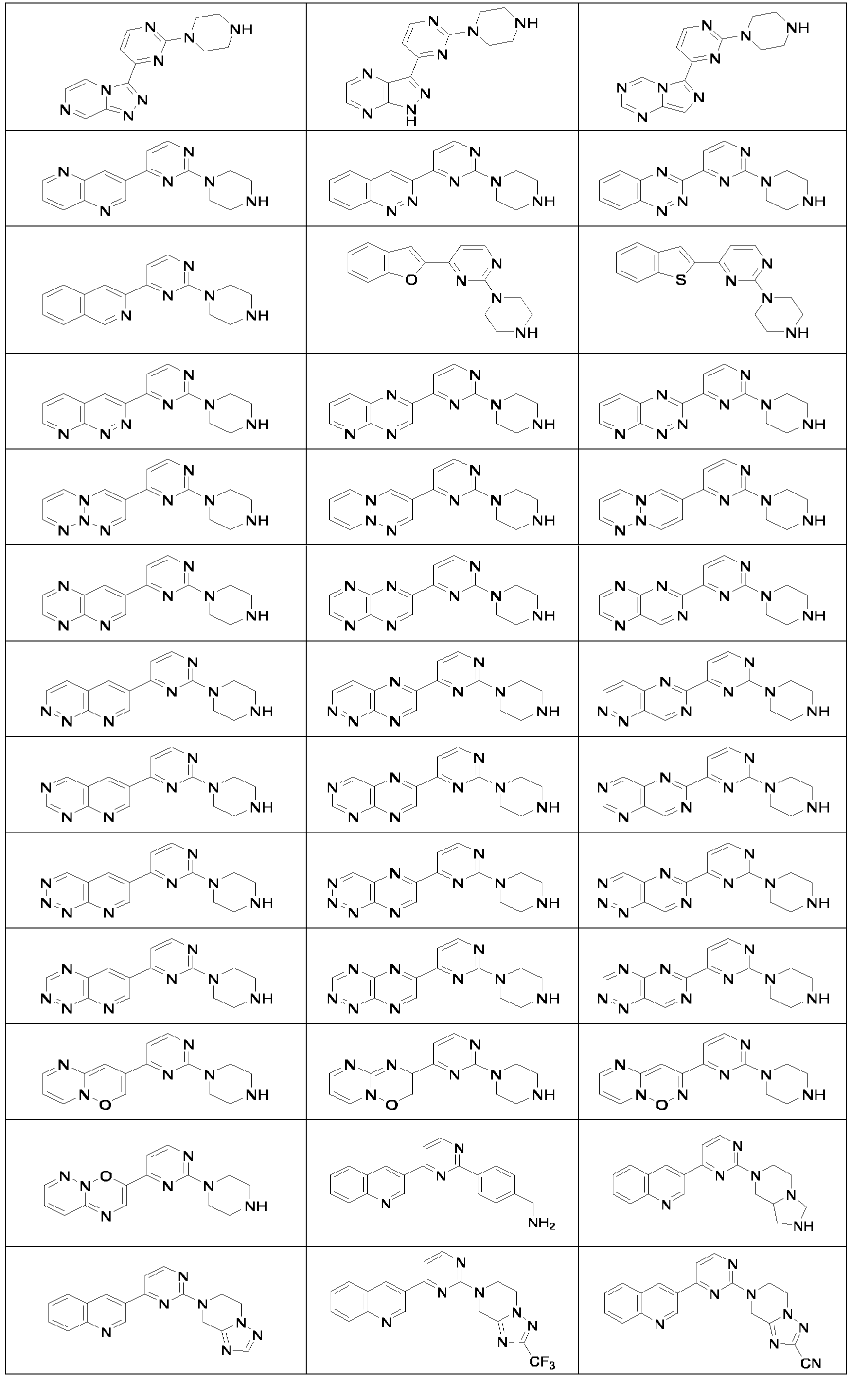

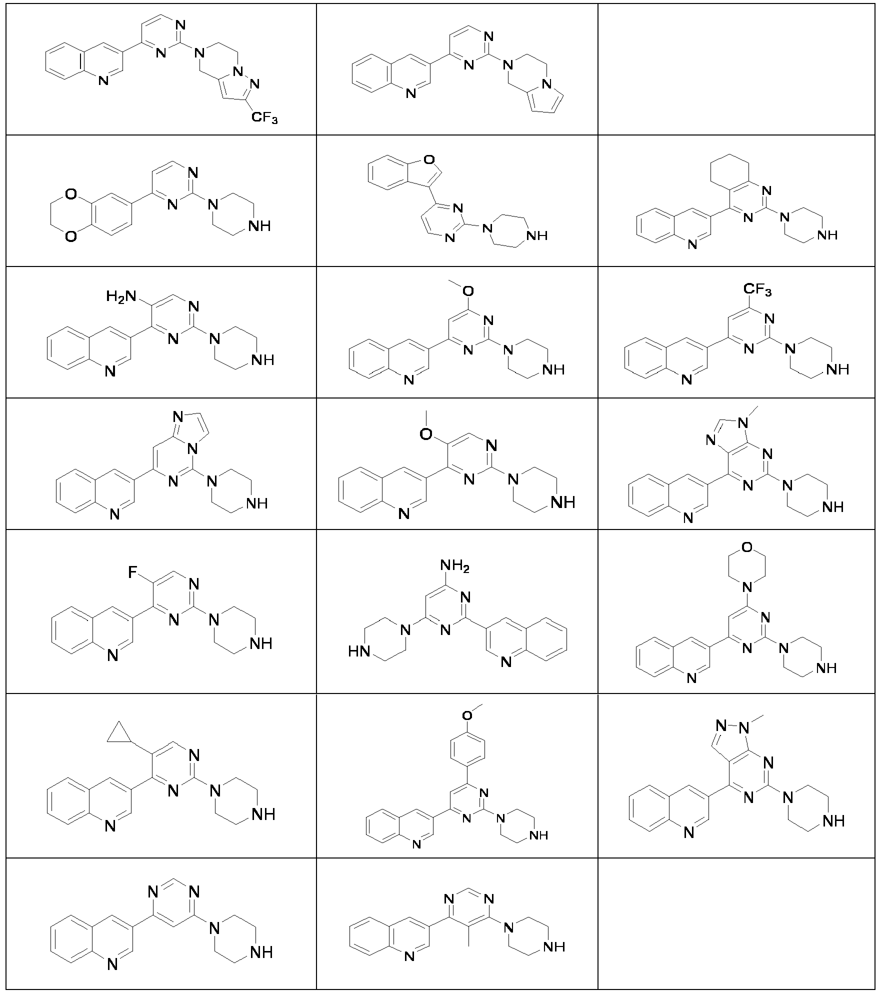

The present invention also provides a pharmaceutical composition, which comprises any of the above compounds or a pharmaceutically acceptable salt, an ester, a deuterated analog, an isomer, a solvate, a prodrug, or an isotopic label thereof and pharmaceutically acceptable excipients.

In one preferred embodiment, the pharmaceutical composition may be formulated, for example, by employing aqueous dispersions, liquid, gels, syrups, elixirs, slurries, suspensions, sprays, controlled-release formulations, instantizing agents, effervescing agents, lyophilized agents, tablets, powders, pills, dragees, capsules, relayed release preparations, sustained-release dosages, pulsed release tablets, microgranules, or immediate release agents.

The present invention also provides any of the above compounds or pharmaceutically acceptable salts, esters, isomers, solvates, prodrugs or isotope markers thereof, or the use of any of the above pharmaceutical compositions in the preparation of drugs for treating CTLA-4 related diseases.

In a preferred embodiment, the CTLA-4 related diseases include cancers, autoimmune diseases, immunodeficiency diseases, viral infections, and organ transplant rejections.

In a more preferred embodiment, the cancer is selected from skin cancer, bladder cancer, breast cancer, pancreatic cancer, bone cancer, brain cancer, neurocytoma, esophageal cancer, labial cancer, laryngeal carcinoma, hypopharynx carcinoma, tongue carcinoma, salivary gland cancer, adenocarcinoma, medullary thyroid cancer, papillary thyroid cancer, choriocarcinoma, pancreatic cancer, urinary cancer, brain tumors such as glioblastoma, astrocytoma, meningioma, medulloblastoma and peripheral neuroectodermal tumors, Hodgkin's lymphoma, non-Hodgkin's lymphoma, Burkitt's lymphoma, adult T-cell leukemia lymphoma, diffuse large B-cell lymphoma (DLBCL), gallbladder cancer, bronchogenic carcinoma, multiple myeloma, basal cell tumor, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing's sarcoma or plasmacytoma, papilloma, blasto glioma, sarcoma (including but not limited to chondrosarcoma, histosarcoma, malignant fibrous histiocytoma, lymphosarcoma and rhabdomyosarcoma), melanoma, hemangioma, keloid, squamous cell carcinoma, astrocytoma, lymphoma (including but not limited to non-Hodgkin's lymphoma, AIDS related lymphoma, cutaneous T-cell lymphoma, Hodgkin's disease, and central nervous system lymphoma), respiratory cancer (including but not limited to lung cancer, such as small cell and non-small cell lung cancer, as well as bronchial adenoma and pleuropulmonary blastoma), head and neck cancer (including but not limited to head cancer, neck cancer, laryngeal carcinoma, hypopharyngeal cancer, nasopharyngeal cancer and/or oropharyngeal cancer, lip and oral cancer), bladder cancer, breast cancer (including but not limited to invasive ductal carcinoma, invasive lobular carcinoma, ductal carcinoma in situ and lobular carcinoma in situ), tumors of the digestive tract (including but not limited to anal cancer, colon cancer, colorectal cancer, esophageal cancer, gallbladder cancer, rectal cancer, gastric cancer, small bowel cancer and salivary gland cancer), thyroid cancer, parathyroid cancer and its remote metastasis, pancreatic cancer, liver cancer (including but not limited to hepatocellular carcinoma, hepatocellular carcinoma with or without fibrolamellar form, cholangiocarcinoma, and mixed hepatocellular cholangiocarcinoma), leukemia (including but not limited to acute lymphoblastic leukemia, chronic lymphoblastic leukemia, acute myeloid leukemia, chronic myeloid leukemia, and villous cell leukemia), brain cancer (including but not limited to brain stem and pituitary glioma, medulloblastoma, cerebellar and cerebral astrocytoma, ependymoma, and neuroectodermal tumor and pineal adenoma), reproductive organ cancer (including but not limited to prostate cancer, testicular cancer, ovarian cancer, endometrial cancer, cervical cancer, endometrial cancer, vaginal cancer and vulval cancer, and uterine sarcoma), urethral cancer, eye cancer (including but not limited to intraocular melanoma and retinoblastoma), skin cancer (including but not limited to Kaposi's sarcoma, squamous cell tumor, malignant melanoma, Merkel cell skin cancer and non-melanoma skin cancer), renal parenchymal carcinoma, renal carcinoma (also known as renal cell cancer and renal adenocarcinoma) and other related cancers.

The features mentioned above, or the features mentioned in the embodiments mentioned herein, may be combined in any combination. All of the features disclosed in this specification may be combined in any combination, and each feature disclosed in this specification may be replaced by any alternative feature serving the same, equivalent or similar purpose. Thus, unless otherwise specified, the features disclosed are only general examples of equivalent or similar features.

The present invention will be described in detail below in connection with specific examples. It should be understood that these examples are only used to describe the present invention and are not intended to limit the scope of the present invention. The experimental methods in the following examples which are not specified with specific conditions are generally carried out according to conventional conditions or according to the conditions recommended by the manufacturer. All percentages, ratios, or parts are calculated by weight, unless otherwise stated.

The units in weight-volume percent in the present invention are well known to those skilled in the art and refer, for example, to the weight of solute in a solution of 100 milliliters.

Unless otherwise defined, all professional and scientific terms used in the text have the same meaning as those familiar to those skilled in the art. In addition, any methods and materials similar or equivalent to those described can be used in the methods of the present invention. The preferred embodiments and materials described herein are exemplary only.

These embodiments are for illustrative purposes only and are not limited to the scope of the claims provided herein.

¹H-NMR spectrum Bruker-400 or OXFORD-AS500 nuclear magnetic resonance instrument, the unit of chemical shift is one millionth, and the internal standard is tetramethylsilane. The coupling constant (J) is close to 0.1Hz. The abbreviations used are indicated as follows: s, single peak; d, double peak; t, triple peak; q, quartet peak; qu, quintet peak; m, multiple peaks; brs, broad peak. Quattro MicroTM API triple quadrupole mass spectrometer was used for mass spectrometry.

### Example 1 Preparation of (1-(4-(quinolin-3-yl)pyrimidin-2-yl)piperidin-4-yl)methylamine (Compound 1)

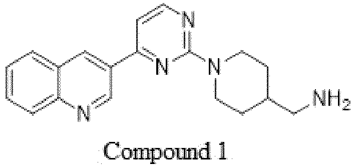

### 3-(2-Chloropyrimidin-4-yl)quinoline

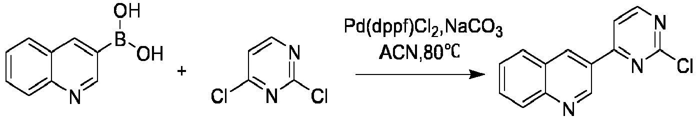

To acetonitrile (100 mL) was added quinoline-3-ylboronic acid (5.0 g, 33.58 mmol) and 2,4-dichloropyrimidine (5.8 g, 33.58 mmol), then 2M sodium carbonate aqueous solution (50 mL), and finally Pd(dppf)Cl₂ (492 mg, 0.67 mmol). When the addition was completed, nitrogen was replaced for three times, and the temperature was raised to 80°C. After the completion of the reaction detected by TLC, the reaction mixture was filtered through celite, and the filtrate was concentrated. Ethyl acetate was added and the layer was separated. The organic phase was evaporated under reduced pressure to remove the solvent, and the residue was purified by flash column chromatography using petroleum ether/ethyl acetate=2/1 as eluent to give the target product (6.2g, 77%).

¹H NMR (400 MHz, *CDCl₃)* δ 9.51 (d, *J* = 4.0 Hz, 1H), 8.95 (d, *J* = 4.0 Hz, 1H), 8.73 (d, *J* = 4.0 Hz, 1H), 8.17 (d, *J* = 8.0 Hz, 1H), 7.97 (d, *J* = 8.0 Hz, 1H), 7.86 - 7.79 (m, 2H), 7.68 - 7.60 (m, 1H).

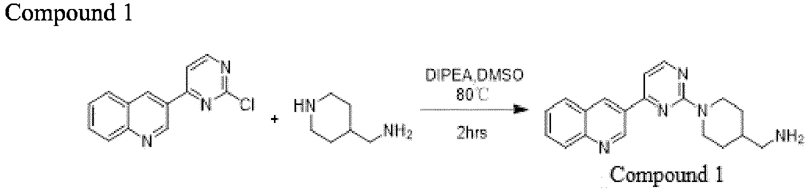

3-(2-Chloropyrimidin-4-yl)quinoline (1.1 g, 4.56 mmol) and piperidin-4-ylmethanamine (0.52 g, 4.56 mmol) were added to DMSO (10 mL), followed by DIPEA (1.2 g, 9.12 mmoL). After the addition was completed, the temperature was raised to 80°C. After 2h, TLC detected the reaction was completed. The reaction solution was added to water (30 mL), extracted with ethyl acetate, and the organic phase was washed with brine and concentrated. The residue was purified by flash column chromatography using dichloromethane/methanol=10/1 as eluent to give the target product (600 mg, 40%)..

¹H NMR (400 MHz, *DMSO-d6)* δ 9.59 (d, *J* = 4.0 Hz, 1H), 9.07 (d, *J* = 4.0 Hz, 1H), 8.50 (d, *J* = 4.0 Hz, 1H), 8.15 (d, *J* = 8.0 Hz, 1H), 8.08 (d, *J* = 8.0 Hz, 1H), 7.87 - 7.81 (m, 1H), 7.74 - 7.65 (m, 1H), 7.38 (d, *J* = 4.0 Hz, 1H), 4.86 (d, *J* = 12.0 Hz, 2H), 3.16 (s, 2H), 3.02 - 2.88 (m, 2H), 1.87 - 1.76 (m, 2H), 1.68 -1.52 (m, 1H), 1.19 - 1.02 (m, 2H).

MS-ESI: 320.5 [M+H]⁺.

### Example 2 Preparation of N-((1-(4-(quinolin-3-yl)pyrimidin-2-yl)piperidin-4-yl)methyl)formamide (Compound 2)

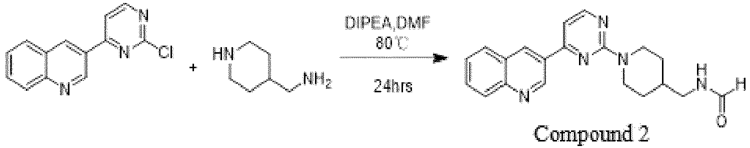

The preparation of **compound 2** was the same as that of **compound 1,** except that the reaction time was 24 hours instead of 2 hours. The yield was 26%.

¹H NMR (400 MHz, *DMSO-d6*) δ 9.58 (d, *J* = 4.0 Hz, 1H), 9.06 (d, *J* = 2.0 Hz, 1H), 8.49 (d, *J* = 4.0 Hz, 1H), 8.13 (d, *J* = 8.0 Hz, 1H), 8.06 (dd, *J* = 12.0, 8.0 Hz, 3H), 7.87 - 7.79 (m, 1H), 7.67 (t, *J* = 8.0 Hz, 1H), 7.37 (d, *J* = 8.0 Hz, 1H), 4.83 (d, *J* = 12.0 Hz, 2H), 3.06 - 3.00 (m, 2H), 2.94 (t, *J* = 12.0 Hz, 2H), 1.75 (d, *J* = 12.0 Hz, 2H), 1.20 - 1.07 (m, 2H).

MS-ESI: 348.6 [M+H]⁺.

### Example 3 Preparation of (1-(4-(quinolin-6-yl)pyrimidin-2-yl)piperidin-4-yl)methylamine (Compound 3)

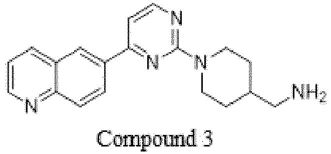

### 6-(2-Chloropyrimidin-4-yl)quinoline

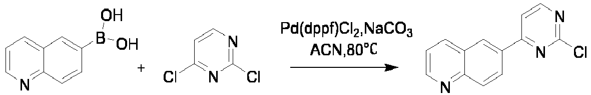

The synthesis of compound 3-(2-chloropyrimidin-4-yl)-6-fluoroquinoline was the same as that of 3-(2-chloropyrimidin-4-yl)quinoline in **Example 1,** except that the quinoline-6-boronic acid compound was used instead of the quinoline-3-boronic acid in **Example 1.** The yield was 81%.

¹H NMR (400 MHz, *CDCl₃)* δ 9.01 (dd, *J* = 4.0, 2.0 Hz, 1H), 8.72 (d, *J* = 8.0 Hz, 1H), 8.68 (d, *J* = 2.0 Hz, 1H), 8.36 (dd, *J* = 8.0, 4.0 Hz, 1H), 8.32 (d, *J* = 8.0 Hz, 1H), 8.24 (d, *J* = 8.0 Hz, 1H), 7.80 (d, *J* = 4.0 Hz, 1H), 7.50 (dd, *J* = 8.0, 4.0 Hz, 1H).

MS-ESI: 242.4 [M+H]⁺.

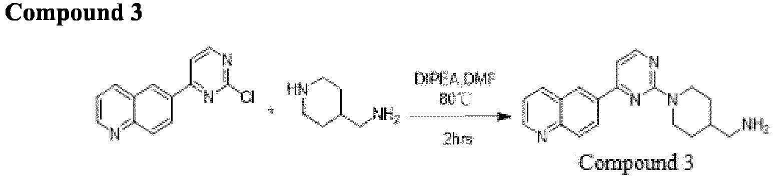

The synthesis of compound 3 was the same as that of compound 1, except that 6-(2-chloropyrimidin-4-yl)quinoline was used instead of 3-(2-chloropyrimidin-4-yl)quinoline. The yield was 46%.

¹H NMR (400 MHz, *DMSO-d6)* δ 8.95 (dd, *J* = 4.0, 2.0 Hz, 1H), 8.77 (d, *J* = 4.0 Hz, 1H), 8.55 - 8.44 (m, 3H), 8.12 (d, *J* = 8.0 Hz, 1H), 7.59 (dd, *J* = 8.0, 4.0 Hz, 1H), 7.32 (d, *J* = 4.0 Hz, 1H), 4.85 (d, *J* = 12.0 Hz, 2H), 2.93 (td, *J* = 12.0, 4.0 Hz, 2H), 2.53 (d, *J* = 4.0 Hz, 2H), 1.89 - 1.76 (m, 2H), 1.70 - 1.57 (m, 1H), 1.20 - 1.06 (m, 2H).

MS-ESI: 320.5 [M+H]⁺.

### Example 4 Preparation of N-((1-(4-(quinolin-6-yl)pyrimidin-2-yl)piperidin-4-yl)methyl)formamide (Compound 4)

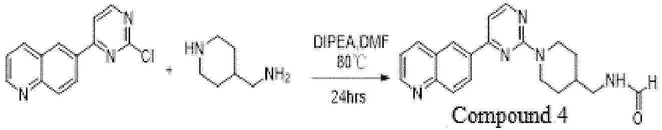

The synthesis of **compound 4** was the same as that of **compound 2** in **Example 2,** except that 6-(2-chloropyrimidin-4-yl)quinoline was used instead of 3-(2-chloropyrimidin-4-yl)quinoline. The yield was 56%.

¹H NMR (400 MHz, *DMSO-d6)* δ 8.95 (dd, *J* = 4.0, 2.0 Hz, 1H), 8.76 (d, *J* = 4.0 Hz, 1H), 8.54 - 8.44 (m, 3H), 8.11 (d, *J* = 8.0 Hz, 1H), 8.03 (s, 1H), 7.59 (dd, *J* = 8.0, 4.0 Hz, 1H), 7.32 (d, *J* = 4.0 Hz, 1H), 4.83 (d, *J* = 12.0 Hz, 2H), 3.03 (t, *J* = 8.0 Hz, 2H), 2.93 (t, *J* = 12.0 Hz, 2H), 178 - 1.70 (m, 3H), 1.20 - 1.05 (m, 2H).

MS-ESI: 348.6 [M+H]⁺.

### Example 5 Preparation of (1-(4-(quinolin-2-yl)pyrimidin-2-yl)piperidin-4-yl)methylamine (Compound 5)

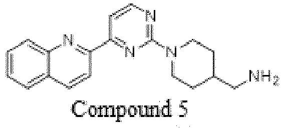

### 1 -(2-(4-(aminomethyl)piperidin-1 -yl)pyrimidin-4-yl)ethan-1 -one

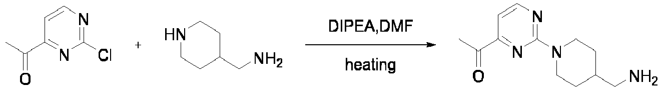

The synthesis of 1-(2-(4-(aminomethyl)piperidin-1-yl)pyrimidin-4-yl)ethan-1-one was the same as that of (1-(4-(quinoline-3)-yl)pyrimidin-2-yl)piperidin-4-yl) methylamine in **Example 1,** except that 1-(2-chloropyrimidin-4-yl)ethan-1-one was used instead of 3-(2-chloropyrimidin-4-yl)quinoline in **Example 4-1.** The yield was 98%.

MS-ESI: 235.4 [M+H]⁺.

### (1-(4-(quinolin-2-yl)pyrimidin-2-yl)piperidin-4-yl)methylamine

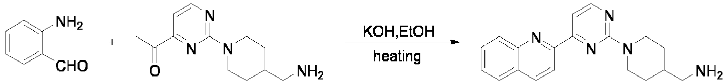

O-aminobenzaldehyde (197 mg, 1.63 mmol) and 1-(2-(4-(aminomethyl)piperidin-1-yl)pyrimidin-4-yl)ethan-1-one (380 mg, 1.63 mmol) ) were dissolved in EtOH (10 mL), and then potassium hydroxide (182 mg, 3.25 mmol) was added. After the addition was completed, the reaction was carried out at 80°C overnight. TLC detected the reaction was completed. The solvent was evaporated under reduced pressure, and ethyl acetate and water were added to the residue. The layers were separated, and the organic phase was washed with water. The solvent was evaporated under reduced pressure, and the residue was purified by flash column chromatography to obtain the product (450 mg, 86%).

¹H NMR (400 MHz, *CDCl₃)* δ 8.50 (t, *J* = 6.8 Hz, 2H), 8.25 (d, *J* = 8.6 Hz, 1H), 8.17 (d, *J* = 8.6 Hz, 1H), 7.84 (d, *J* = 8.0 Hz, 1H), 7.73 (t, *J* = 7.2 Hz, 2H), 7.56 (t, *J* = 7.4 Hz, 1H), 4.97 (d, *J* = 13.2 Hz, 2H), 3.01 - 2.94 (m, 2H), 2.67 (d, *J* = 6.6 Hz, 2H), 2.32 (brs, 2H), 1.89 (d, *J* = 13.2 Hz, 2H), 1.75 -1.65 (m, 1H), 1.32 - 1.19 (m, 2H).

MS-ESI: 320.5 [M+H]⁺.

### Example 6 Preparation of (1-(4-(quinoxalin-2-yl)pyrimidin-2-yl)piperidin-4-yl)methylamine (Compound 6)

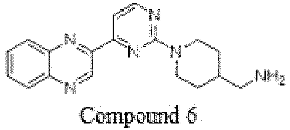

### 2-(2-chloropyrimidin-4-yl)quinoxaline

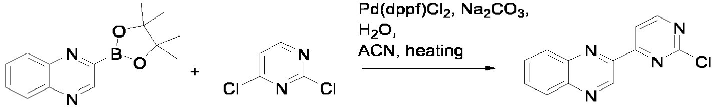

The synthesis of compound 2-(2-chloropyrimidin-4-yl)quinoxaline was the same as that of 3-(2-chloropyrimidin-4-yl)quinoline in **Example 1,** except that 2-(4,4,5,5-tetramethyl-1,3,2-dioxarene-2-yl)quinoxaline was used instead of quinolin-3-ylboronic acid in **Example 1.** The yield was 57%.

MS-ESI: 243.2 [M+H]⁺.

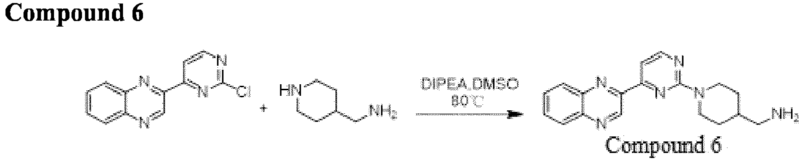

The synthesis of **compound 6** was the same as that of **compound 1** in **Example 1,** except that 2-(2-chloropyrimidin-4-yl)quinoxaline was used instead of 3-(2-chloropyrimidin-4-yl)quinoline in **Example 1.** The yield was 63%.

MS-ESI: 321.2 [M+H]⁺.

### Example 7 Preparation of (1-(4-(6-fluoroquinolin-3-yl)pyrimidin-2-yl)piperidin-4-yl)methylamine (Compound 7)

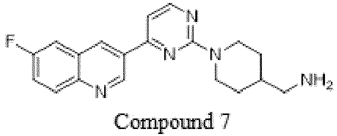

### 3-(2-chloropyrimidin-4-yl)-6-fluoroquinoline

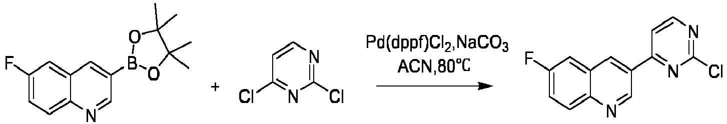

The synthesis of compound 3-(2-chloropyrimidin-4-yl)-6-fluoroquinoline was the same as that of 3-(2-chloropyrimidin-4-yl)quinoline in **Example 1,** except that 6-fluoro-3-(4,4,5,5-tetramethyl-1,3,2-dioxarene-2-yl)quinoline compound was used instead of quinoline-3-ylboronic acid in **Example 1.** The yield was 56%.

¹H NMR (400 MHz, *CD₃OD)* δ 9.54 (d, *J* = 4.0 Hz, 1H), 9.13 (d, *J* = 4.0 Hz, 1H), 8.80 (d, *J* = 8.0 Hz, 1H), 8.17 (d, *J* = 8.0 Hz, 1H), 8.16 - 8.12 (m, 1H), 7.79 (dd, *J* = 8.0, 4.0 Hz, 1H), 7.73 - 7.66 (m, 1H).

MS-ESI: 260.3 [M+H]⁺.

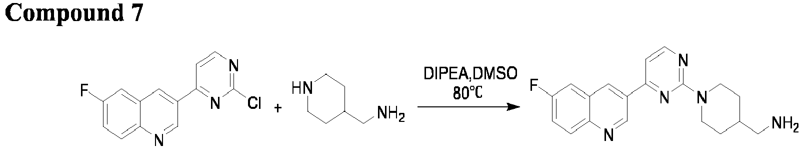

The synthesis of **compound 7** was the same as that of **compound 1** in **Example 1,** except that 3-(2-chloropyrimidin-4-yl)-6-fluoroquinoline was used instead of 3-(2-chloropyrimidin-4-yl)quinoline in **Example 1.** The yield was 73%.

¹H NMR (400 MHz, *CD₃OD)* δ 9.51 (d, *J* = 4.0 Hz, 1H), 8.97 (d, *J* = 4.0 Hz, 1H), 8.44 (d, *J* = 8.0 Hz, 1H), 8.15 - 8.10 (m, 1H), 7.76 (dd, *J* = 12.0, 4.0Hz, 1H), 7.66 (td, *J* = 8.0, 4.0 Hz, 1H), 7.25 (d, *J* = 4.0 Hz, 1H), 4.96 (d, *J* = 12.0 Hz, 2H), 3.00 (td, *J* = 12.0, 4.0 Hz, 2H), 2.66 (d, *J* =8.0 Hz, 2H), 1.89 (d, *J* = 12.0 Hz, 2H), 1.86 - 1.72 (m, 1H), 1.30 - 1.18 (m, 2H).

MS-ESI: 338.4 [M+H]⁺.

### Example 8 (1-(3-(2-(4-(aminomethyl)piperidin-1-yl)pyrimidin-4-yl)quinolin-2-yl)piperidin-4-yl)methylamine (Compound 8)

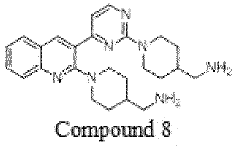

### 3-(2-chloropyrimidin-4-yl)-2-fluoroquinoline

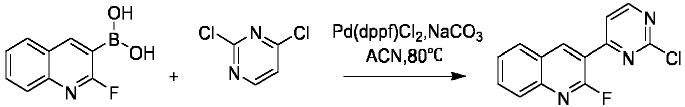

The synthesis of compound 3-(2-chloropyrimidin-4-yl)-6-fluoroquinoline was the same as that of 3-(2-chloropyrimidin-4-yl)quinoline in **Example 1,** except that (2-fluoroquinolin-3-yl)boronic acid compound was used instead of quinolin-3-ylboronic acid in Example 1. The yield was 42%.

¹H NMR (400 MHz, *CD₃OD)* δ 9.28 (d, *J* = 8.0 Hz, 1H), 8.82 (d, *J* = 8.0 Hz, 1H), 8.16 (d, *J* = 8.0 Hz, 1H), 8.05 (dd, *J* = 8.0, 4.0 Hz, 1H), 7.97 -7.85 (m, 2H), 7.69 (t, *J* = 8.0 Hz, 1H).

MS-ESI: 260.3 [M+H]⁺.

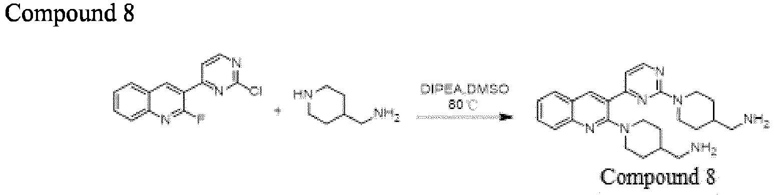

The synthesis of **compound 8** was the same as that of **compound 1** in **Example 1,** except that 3-(2-chloropyrimidin-4-yl)-2-fluoroquinoline was used instead of 3-(2-chloropyrimidin-4-yl)quinoline in **Example 1.** The yield was 73%.

¹H NMR (400 MHz, *CD₃OD* ) δ 8.40 - 8.32 (m, 2H), 7.81 (dd, *J* = 8.0, 4.0 Hz, 2H), 7.65 (t, *J* = 8.0 Hz, 1H), 7.39 (t, *J* = 8.0 Hz, 1H), 7.19 (d, *J* = 8.0 Hz, 1H), 4.96 (d, *J* = 16.0 Hz, 2H), 3.79 (d, *J* = 16.0 Hz, 2H), 3.01 (t, *J* = 12.0 Hz, 2H), 2.93 - 2.85 (m, 6H), 2.08 - 1.96 (m, 1H), 1.94 - 1.74 (m, 5H), 1.46 - 1.26 (m, 4H).

MS-ESI: 432.5 [M+H]⁺.

### Example 9 Preparation of (1-(4-(2-methoxyquinolin-3-yl)pyrimidin-2-yl)piperidin-4-yl)methylamine (Compound 9)

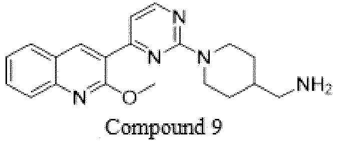

### 3-(2-chloropyrimidin-4-yl)-2-methoxyquinoline

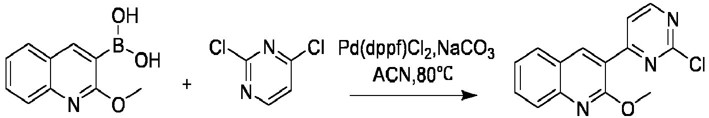

The synthesis of compound 3-(2-chloropyrimidin-4-yl)-2-methoxyquinoline was the same as that of 3-(2-chloropyrimidin-4-yl)quinoline in **Example 1,** except that the compound (2-methoxyquinolin-3-yl)boronic acid was used instead of the quinolin-3-ylboronic acid in **Example 1.** The yield was 52%.

¹H NMR (400 MHz, *CDCl₃*) :δ 9.00 (s, 1H), 8.67 (d, *J* = 4.0 Hz, 1H), 8.17 (d, *J* = 8.0 Hz, 1H), 7.94 -7.85 (m, 2H), 7.72 (t, *J* = 8.0 Hz, 1H), 7.46 (t, *J* = 4.0 Hz, 1H), 4.21 (s, 3H).

MS-ESI: 272.3 [M+H]⁺.

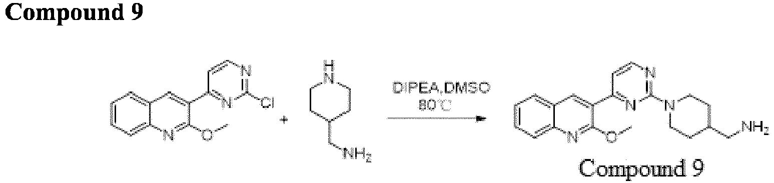

The synthesis of **compound 9** was the same as that of **compound 1** in **Example 1,** except that 3-(2-chloropyrimidin-4-yl)-2-methoxyquinoline was used instead of 3-(2-chloropyrimidin-4-yl)quinoline in **Example 1.** The yield was 76%.

¹H NMR (400 MHz, *CDCl₃):* δ 8.75 (s, 1H), 8.37 (d, *J* = 4.0 Hz, 1H), 7.85 (t, *J* = 7.0 Hz, 2H), 7.65 (t, *J* = 7.6 Hz, 1H), 7.40 (t, *J* = 8.0 Hz, 1H), 7.27 (s, 1H), 4.93 (d, *J* = 16.0 Hz, 2H), 4.15 (s, 3H), 3.71 (q, *J* = 7.0 Hz, 2H), 2.94 (t, *J* = 12.8 Hz, 2H), 2.65 (d, J= 4.0 Hz, 2H), 1.86 (d, *J* = 16.0 Hz, 2H), 1.78 -1.60 (m, 1H), 1.31 -1.17 (m, 2H).

MS-ESI: 350.4 [M+H]⁺.

### Example 10 Preparation of 3-(2-(4-(aminomethyl)piperidin-1-yl)pyrimidin-4-yl)quinolin-2-ol (Compound 10)

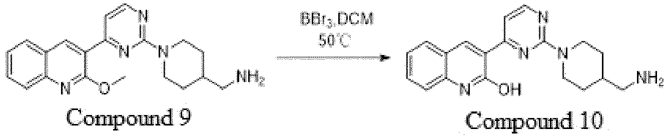

**Compound 9** (300 mg, 0.86 mmol) and DCM (6 mL) were added to a sealed tube, followed by boron tribromide (539 mg, 2.16 mmol). The reaction was heated to 50°C. After the completion of the reaction detected by TLC, water was added for separation, and the organic phase was washed with saturated sodium bicarbonate and water, respectively. The solvent was evaporated under reduced pressure, and the residue was purified by flash column chromatography to obtain **compound 10** (155 mg, 54%).

¹H NMR (400 MHz, *DMSO-d6)* δ 8.86 (s, 1H), 8.39 (d, *J* = 4.0 Hz, 1H), 7.90 (d, *J* = 8.0 Hz, 1H), 7.74 (d, *J* = 4.0 Hz, 1H), 7.60 - 7.54(m, 1H), 7.36 (d, *J* = 8.0 Hz, 1H), 7.25 - 7.9 (m, 1H), 4.78 (d, *J* = 12.0 Hz, 2H), 3.47 - 3.42 (m, 2H), 2.97 - 2.76 (m, 4H), 1.75 (d, *J* = 16.0 Hz, 2H),1.70-1.60(m, 1H).

MS-ESI: 336.4 [M+H]⁺.

### Example 11 Preparation of (1-(4-(quinolin-3-yl-2-d)pyrimidin-2-yl)piperidin-4-yl)methylamine (Compound 11)

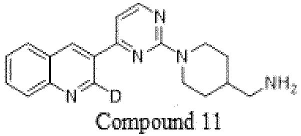

### 3-(2-chloropyrimidin-4-yl)quinoline-2-d

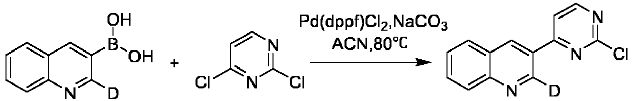

The synthesis of 3-(2-chloropyrimidin-4-yl)quinoline-2-d was the same as that of 3-(2-chloropyrimidin-4-yl)quinoline in **Example 1,** except that (quinolin-3-yl-2-d)boronic acid was used instead of quinolin-3-ylboronic acid in **Example 1.** The yield was 32%.

¹H NMR (400 MHz, *CD₃OD)* δ 9.01 (s, 1H), 8.43 (d, *J* = 4.0 Hz, 1H), 8.09 (d, *J* = 4.0 Hz ,2H), 7.90 - 7.78 (m, 1H), 7.73 - 7.64 (m, 1H), 7.27 (d, *J* = 4.0 Hz, 1H).

MS-ESI: 243.2 [M+H]⁺.

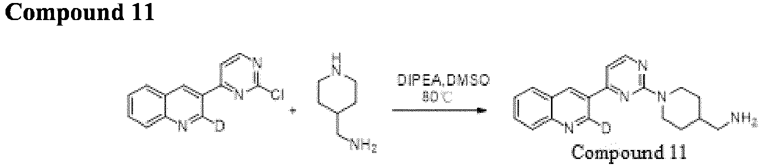

The synthesis of **compound 11** was the same as that of **compound** 1 in **Example 1,** except that 3-(2-chloropyrimidin-4-yl)quinoline-2-d was used instead of 3-(2-chloropyrimidin-4-yl)quinoline in **Example 1.** The yield was 67%.

¹H NMR (400 MHz, *CD₃OD)* δ 9.01 (s, 1H), 8.43 (d, *J* = 4.0 Hz, 1H), 8.13 - 8.03 (m, 2H), 7.89 - 7.81 (m, 1H), 7.72 - 7.63 (m, 1H), 7.27 (d, *J* = 4.0 Hz, 1H), 4.96 (d, *J* = 16.0 Hz, 2H), 2.99 (td, *J* = 12.0, 4.0 Hz, 2H), 2.59 (d, *J* = 8.0 Hz, 2H), 1.94 - 1.84 (m, 2H), 1.80 - 1.66 (m, 1H), 1.27 - 1.19 (m, 2H).

MS-ESI: 321.3 [M+H]⁺.

### Example 12 Preparation of (1-(4-(quinolin-3-yl)pyridin-2-yl)piperidin-4-yl)methylamine (Compound 12)

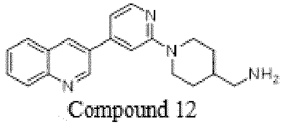

### 3-(2-chloropyridin-4-yl)quinoline

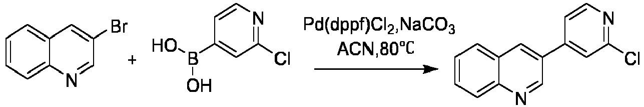

The synthesis of **compound 12** was the same as that of 3-(2-chloropyrimidin-4-yl)quinoline in **Example 1,** expect that the compound 3-bromoquinoline was used instead of quinoline-3-ylboronic acid in **Example 1** and (2-chloropyrimidin-4-yl)boronic acid was used instead of 2,4-dichloropyrimidine in **Example 4-1.** The yield was 65%.

¹H NMR (400 MHz, *DMSO-d6)* δ 9.37 (d, *J* = 4.0 Hz, 1H), 8.94 (d, *J* = 4.0 Hz, 1H), 8.57 (d, *J* = 4.0 Hz, 1H), 8.13 (s, 1H), 8.10 (d, *J* = 12.0 Hz, 2H), 8.00 (dd, *J* = 8.0, 4.0 Hz, 1H), 7.89 - 7.83 (m, 1H), 7.71 (t, *J* = 8.0 Hz, 1H).

MS-ESI: 241.2 [M+H]⁺.

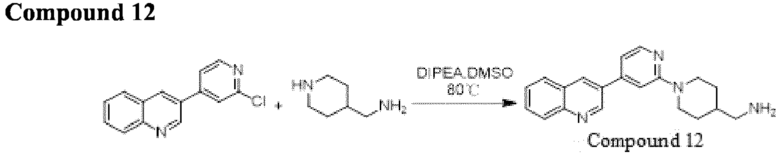

The synthesis of **compound 12** was the same as that of **compound 1** in **Example 1,** except that 3-(2-chloropyridin-4-yl)quinoline was used instead of 3-(2-chloropyrimidin-4-yl)quinoline in **Example 1.** The yield was 64%.

¹H NMR (400 MHz, *DMSO-d6)* δ 9.29 (d, *J* = 4.0 Hz, 1H), 8.77 (d, *J* = 4.0 Hz, 1H), 8.22 (d, *J* = 8.0 Hz, 1H), 8.06 (d, *J* = 12.0 Hz, 2H), 7.80 (t, *J* = 8.0 Hz, 1H), 7.66 (t, *J* = 8.0 Hz, 1H), 7.28 (s, 1H), 7.10 (d, *J* = 4.0 Hz, 1H), 4.49 (d, *J* = 12.0 Hz, 2H), 2.84 (t, *J* = 12.0 Hz, 2H), 2.63 (d, *J* = 4.0 Hz, 2H), 1.78 (d, *J* = 12.0 Hz, 2H), 1.75 - 1.65 (m, 1H), 1.26 - 1.14 (m, 2H).

MS-ESI: 319.3 [M+H]⁺.

### Example 13 Preparation of 2-(2-(4-(aminomethyl)piperidin-1-yl)pyrimidin-4-yl)-4hydro-benzopyran-4-one (Compound 13)

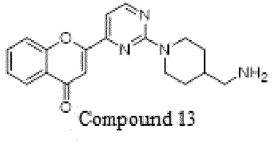

### methyl 2-(4-((t-butyloxycarbonyl)amino)methyl)piperidin-1-yl)pyrimidine-4-carboxylate

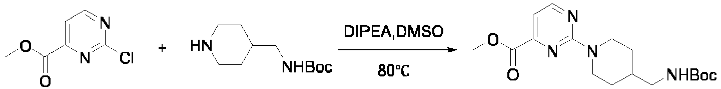

The synthesis of 2-(4-((t-butyloxycarbonyl)amino)methyl)piperidin-1-yl)pyrimidine-4-carboxylicacid methyl ester was the same as that of (1-(4-(quinolin-3-yl)pyrimidin-2-yl)piperidin-4-yl)methylamine in **Example 1,** except that 2-chloropyrimidin-4-carboxylicacid methyl ester was used instead of 3-(2-chloropyrimidin-4-yl)quinoline in **Example 1.** The yield was 88%.

MS-ESI: 351.2[M+H]⁺.

### tert-butyl ((1-(4-(hydroxymethyl)pyrimidin-2-yl)piperidin-4-yl)methyl) carbamate

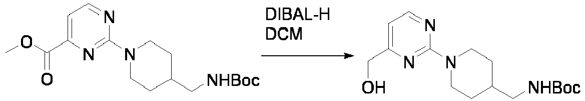

Methyl 2-(4-(((t-butyloxycarbonyl)amino)methyl)piperidin-1-yl)pyrimidine-4-carboxylate (1.56 g, 4.5 mmol) was dissolved in DCM (30 mL) and stirred. DIBAL-H (4.5 mL, 6.7 mmol) was added slowly at -70°C. The next day TLC detected that there were still raw materials left, then DIBAL-H (3 mL) was added. After 3h, the reaction was complete, and MeOH (5 mL) was added to quench the reaction. The reaction mixture was washed with saturated NaHCO₃ and concentrated under reduced pressure, and the residue was purified by flash column chromatography to obtain the target product (760 mg, 55%).

¹H NMR (400 MHz, *CDCl₃)* : δ 8.21 (dd, *J* = 8.0, 0.6 Hz, 1H), 6.35 (d, *J* = 4.0 Hz, 1H), 4.79 (d, *J* = 16.0 Hz, 2H), 4.65 (s, 1H), 4.54 (d, *J* = 4.0 Hz, 2H), 3.73-3.71 (m, 1H), 3.05-3.02 (m, 2H), 2.86 (t, *J* =12.0, 7.6 Hz, 2H), 1.79-1.75 (m, 2H), 1.45 (s, 9H), 1.18 (dd, *J* = 12.2, 3.8 Hz, 2H).

MS-ESI: 323.4 [M+H]⁺.

### tert-butyl ((1-(4-formylpyrimidin-2-yl)piperidin-4-yl)methyl) carbamate

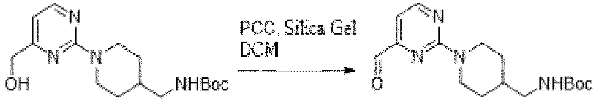

tert-butyl ((1-(4-formylpyrimidin-2-yl)piperidin-4-yl)methyl) carbamate was dissolved in DCM (30 mL) and then PCC (1.5g, 7.0mmol) and silica gel (1.5g) were mixed and added to the reaction system. After 3h, TLC detected the reaction was completed. The reaction mixture was filtered and the filtrate was separated. The organic phase was washed with brine, concentrated under reduced pressure, and the residue was purified by flash column chromatography to obtain the target product (360 mg, 46%) .

¹H NMR (400 MHz, *CDCl₃)* :δ 9.81 (s, 1H), 8.50 (d, *J* = 4.0 Hz, 1H), 6.92 (d, *J* = 4.0 Hz, 1H), 4.87 (d, *J* = 12.0 Hz, 2H), 3.07-3.04 (m, 2H), 2.86 (t, *J* =12.0, 7.6 Hz, 2H), 1.87 - 1.74 (m, 3H), 1.45 (s, 9H), 1.29 -1.11 (m, 2H).

MS-ESI: 321.6 [M+H]⁺.

### tert-butyl ((1-(4-(1-hydroxy-3-(2-hydroxyphenyl)-3-oxypropyl)pyrimidin-2-yl)piperidin-4-yl)methyl) carbamate

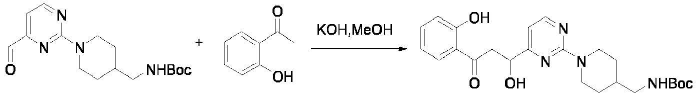

tert-butyl ((1-(4-formylpyrimidin-2-yl)piperidin-4-yl)methyl) carbamate (360 mg, 0.1 mmol) and 1-(2-hydroxyphenyl)ethane-1-one (184 mg, 0.1 mmol) was dissolved in MeOH (20 mL), then KOH (126 mg, 0.2 mmol) was added. After 3h, TLC detected the reaction was completed. The pH was adjusted to 6-7 with 2M HCl. The solvent was evaporated under reduced pressure, and the residue was dried under high vacuum to obtain a crude product which was used directly without further purification.

MS-ESI: 457.5 [M+H]⁺.

### tert-butyl ((1-(4-(4-oxo-4H-chromen-2-yl)pyrimidin-2-yl)piperidin-4-yl)methyl) carbamate

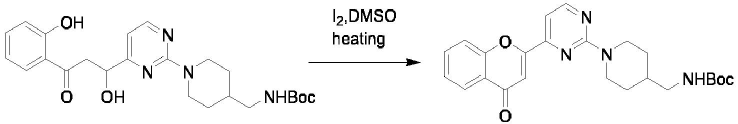

tert-butyl ((1-(4-(1-hydroxy-3-(2-hydroxyphenyl)-3-oxypropyl)pyrimidin-2-yl)piperidin-4-yl)methyl) carbamate was dissolved in DMSO (6mL), and I₂ (5mg) was added. After the addition was completed, the reaction was raised to 100°C. TLC detected the reaction was basically completed on the next day, then water was added, and the mixture was extracted with DCM. The organic phase was washed with saturated sodium bicarbonate, and the solvent was evaporated under reduced pressure. The residue was purified by flash column chromatography to give the target product (40 mg, 38%).

¹H NMR (400 MHz, *CDCl₃)* :*δ* 8.41 (d, *J* = 4.0 Hz, 1H), 7.82-7.79 (m, 1H), 7.71-7.67 (m, 1H), 7.32 (d, *J* = 8.0 Hz, 1H), 7.29 -7.27 (m, 1H), 7.24 -7.21 (m, 1H), 6.77-6.72 (m, 1H), 4.89-4.79 (m, 2H), 3.78 - 3.66 (m, 1H), 3.08-3.02 (m, 2H), 2.92-2.86 (m, 2H), 1.80 (d, *J* = 12.0 Hz, 2H), 1.44 (s, 9H), 1.33-1.15 (m, 2H).

MS-ESI: 437.4 [M+H]⁺.

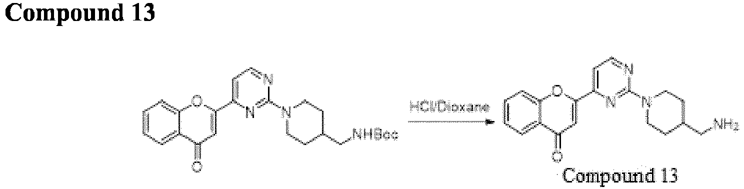

tert-butyl ((1-(4-(4-oxo-4H-chromen-2-yl)pyrimidin-2-yl)piperidin-4-yl)methyl) carbamate was dissolved in HCl-Dioxane (6 mL), and TLC detected the reaction was completed after 1h. The solvent was evaporated under reduced pressure, and saturated sodium bicarbonate was added to adjust the pH to 6-7. The residue was purified by flash column chromatography to obtain **compound 13** (20 mg, 65%).

¹H NMR (400 MHz, *CD₃OD*) :*δ* 8.44 (d, *J* = 4.0 Hz, 1H), 7.81 (t, *J* = 7.4Hz, 2H), 7.45 (d, *J* = 8.0 Hz, 1H), 7.36 (dd, *J* = 16.0, 6.4 Hz, 2H), 6.61 (s, 1H), 4.59 (s, 2H), 2.95 (t, *J* = 12.0, 7.4 Hz, 2H), 2.87 (d, *J* = 8.0 Hz, 2H), 2.0-1.90 (m, 1H), 1.86 (d, *J* = 12.8 Hz, 2H), 1.35- 1.22 (m, 2H).

MS-ESI: 337.3 [M+H]⁺.

### Example 14 Preparation of (1-(6-(quinolin-3-yl)pyridin-2-yl)piperidin-4-yl)methylamine (Compound 14)

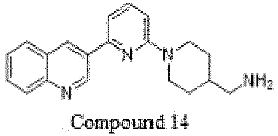

### (1 -(6-bromopyridin-2-yl)piperidin-4-yl)methylamine

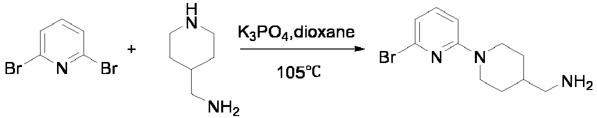

2,6-dibromopyridine (1.7 g, 7.17 mmol) and piperidin-4-ylmethylamine (0.9 g, 7.88 mmol) were dissolved in 1,4-dioxane (15 mL), and potassium phosphate (1.52 g, 7.17 mmol) was added. Then nitrogen was replaced for three times, and the reaction was heated to 105°C. After 8h, the reaction was basically completed, the mixture was concentrated, and ethyl acetate and water were added. The solution was separated, and the organic phase was evaporated under reduced pressure to remove the solvent. The residue purified by flash column chromatography to obtain the product (1.36 g, 70%).

¹H NMR (400 MHz, *DMSO-d6)* δ 7.41 - 7.33 (m, 1H), 6.77 (d, *J* = 12.0 Hz, 1H), 6.70 (d, *J* = 8.0 Hz, 1H), 4.21 (d, *J* = 16.0 Hz, 2H), 2.76 (td, *J* = 12.0, 4.0 Hz, 2H), 2.42 (d, *J* = 8.0 Hz, 2H), 1.73 (d, *J* = 12.0 Hz, 2H), 1.55 - 1.40 (m, 1H), 1.03 (qd, *J* = 12.0, 4.0 Hz, 2H).

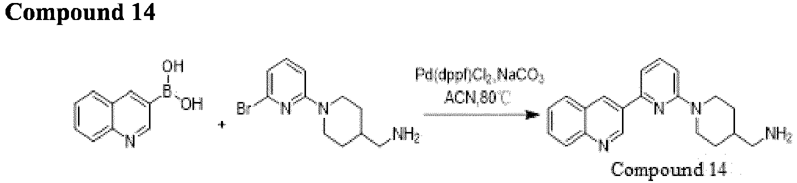

The synthesis of **compound 14** was the same as that of the **compound 1** in **Example 1,** except that the compound (1-(6-bromopyridin-2-yl)piperidin-4-yl) methylamine was used instead of 2,4-dichloropyrimidine in **Example 1.** The yield was 42%.

¹H NMR (400 MHz, *DMSO-d6)* δ 9.57 (d, *J* = 4.0 Hz, 1H), 8.93 (d, *J* = 4.0 Hz, 1H), 8.11 (d, *J* = 8.0 Hz, 1H), 8.04 (d, *J* = 8.0 Hz, 1H), 7.82 - 7.74 (m, 1H), 7.73 - 7.60 (m, 2H), 7.42 (d, *J* = 4.0 Hz, 1H), 6.92 (d, *J* = 8.0 Hz, 1H), 4.50 (d, *J* = 12.0 Hz, 2H), 2.88 (t, *J* = 12.0 Hz, 3H), 2.69 (d, *J* = 4.0 Hz, 3H), 1.95 - 1.85 (d, *J* = 11.3 Hz, 3H), 1.33 - 1.14 (m, 2H).

MS-ESI: 319.3 [M+H]⁺.

### Example 15 Preparation of (1-(3-(quinolin-3-yl)phenyl)piperidin-4-yl)methylamine (Compound 15)

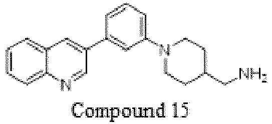

### 3-(3-bromophenyl)quinoline

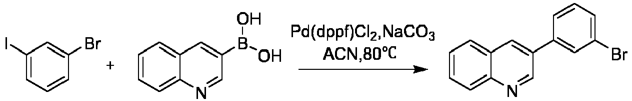

The synthesis of **compound 15** was the same as that of 3-(2-chloropyrimidin-4-yl)quinoline in **Example 1,** except that compound 1-bromo-3-iodobenzene was used instead of 2,4-dichloropyrimidine in **Example 1.** The yield was 82%.

¹H NMR (400 MHz, *DMSO-d6)* δ 9.26 (d, *J* = 4.0 Hz, 1H), 8.71 (d, *J* = 4.0 Hz, 1H), 8.11 (t, *J* = 4.0 Hz, 1H), 8.06 (d, *J* = 8.0 Hz, 2H), 7.91 (d, *J* = 8.0 Hz, 1H), 7.82 - 7.76 (m, 1H), 7.69 - 7.63 (m, 2H), 7.51 (t, *J* = 8.0 Hz, 1H).

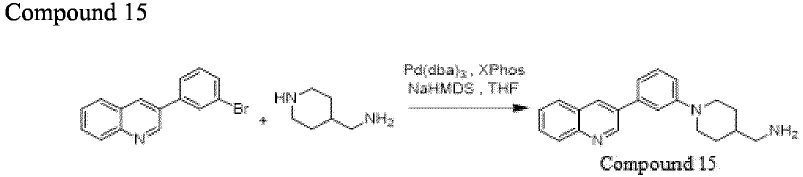

To a mixture of 3-(3-bromophenyl)quinoline (630 mg, 2.22 mmol), piperidin-4-ylmethylamine (380 mg, 3.33 mmol) and XPhos (21 mg, 0.044 mmol) in THF (10 mL) was added 2.0M NaHMDS (1.42 g, 7.77 mmol) slowly, and finally Pd(dba)₃ (41 mg, 0.044 mmol). Then nitrogen was replaced for three times. The reaction was carried out at 70°C for 7h and TLC detected the reaction was basically completed. The reaction mixture was neutralized by adding hydrochloric acid, and then concentrated. DCM and water were added. The organic phase was evaporated under reduced pressure to remove the solvent, and the residue was purified by flash column chromatography to give the product (150 mg, 21%).

¹H NMR (400 MHz, *DMSO-d6*) *δ* 9.23 (d, *J* = 4.0 Hz, 1H), 8.64 (d, *J* = 4.0 Hz, 1H), 8.05 (t, *J* = 8.0 Hz, 1H), 7.76 (d, *J* = 8.0 Hz, 2H), 7.67 - 7.61 (m, 1H), 7.38 (d, *J* = 4.0 Hz, 1H), 7.23 (d, *J* = 8.0 Hz, 1H), 7.10 (d, *J* = 12.0 Hz, 1H), 7.06 - 6.98 (m, 1H), 3.87 (d, *J* = 12.0 Hz, 2H), 2.80 - 2.64 (m, 4H), 1.90 - 1.80 (m , 3H),1.38 - 1.26 (m , 2H).

### Example 16 Preparation of 3-(2-(4-(aminomethyl)piperidin-1-yl)pyrimidin-4-yl)quinolin-2-amine (Compound 16)

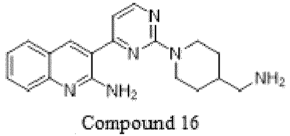

### 3-(2-chloropyrimidin-4-yl)quinolin-2-amine

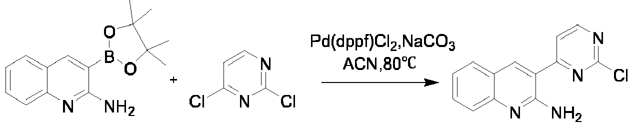

The synthesis of compound 3-(2-chloropyrimidin-4-yl)quinolin-2-amine was the same as that of 3-(2-chloropyrimidin-4-yl)quinoline in **Example 1,** except that compound 3-(4,4,5,5-tetramethyl-1,3,2-dioxybenzaldehyde-2-yl)quinolin-2-amine was used instead of quinolin-3-ylboronic acid in **Example 1.** The yield was 64%.

¹H NMR (400 MHz, *CDCl₃)* δ 8.92 (d, *J* = 8.0 Hz, 1H), 8.51 (d, *J* =4.0 Hz, 1H), 8.14 (s, 1H), 8.08 (d, *J* = 8.0 Hz, 1H), 7.81 (d, *J* = 8.0 Hz, 2H), 7.57 (t, *J* = 8.0 Hz, 1H), 7.39 (s, 1H).

MS-ESI: 257.3 [M+H]⁺.

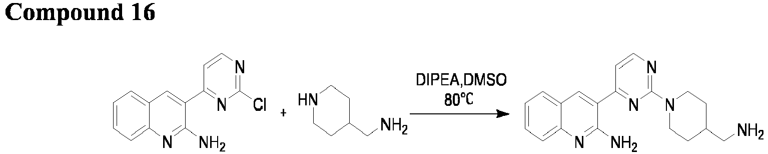

The synthesis of **compound 16** was the same as that of **compound 1** in **Example 1,** except that 3-(2-chloropyrimidin-4-yl)quinolin-2-amine was used instead of 3-(2-chloropyrimidin-4-yl)quinoline in **Example 1.** The yield was 82%.

¹H NMR (400 MHz, *DMSO-d6*) *δ* 8.29 (s, 1H), 8.12 (s, 1H), 7.85 (d, *J* = 8.0 Hz, 2H), 7.79 - 7.68 (m, 1H), 7.66 - 7.58 (m , 1H),7.54 - 7.46 (m, 1H), 4.73 (d, *J* = 12.0 Hz, 2H), 3.11 (t, *J* = 12.0 Hz, 2H), 2.90 (d, *J* = 8.0 Hz, 2H), 2.09 - 1.99 (m, 1H), 1.92 (d, *J* = 16.0 Hz, 2H), 1.38 (dd, *J* = 12.0, 4.0 Hz, 2H).

MS-ESI: 335.4 [M+H]⁺.

### Example 17 Preparation of 1-(4-(quinolin-3-yl)pyrimidin-2-yl)piperidine-4-amine (Compound 17)

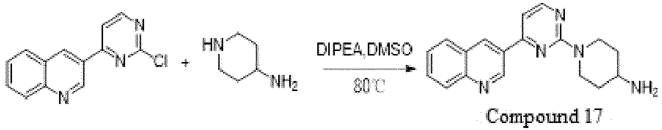

The synthesis of **compound 17** was the same as that of **compound 1** in **Example 1,** except that piperidin-4-amine was used instead of piperidin-4-ylmethylamine in **Example 1.** The yield was 65%.

¹H NMR (400 MHz, DMSO-*d6*) δ 9.63 (d, *J =* 4.0 Hz, 1H), 9.11 (d, *J* = 4.0 Hz, 1H), 8.54 (d, *J* = 4.0 Hz, 1H), 8.18 (d, *J* = 8.0 Hz, 1H), 8.11 (d, *J* = 8.0 Hz, 1H), 7.91 - 7.84 (m, 1H), 7.71 (t, *J* = 8.0 Hz, 1H), 7.42 (d, *J* = 4.0 Hz, 1H), 4.75 (d, *J* = 8.0 Hz, 2H), 3.11 (t, *J* = 12.0 Hz, 2H), 3.04 - 2.94 (m, 1H), 1.89 (d, *J* = 8.0 Hz, 2H), 1.40 - 1.20 (m, 2H).

MS-ESI: 306.3 [M+H]⁺.

### Example 18 Preparation of 3-(2-(piperazin-1-yl)pyrimidin-4-yl)quinoline (Compound 18)

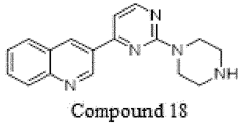

### tert-butyl 4-(4-(quinolin-3-yl)pyrimidin-2-yl)piperazine-1 carboxylate

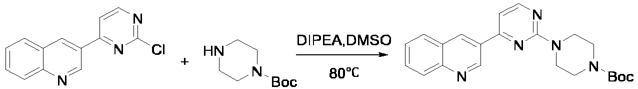

The synthesis of tert-butyl 4-(4-(quinolin-3-yl)pyrimidin-2-yl)piperazin-1 carboxylate was the same as that of (1-(4-(quinolin-3-yl)pyrimidin-2-yl)piperidin-4-yl)methylamine in **Example 1,** except that tert-butyl piperazin-1 carboxylate was used instead of piperidin-4-ylmethylamine in **Example 1.** The yield was 95%.

MS-ESI: 392.2 [M+H]⁺.

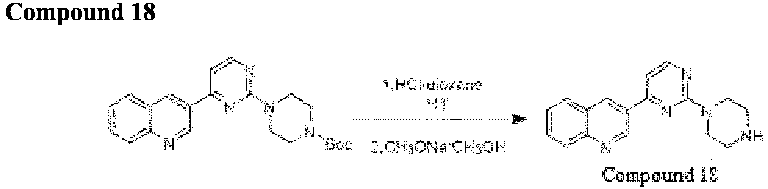

4M hydrochloric acid/1,4-dioxane solution (50 mL) was added to tert-butyl 4-(4-(quinolin-3-yl)pyrimidin-2-yl)piperazine-1 carboxylate (6.1 g, 15.56 mmol), and stirred at room temperature for 30 minutes. TLC detected the reaction was completed. The reaction system was concentrated, and the methanol solution of sodium methoxide was added to free the product. The solvent was evaporated under reduced pressure, and the residue was purified by flash column chromatography to obtain the product (4.55 g, 98%).

¹H NMR (400 MHz, *DMSO-d6*) *δ* 9.65 (d, *J* = 4.0 Hz, 1H), 9.15 (d, *J* =4.0 Hz, 1H), 8.60 (d, *J* =8.0Hz, 1H), 8.17 - 8.12 (m, 1H), 8.09 (d, *J* = 8.0 Hz, 1H), 7.86 (t, *J* = 8.0 Hz, 1H), 7.70 (t, *J* = 8.0 Hz, 1H), 7.56 (d, *J* = 8.0 Hz, 1H), 4.15 - 4.08 (m, 4H), 3.25 - 3.12 (m, 4H).

MS-ESI: 292.2 [M+H]⁺.

### Example 19 Preparation of 2-(1-(4-(quinolin-3-yl)pyrimidin-2-yl)piperidin-4-yl)ethylamine (Compound 19)

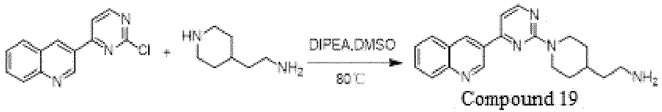

The synthesis of **compound 19** was the same as that of **compound 1** in **Example 1,** except that piperidin-4-ylethylamine was used instead of piperidin-4-ylmethylamine in **Example 1.** The yield was 36%.

¹H NMR (400 MHz, DMSO-*d6*) δ 9.60 (d, *J* = 4.0 Hz, 1H), 9.09 (d, *J* = 4.0 Hz, 1H), 8.51 (d, *J* = 4.0 Hz, 1H), 8.15 (d, *J* = 8.0 Hz, 1H), 8.09 (d, *J* = 8.0 Hz, 1H), 7.88-7.83 (m, 1H), 7.69 (t, *J* = 8.0 Hz, 1H), 4.86 (d, *J* = 12.0 Hz, 2H), 2.98 - 2.92 (m, 2H), 2.90 - 2.83 (m, 2H), 2.54 (s, 2H), 1.79 (d, *J* = 8.0 Hz, 2H), 1.54 -1.48 (m, 1H), 1.20 - 1.12(m, 2H).

MS-ESI: 334.2 [M+H]⁺.

### Example 20 Preparation of (1-(4-(quinolin-3-yl)pyrimidin-2-yl)piperidin-4-yl)methanol (Compound 20)

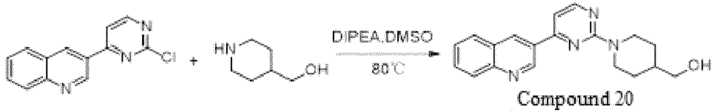

The synthesis of **compound 20** was the same as that of **compound 1** in **Example 1,** except that piperidin-4-ylmethanol was used instead of piperidin-4-ylmethylamine in **Example** 1. The yield was 83%.

¹H NMR (400 MHz, *DMSO-d6*) *δ* 9.58 (d, J = 4.0 Hz, 1H), 9.07 (d, *J* = 4.0 Hz, 1H), 8.49 (d, *J* = 4.0 Hz, 1H), 8.14 (d, *J* = 8.0 Hz, 1H), 8.07 (d, *J* = 8.0 Hz, 1H), 7.88 - 7.79 (m, 1H), 7.73 - 7.63 (m, 1H), 7.36 (d, *J* = 4.0 Hz, 1H), 4.85 (d, *J* = 12.0 Hz, 2H), 4.51 (t, J = 8.0 Hz, 1H), 3.31 - 3.24 (m, 2H), 2.98 - 2.86 (m, 2H), 1.76 (d, *J* = 12.0 Hz, 2H), 1.72 - 1.63 (m, 1H), 1.11 (qd, *J* = 12.0, 4.0 Hz, 2H).

MS-ESI: 321.2 [M+H]⁺.

### Example 21 Preparation of (1-(4-(quinolin-3-yl)pyrimidin-2-yl)piperidin-4-yl)ethanol (Compound 21)

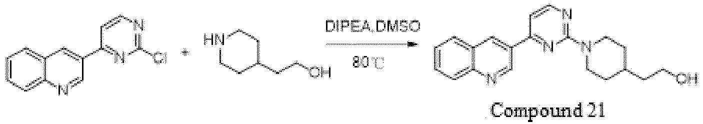

The synthesis of **compound 21** was the same as that of **compound 1** in **Example 1,** except that piperidin-4-ylethanol was used instead of piperidin-4-ylmethylamine in **Example 1.** The yield was 49%.

¹H NMR (400 MHz, *DMSO-d6*) *δ* 9.57 (d, *J* = 4.0 Hz, 1H), 9.05 (d, *J* = 1.9 Hz, 1H), 8.48 (d, *J* = 4.0 Hz, 1H), 8.13 (d, *J* = 8.0 Hz, 1H), 8.07 (d, *J* = 8.0 Hz, 1H), 7.88 - 7.77 (m, 1H), 7.67 (t, *J* = 8.0 Hz, 1H), 7.35 (d, *J* = 8.0 Hz, 1H), 4.81 (d, *J* = 12.0 Hz, 2H), 4.40 (s, 1H), 3.47 (d, *J* = 4.0 Hz, 1H), 2.91 (t, *J*= 12.0 Hz, 1H), 1.82 - 1.63 (m, 3H), 1.38 (q, *J* = 8.0 Hz, 2H), 1.17 - 0.99 (m, 2H).

MS-ESI: 335.3 [M+H]⁺.

### Example 22 Preparation of 3-(2-((3S,SR)-3,5-dimethylpiperazin-1-yl)pyrimidin-4-yl)quinoline (Compound 22)

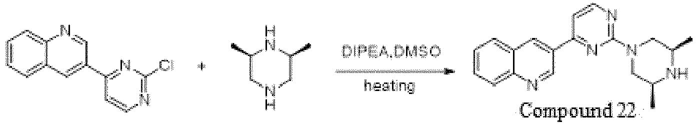

The synthesis of **compound 22** was the same as that of **compound 1** in **Example 1,** except that (2S,6R)-2,6-dimethylpiperazine was used instead of piperidin-4-ylmethylamine in **Example 1.** The yield was 83%.

¹H NMR (400 MHz, DMSO-*d6*): δ 9.61 (d, *J* = 2.2 Hz, 1H), 9.08 (d, *J* = 2.2 Hz, 1H), 8.51 (d, *J* = 5.2 Hz, 1H), 8.15 (d, *J* = 8.2 Hz, 1H), 8.09 (d, *J* = 8.4 Hz, 1H), 7.89 - 7.81 (m, 1H), 7.69 (t, *J* = 7.4 Hz, 1H), 7.40 (d, *J* = 5.2 Hz, 1H), 4.72 (d, *J* = 12.4 Hz, 2H), 2.81 - 2.74 (m, 2H), 2.45 (t, *J* = 12.0 Hz, 2H), 1.09 (s, 3H), 1.07 (s, 3H).

MS-ESI: 320.2 [M+H]⁺.

### Example 23 Preparation of 3-(2-(4-methylpiperazin-1-yl)pyrimidin-4-yl)quinoline (Compound 23)

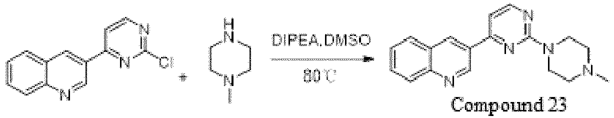

The synthesis of **compound 23** was the same as that of **compound 1** in **Example 1,** except that 1-methylpiperazine was used instead of piperidin-4-ylmethylamine in **Example 1.** The yield was 78%.

¹H NMR (400 MHz, *CD₃OD*): *δ* 9.56 (d, *J* = 4.0 Hz, 1H), 9.05 (d, *J* = 2.4 Hz, 1H), 8.48 (d, *J* = 8.0 Hz, 1H), 8.10 (d, *J* = 8.0Hz, 1H), 8.04 (d, *J* = 8.0 Hz, 1H), 7.80 (t, *J* = 7.2 Hz, 1H), 7.64 (t, *J* = 7.2 Hz, 1H), 7.39 (d, *J* = 8.0 Hz, 1H), 3.86-3.79 (m, 4H), 2.41 -2.34 (m, 4H), 2.20 (s, 3H).

MS-ESI: 306.4 [M+H]⁺.

### Example 24 Preparation of 3-(2-(1,4-diaza-1-yl)pyrimidin-4-yl)quinoline (Compound 24)

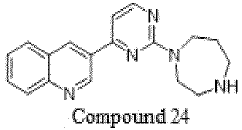

### tert-butyl 4-(4-(quinolin-3-yl)pyrimidin-2-yl)-1,4-diazane-1 carboxylate

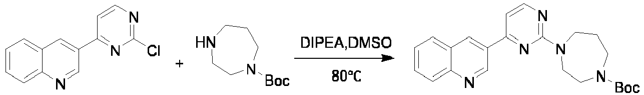

The synthesis of tert-butyl 4-(4-(quinolin-3-yl)pyrimidin-2-yl)-1,4-diazane-1 carboxylate was the same as that of (1-(4-(quinolin-3-yl)pyrimidin-2-yl)piperidin-4-yl)methylamine in **Example 1,** except that tert-butyl 1,4-diazo-1 carboxylate was used instead of piperidin-4-methylamine in **Example** 1. The yield was 71%.

MS-ESI: 406.3 [M+H]⁺.

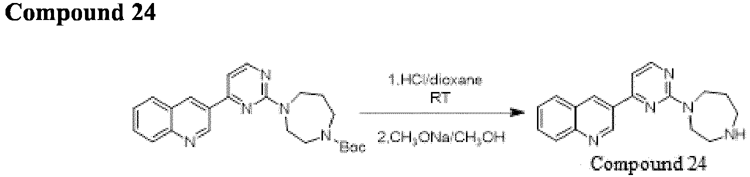

The synthesis of **compound 24** was the same as that of **compound 18** in **Example 18,** except that tert-butyl 4-(4-(quinolin-3-yl)pyrimidin-2-yl)-1,4-diazane-1 carboxylate was used instead of tert-butyl 4-(4-(quinolin-3-yl)pyrimidin-2-yl)piperazine-1 carboxylate in **Example 18.** The yield was 84%.

¹H NMR (400 MHz, *CDCl₃*) *δ* 9.59 (d, *J* = 4.0 Hz, 1H), 8.75 (d, *J* = 4.0 Hz, 1H), 8.45 (d, *J* = 4.0 Hz, 1H), 8.15 (d, *J* = 8.0 Hz, 1H), 7.94 (d, *J* = 8.0 Hz, 1H), 7.82 - 7.74 (m, 1H), 7.60 (t, *J* = 8.0 Hz, 1H), 7.07 (d, *J* = 8.0 Hz, 1H), 4.12 - 3.84 (m, 4H), 3.13 (s, 2H), 3.03 - 2.86 (m, 2H), 2.38 -2.20 (m, 1H), 2.00 (s, 2H).

MS-ESI:306.2 [M+H]⁺.

### Example 25 Preparation of 3-(2-(3,8-diazabicyclo[3.2.1]octan-3-yl)pyrimidin-4-yl)quinoline (Compound 25)

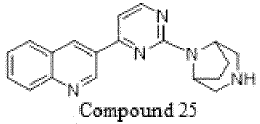

### tert-butyl 8-(4-(quinolin-3-yl)pyrimidin-2-yl)-3,8-diazabicyclo[3.2.1]octane-3 carboxylate

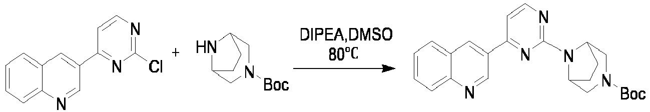

The synthesis of tert-butyl 8-(4-(quinolin-3-yl)pyrimidin-2-yl)-3,8-diazabicyclo[3.2.1]octane-3 carboxylate was the same as that of (1-(4-(quinolin-3-yl)pyrimidin-2-yl)piperidin-4-yl)methylamine in **Example 1,** except that tert-butyl 3,8-diazabicyclo[3.2.1]octane-3 carboxylate was used instead of piperidin-4-ylmethylamine in **Example 1.** The yield was 41%.

MS-ESI: 418.2 [M+H]⁺.

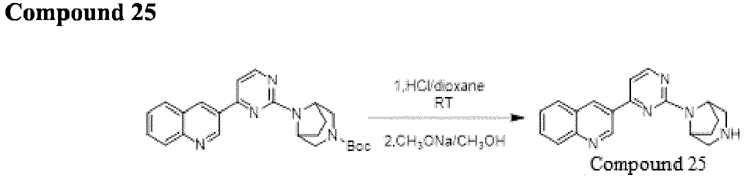

The synthesis of **compound 25** was the same as that of **compound 18** in **Example 18,** except that tert-butyl 8-(4-(quinolin-3-yl)pyrimidin-2-yl)-3,8-diazabicyclo[3.2.1]octane-3 carboxylate was used instead of tert-butyl 4-(4-(quinolin-3-yl)pyrimidin-2-yl)piperazine-1 carboxylate in **Example 18.** The yield was 63%.

¹H NMR (400 MHz, *CDCl₃*) *δ* 9.56 (d, *J* = 4.0 Hz, 1H), 8.76 (d, *J* = 4.0 Hz, 1H), 8.46 (d, *J* = 8.0 Hz, 1H), 8.16 (d, *J* = 8.0 Hz, 1H), 7.95 (d, *J* = 8.0 Hz, 1H), 7.78 (t, *J* = 8.0 Hz, 1H), 7.61 (t, *J* = 8.0 Hz, 1H), 7.10 (d, *J* = 8.0 Hz, 1H), 3.22 (d, *J* = 12.0 Hz, 2H), 2.84 (d, *J* = 12.0 Hz, 2H), 2.20 - 1.99 (m, 6H).

MS-ESI: 318.2 [M+H]⁺.

### Example 26 Preparation of N-(4-(quinolin-3-yl)pyrimidin-2-yl)ethane-1,2-diamine (Compound 26)

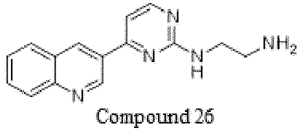

### tert-butyl (2-((4-(quinolin-3-yl)pyrimidin-2-yl)amino)ethyl) carbamate

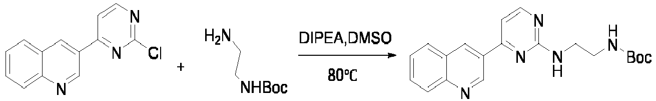

The synthesis of tert-butyl (2-((4-(quinolin-3-yl)pyrimidin-2-yl)amino)ethyl) carbamate was the same as that of (1-(4-(quinolin-3-yl)pyrimidin-2-yl)piperidin-4-yl)methylamine in **Example 1,** except that tert-butyl (2-aminoethyl) carbamate was used instead of piperidin-4-ylmethylamine in **Example 1.** The yield was 64%.

MS-ESI: 366.2 [M+H]⁺.

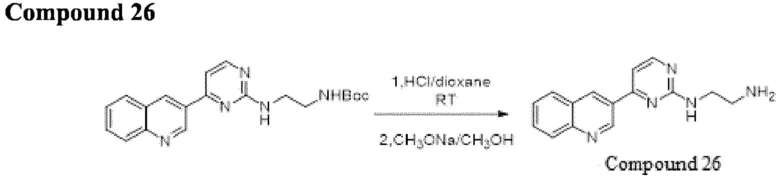

The synthesis of **compound 26** was the same as that of **compound 18** in **Example 18,** except that tert-butyl (2-((4-(quinolin-3-yl)pyrimidin-2-yl)amino)ethyl) carbamate was used instead of tert-butyl 4-(4-(quinolin-3-yl)pyrimidin-2-yl)piperazine-1 carboxylate in **Example 18.** The yield was 79%.

¹H NMR (400 MHz, *CDCl₃*) *δ* 9.54 (s, 1H), 8.78 (s, 1H), 8.42 (d, *J* = 8.0 Hz, 1H), 8.15 (d, *J* = 12.0 Hz, 1H), 7.94 (d, *J* = 8.0 Hz, 1H), 7.78 (t, *J* = 8.0 Hz, 1H), 7.61 (t, *J* = 8.0 Hz, 1H), 7.14 (d, *J* = 8.0 Hz, 1H), 3.68 - 3.56 (m, 2H), 3.02 (t, *J* =8.0 Hz, 2H).

MS-ESI: 266.2 [M+H]⁺.

### Example 27 Preparation of N-(4-(quinolin-3-yl)pyrimidin-2-yl)propane-1,3-diamine (Compound 27)

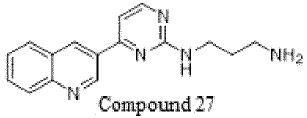

### tert-butyl (3-((4-(quinolin-3-yl)pyrimidin-2-yl)amino)propyl) carbamate

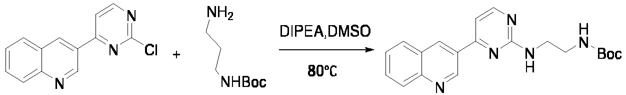

The synthesis of tert-butyl (2-((4-(quinolin-3-yl)pyrimidin-2-yl)amino)propyl) carbamate was the same as that of (1-(4-(quinolin-3-yl)pyrimidin-2-yl)piperidin-4-yl)methylamine in **Example 1,** except that tert-butyl (2-aminopropyl) carbamate was used instead of piperidin-4-ylmethylamine in **Example 1.** The yield was 50%.

MS-ESI: 380.2 [M+H]⁺.

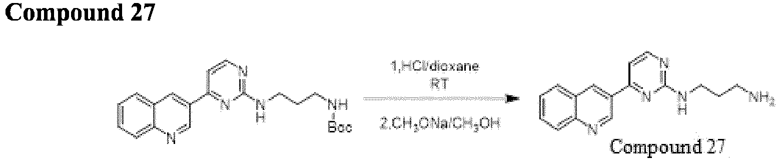

The synthesis of **compound 27** was the same as that of **compound 18** in **Example 18,** except that tert-butyl (2-((4-(quinolin-3-yl)pyrimidin-2-yl)amino)propyl) carbamate was used instead of tert-butyl 4-(4-(quinolin-3-yl)pyrimidin-2-yl)piperazine-1 carboxylate in **Example 18.** The yield was 98%.

¹H NMR (400 MHz, *CDCl₃*) *δ* 9.54 (s, 1H), 8.78 (d, *J* = 4.0 Hz, 1H), 8.41 (d, *J* = 8.0 Hz, 1H), 8.15 (d, *J* = 8.0 Hz, 1H), 7.94 (d, *J* = 8.0 Hz, 1H), 7.81 - 7.75 (m, 1H), 7.60 (t, *J* = 8.0 Hz, 1H), 7.11 (d, *J* = 4.0 Hz, 1H), 5.68 (s, 1H), 3.67 - 3.62 (m, 2H), 2.88 (t, *J* = 8.0 Hz, 2H), 1.82 (p, *J* =8.0 Hz, 2H), 1.41 (s, 2H).

MS-ESI:280.3 [M+H]⁺.

### Example 28 Preparation of 7-(2-(piperazin-1-yl)pyrimidin-4-yl)-2,3-dihydro-[1,4]dioxy[2,3-b]pyridine (Compound 28)

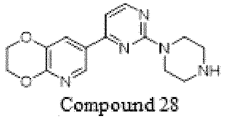

### tert-butyl 4-(4-chloropyrimidin-2-yl)piperazine-1 carboxylate

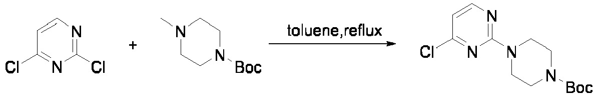

Compound 2,4-dichloropyrimidine (3.72 g, 21.5 mmol) and tert-butyl 4-methylpiperazine-1 carboxylate (4.3 g, 21.5 mmol) were dissolved in toluene (50 mL) and reacted under reflux overnight. The next day TLC detected the reaction was completed. The solvent was evaporated under reduced pressure, n-propanol/water (55mL/83mL) was added to the residue, the temperature was raised to 90°C, and the system was dissolved. The solution was cooled to room temperature to separate solid, filtered, washed with n-propanol/water (1v/1.5v, 30mL×3), and dried under high vacuum to obtain the product (4.0 g, 62%).

¹H NMR (400 MHz, CDCl₃) *δ* 8.16 (d, *J* = 5.2 Hz, 1H), 6.52 (d, *J* = 5.2 Hz, 1H), 3.85 - 3.72 (m, 4H), 3.52 - 3.41 (m, 4H), 1.48 (s, 9H).

### 7-(4,4,5,5-tetramethyl-1,3,2-dioxaborolan-2-yl)2,3-dihydro[1,4]dioxino[2,3b]pyridine

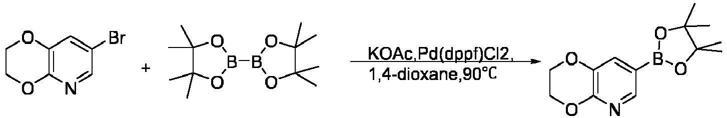

Compound 7-bromo-2,3-dihydro-[1,4]dioxin[2,3-b]pyridine (180 mg, 0.84 mmol), bis(pinacolato)diboron (318 mg, 1.26 mmol) ), potassium acetate (329 mg, 3.36 mmol) and Pd(dppf)Cl₂ (61 mg, 0.084 mmoL) were added to 1,4-dioxane (5 mL) and reacted overnight at 90°C under nitrogen protection. The reaction was detected by TLC the next day. The reaction system was filtered with celite, the filtrate was concentrated under reduced pressure, and ethyl acetate was added to the residue. The solution was separated, and the solvent was evaporated under reduced pressure. The residue was purified by flash column chromatography to obtain the target product (186 mg, 84%).

¹H NMR (400 MHz, *CDCl₃*) *δ* 8.19 (d, *J* = 1.6 Hz, 1H), 7.53 (d, *J* = 1.6 Hz, 1H), 7.26 (s, 1H), 4.48 - 4.41 (m, 2H), 4.27 - 4.20 (m, 2H), 1.33 (s, 12H).

MS-ESI: 264.4 [M+H].

### tert-butyl 4-(4-(2,3-dihydro-[1,4]dioxo[2,3-b]pyridin-7-yl)pyrimidin-2-yl)piperazine-1 carboxylate

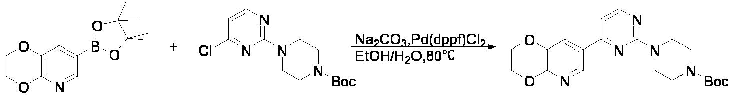

The synthesis of tert-butyl 4-(4-(2,3-dihydro-[1,4]dioxo[2,3-b]pyridin-7-yl)pyrimidin-2-yl)piperazine-1 carboxylate was the same as that of 3-(2-chloropyrimidin-4-yl)quinoline in **Example 1,** except that 7-(4,4,5,5-tetramethyl-1,3,2-dioxaborolane-2-yl)2,3-dihydro-[1,4]dioxin[2,3b]pyridine was used instead of quinolin-3-ylboronic acid and tert-butyl 4-(4-chloropyrimidin-2-yl)piperazine-1 carboxylate was used instead of 2,4-dichloropyrimidine. The yield was 46%.

¹H NMR (400 MHz, *CDCl₃*) *δ* 8.47 (d, *J* = 2.2 Hz, 1H), 8.36 (d, *J* = 5.2 Hz, 1H), 7.88 (d, *J* = 2.2 Hz, 1H), 6.89 (d, *J* = 5.2 Hz, 1H), 4.54 - 4.44 (m, 2H), 4.37 - 4.26 (m, 2H), 3.94 - 3.84 (m, 4H), 3.56 - 3.46 (m, 4H), 1.49 (s, 9H).

MS-ESI: 400.5 [M+H]⁺.

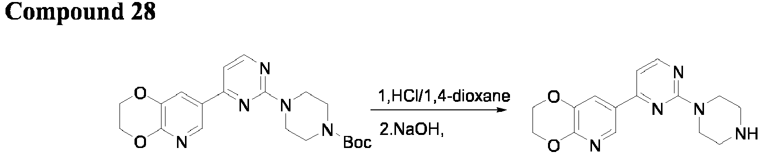

The synthesis of **compound 28** was the same as that of **compound 18** in **Example 18,** except that tert-butyl 4-(4-(2,3-dihydro-[1,4]dioxo[2,3-b]pyridin-7-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 92%.

¹H NMR (400 MHz,*DMSO-d6*) *δ* 8.52 (d, *J* = 2.2 Hz, 1H), 8.39 (d, *J* = 5.2 Hz, 1H), 7.95 (d, *J* = 2.2 Hz, 1H), 7.18 (d, *J* = 5.2 Hz, 1H), 4.52 - 4.42 (m, 2H), 4.33 - 4.27 (m, 2H), 3.77 - 3.69 (m, 4H), 2.83 - 2.72 (m, 4H).

### Example 29 Preparation of 4-(2,3-dihydrobenzo[b][1,4]dioxin-6-yl)-2-(piperazin-1-yl)pyrimidine (Compound 29)

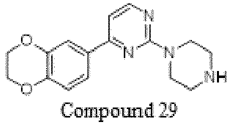

### tert-butyl 4-(4-(2,3-dihydrobenzo[b][1,4]dioxin-6-yl)pyrimidin-2-yl)piperazine-1 carboxylate

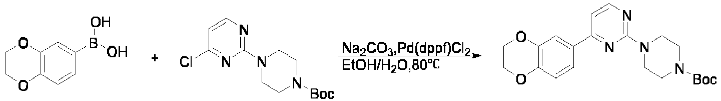

The synthesis of tert-butyl 4-(4-(2,3-dihydrobenzo[b][1,4]dioxin-6-yl)pyrimidin-2-yl)piperazine-1 carboxylate was the same as that of 3-(2-chloropyrimidin-4-yl)quinoline in **Example 1,** except that (2,3-dihydrobenzo[b][1,4]dioxin-6-yl)boronic acid was used instead of quinolin-3-boronic acid in **Example 1.** The yield was 57%.

¹H NMR (400 MHz, *CDCl₃*) *δ* 8.32 (d, *J* = 5.2 Hz, 1H), 7.63 (d, *J* = 2.2 Hz, 1H), 7.55 (dd, *J* = 8.4, 2.2 Hz, 1H), 6.94 (d, *J* = 8.4 Hz, 1H), 6.88 (d, *J* = 5.2 Hz, 1H), 4.36 - 4.20 (m, 4H), 3.89 (t, *J* = 5.2 Hz, 4H), 3.52 (t, *J=* 5.2 Hz, 4H), 1.50 (s, 9H).

MS-ESI: 399.5 [M+H]⁺.

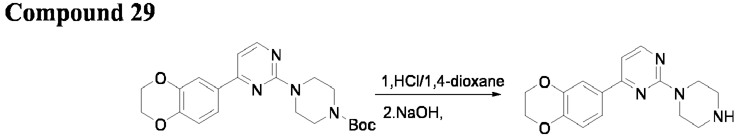

The synthesis of **compound 29** was the same as that of **compound 18** in **Example 18,** except that tert-butyl 4-(4-(2,3-dihydrobenzo[b][1,4]dioxin-6-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 53%.

¹H NMR (400 MHz, *CDCl₃*) *δ* 8.32 (d, *J* = 5.2 Hz, 1H), 7.63 (d, *J* = 2.2 Hz, 1H), 7.54 (dd, *J* = 8.4, 2.2 Hz, 1H), 6.93 (d, *J* = 8.4 Hz, 1H), 6.86 (d, *J* = 5.2 Hz, 1H), 4.36 - 4.26 (m, 4H), 3.95 - 3.88 (m, 4H), 3.00 - 2.98 (m, 4H).

MS-ESI: 299.5 [M+H]⁺.

### Example 30 Preparation of 4-(5,6-dimethoxy-pyridin-3-yl)-2-piperazin-1-yl-pyrimidine (Compound 30)

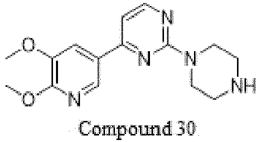

### 2,3 -dimethoxy- 5 -(4,4,5,5 - tetramethyl- [1,3,2] dioxaborolane-2- yl)- pyridine

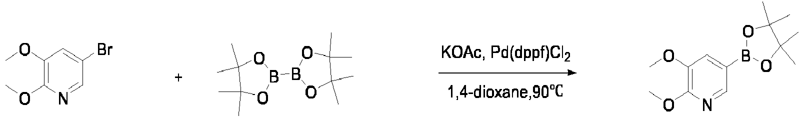

The synthesis of 2,3-dimethoxy-5-(4,4,5,5-tetramethyl-[1,3,2]dioxaborolane-2-yl)-pyridine was the same as that of 7-(4,4,5,5-tetramethyl-1,3,2-dioxaborolane-2-yl)2,3-dihydro-[1,4]dioxin[2,3b]pyridine in **Example 28,** except that 5-bromo-2,3-dimethoxypyridine was used instead of 7-bromo-2,3-dihydro-[1,4]dioxin[2,3-b]pyridine. The yield was 93%.

¹H NMR (400 MHz, *CDCl₃*) *δ* 8.12 (s, 1H), 7.34 (s, 1H), 4.04 (s, 3H), 3.89 (s, 3H), 1.34 (s, 12H).

MS-ESI: 266.4 [M+H]⁺.

### tert-butyl 4-[4-(5,6-dimethoxy-pyridin-3-yl)-pyrimidin-2-yl]-piperazine-1 carboxylate

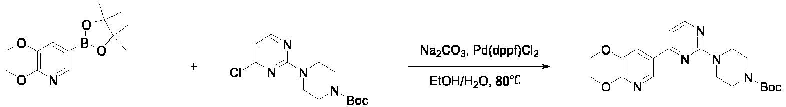

The synthesis of tert-butyl 4-[4-(5,6-dimethoxy-pyridin-3-yl)-pyrimidin-2-yl]-piperazine-1 carboxylate was the same as that of tert-butyl 4-(4-(2,3-dihydro-[1,4]dioxo[2,3-b]pyridin-7-yl)pyrimidin-2-yl)piperazine-1 carboxylate in **Example 28,** except that 2,3-dimethoxy-5-(4,4,5,5-tetramethyl-[1,3,2]dioxaborolane-2-yl)-pyridine was used instead of 7-(4,4,5,5-tetramethyl-1,3,2-dioxaborolane-2-yl)2,3-dihydro-[1.4]dioxin[2,3b]pyridine. The yield was 54%.

¹H NMR (400 MHz, *CDCl₃*) *δ* 8.39 (d, *J* = 1.8 Hz, 1H), 8.36 (d, *J* = 5.2 Hz, 1H), 7.74 (d, *J* = 1.8 Hz, 1H), 6.91 (d, *J* = 5.2 Hz, 1H), 4.08 (s, 3H), 3.97 (s, 3H), 3.93 - 3.85 (m, 4H), 3.59 - 3.49 (m, 4H), 1.50 (s, 9H).

MS-ESI: 402.5 [M+H]⁺.

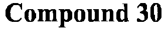

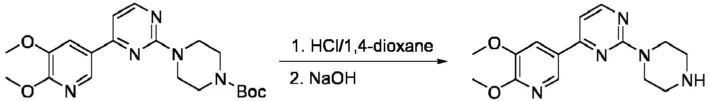

The synthesis of **compound 30** was the same as that of **compound 18** in **Example 18,** except that tert-butyl 4-[4-(5,6-dimethoxy-pyridin-3-yl)-pyrimidin-2-yl]-piperazine-1 carboxylate was used instead of tert-butyl 4-(4-(quinolin-3-yl)pyrimidin-2-yl)piperazine-1 carboxylate in **Example 18.** The yield was 40%.

¹H NMR (400 MHz, *DMSO-d6*) *δ* 8.50 (d, *J* = 1.8 Hz, 1H), 8.41 (d, *J* = 5.2 Hz, 1H), 7.87 (d, *J* = 1.8 Hz, 1H), 7.23 (d, *J* = 5.2 Hz, 1H), 3.93 (s, 3H), 3.87 (s, 3H), 3.82 - 3.76 (m, 4H), 2.90 - 2.80 (m, 4H).

MS-ESI: 302.4 [M+H]⁺.

### Example 31 Preparation of 4-(3,4-dimethoxy-phenyl)-2-piperazin-1-yl-pyrimidine (Compound 31)

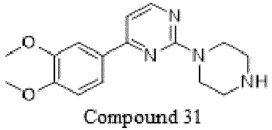

### tert-butyl 4-[4-(3,4-dimethoxy-phenyl)-pyrimidin-2-yl]-piperazine-1 carboxylate

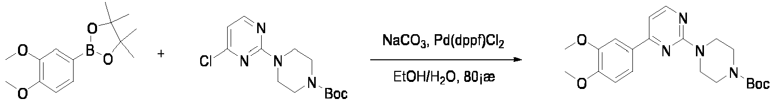

The synthesis of tert-butyl 4-[4-(3,4-dimethoxy-phenyl)-pyrimidin-2-yl]-piperazine-1 carboxylate was the same as that of tert-butyl 4-[4-(5,6-dimethoxy-pyridin-3-yl)-pyrimidin-2-yl]-piperazine-1 carboxylate in Example 30, except that 2-(3,4-dimethoxy-phenyl)-4,4,5,5-tetramethyl-[1,3,2]dioxaborolane was used instead of 2,3-dimethoxy-5-(4,4,5,5-tetramethyl-[1,3,2]dioxaborolane-2-yl)-pyridine. The yield was 60%.

¹H NMR (400 MHz, CDCl₃) *δ* 8.33 (d, *J* = 5.2 Hz, 1H), 7.67 - 7.60 (m, 2H), 6.94 (d, *J* = 8.4 Hz, 1H), 6.92 (d, *J* = 5.2 Hz, 1H), 3.98 (s, 3H), 3.94 (s, 3H), 3.92 - 3.86 (m, 4H), 3.57 - 3.50 (m, 4H), 1.49 (s, 9H).

MS-ESI: 401.5 [M+H]⁺.

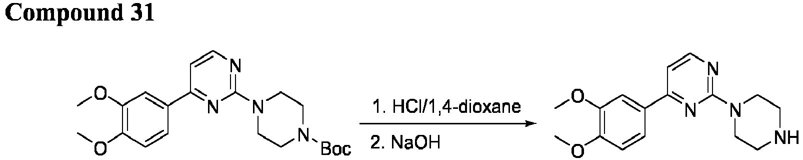

The synthesis of **compound 31** was the same as that of **compound 18** in **Example 18,** except that tert-butyl 4-[4-(3,4-dimethoxy-phenyl)-pyrimidin-2-yl]-piperazine-1 carboxylate was used instead of tert-butyl 4-(4-(quinolin-3-yl)pyrimidin-2-yl)piperazine-1 carboxylate in **Example 18.** The yield was 51%.

¹H NMR (400 MHz, *DMSO-d6*) *δ* 8.36 (d, *J* = 5.2 Hz, 1H), 7.72 (dd, *J* = 8.4, 1.8 Hz, 1H), 7.68 (d, *J* = 1.8 Hz, 1H), 7.16 (d, *J* = 5.2 Hz, 1H), 7.06 (d, *J* = 8.4 Hz, 1H), 3.84 (s, 3H), 3.82 (s, 3H), 3.79 - 3.72 (m, 4H), 2.83 - 2.76 (m, 4H).

MS-ESI: 301.5 [M+H]⁺.

### Example 32 Preparation of 3-(2-(piperazin-1-yl)pyrimidin-4-yl)-1H-indole (Compound 32)

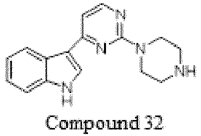

### tert-butyl 3-(2-(4-(tert-butyloxycarbonyl)piperazin-1-yl)pyrimidin-4-yl)-1H-indole-1 carboxylate

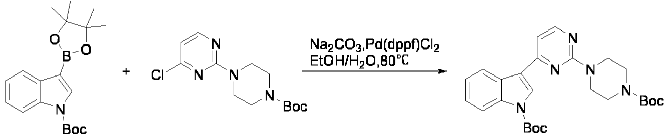

The synthesis of tert-butyl 3-(2-(4-(tert-butyloxycarbonyl)piperazin-1-yl)pyrimidin-4-yl)-1H-indole-1 carboxylate was the same as that of 3-(2-chloropyrimidin-4-yl)quinoline, except that tert-butyl 3-(4,4,5,5-tetramethyl-1,3,2-dioxolan-2-yl)-1H-indole-1 carboxylate was used instead of quinolin-3-boronic acid in **Example 1.** The yield was 69%.

1H NMR (400 MHz, *CDCl₃*): *δ* 8.40 - 8.36 (m, 1H), 8.34 (d, *J* = 8.0Hz, 1H), 8.19 (s, 2H), 7.39-7.5 (m, 2H), 6.93 (d, *J* = 8.0 Hz, 1H), 3.93 (t, *J* = 8.0Hz, 4H), 3.57 (t, *J* = 8.0 Hz, 4H), 1.71 (s, 9H), 1.51 (s, 9H).

MS-ESI: 480.6[M+H]⁺.

### tert-butyl 3-(2-(piperazin-1-yl)pyrimidin-4-yl)-1H-indole-1carboxylate

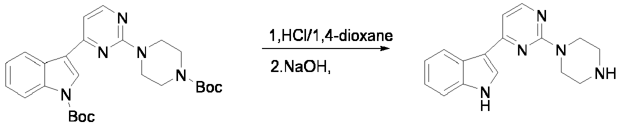

Compound tert-butyl 3-(2-(4-(tert-butyloxycarbonyl)piperazin-1-yl)pyrimidin-4-yl)-1H-indole-1 carboxylate (284 mg, 0.59 mmol) was dissolved in DCM (8 mL), HCl/1,4-dioxane (4 M, 5 mL) was added, and the reaction was carried out at room temperature for 30 min. TLC detected the reaction was completed. The solvent was evaporated under reduced pressure, water (5 mL) was added to the residue, and the pH was adjusted to 14 with NaOH (3 M). The solvent was evaporated under reduced pressure, and the residue was purified by flash column chromatography to obtain the target product (210 mg, 93%).

¹H NMR (400 MHz, *CDCl₃*): *δ* 8.39 - 8.36 (m, 1H), 8.33 (d, *J* =4.0Hz, 1H), 8.19 (d, *J* = 8.0Hz, 2H), 7.39-7.34 (m, 2H), 6.92 (d, *J* = 4.0 Hz, 1H), 3.98 (t, *J* = 12.0 Hz, 4H), 3.05(t, *J* = 12.0 Hz, 4H), 1.71 (s, 9H).

MS-ESI: 380.5[M+H]⁺.

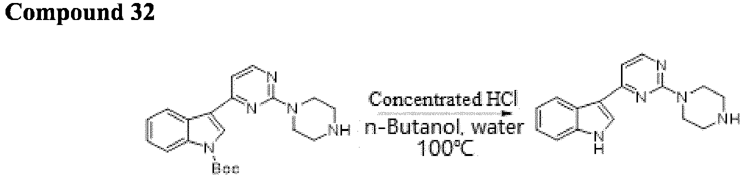

Compound tert-butyl 3-(2-(piperazin-1-yl)pyrimidin-4-yl)-1H-indole-1 carboxylate (210 mg, 0.551 mmol) was dissolved in a mixed system of n-butanol (2.1 mL) and water (1.2 mL), concentrated hydrochloric acid (0.23 mL) was added, the reaction was heated overnight, and TLC detected the reaction was completed. The solvent was evaporated under reduced pressure. Water (5 mL) was added to the residue and the pH was adjusted to 14 with NaOH (3 M). The solvent was evaporated under reduced pressure, and the residue was purified by flash column chromatography to obtain the target product (124 mg, 80%).

¹H NMR (400 MHz, *CDCl₃*) *δ* 8.53 (s, 1H), 8.44 - 8.38 (m, 1H), 8.29 (d, *J* = 4.0 Hz, 1H), 7.88 (d, *J* = 2.0 Hz, 1H), 7.45 - 7.39 (m, 1H), 7.29 (s, 1H), 6.88 (d, *J* = 5.2 Hz, 1H), 3.96 - 3.90 (m, 4H), 3.03-3.00 (m, 4H).

MS-ESI: 280.5[M+H]⁺.

### Example 33 Preparation of 4-(benzofuran-3-yl)-2-(piperazin-1-yl)pyrimidine (Compound 33)

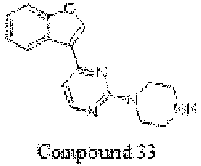

### tert-butyl 4-(4-(benzofuran-3-yl)pyrimidin-2-yl)piperazine-1 carboxylate

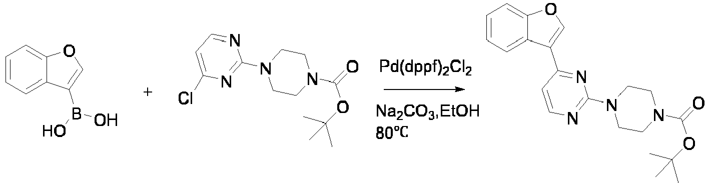

The synthesis of tert-butyl 4-(4-(benzofuran-3-yl)pyrimidin-2-yl)piperazine-1 carboxylate was the same as that of 3-(2-chloropyrimidin-4-yl)quinoline in **Example 1,** except that benzofuran-3-ylboronic acid was used instead of quinolin-3-ylboronic acid and tert-butyl 4-(4-Chloropyrimidin-2-yl)piperazine-1 carboxylate was used instead of 2,4-dichloropyrimidine. The yield was 84%.

¹H NMR (400 MHz, *CDCl₃*) δ 8.36 (d, *J* = 5.1 Hz, 1H), 8.23 (q, *J* = 4.3, 3.4 Hz, 2H), 7.67 - 7.50 (m, 1H), 7.43 - 7.30 (m, 2H), 6.89 (d, *J* = 5.1 Hz, 1H), 4.01 - 3.85 (m, 4H), 3.57 (dd, *J* = 6.5, 4.0 Hz, 4H), 1.50 (s, 9H).

MS-ESI: 381.5 [M+H]⁺.

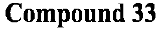

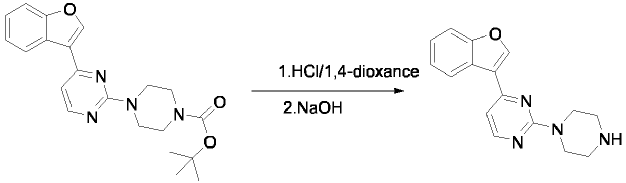

The synthesis of **compound 33** was the same as that of **compound 18** in **Example 18,** except that tert-butyl 4-(4-(benzofuran-3-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 86%.

¹H NMR (400 MHz, *CDCl₃*) *δ* 8.35 (d, *J* = 5.1 Hz, 1H), 8.24 (d, *J* = 10.3 Hz, 2H), 7.65 - 7.50 (m, 1H), 7.43 - 7.31 (m, 2H), 6.86 (d, *J* = 5.1 Hz, 1H), 3.98 - 3.89 (m, 4H), 3.01 (dd, *J* = 6.0, 4.2 Hz, 4H).

MS-ESI: 281.4 [M+H]⁺.

### Example 34 Preparation of 3-methoxy-5-(2-piperazin-1-yl-pyrimidin-4-yl)-pyridin-2-ol (Compound 34)

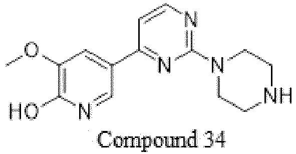

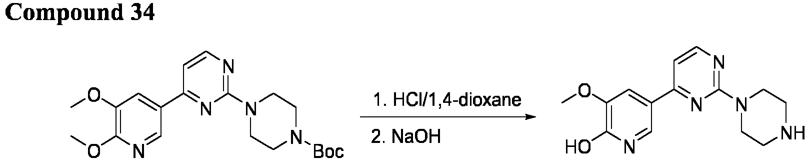

The synthesis of **compound 34** was the same as that of 4-(5,6-dimethoxy-pyridin-3-yl)-2-piperazin-1-yl-pyrimidine in **Example 30,** except extending the reaction time of removing Boc from hydrochloric acid to overnight. The yield was 50%.

¹H NMR (400 MHz, *DMSO*) *δ* 8.31 (d, *J* = 5.2 Hz, 1H), 7.87 (d, *J* = 2.2 Hz, 1H), 7.44 (d, *J* = 2.2 Hz, 1H), 7.07 (d, *J* = 5.4 Hz, 1H), 3.79 (s, 3H), 3.76 - 3.64 (m, 4H), 2.83 - 2.68 (m, 4H).

MS-ESI: 288.4 [M+H]⁺.

### Example 35 Preparation of 3-(4-(piperazin-1-yl)-1,3,5-triazin-2-yl)quinoline (Compound 35)

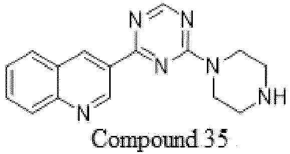

### tert-butyl 4-(4-(quinolin-3-yl)pyrimidin-2-yl)piperazine-1 carboxylate

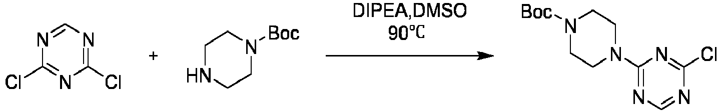

The synthesis of tert-butyl 4-(4-chloro-1,3,5-triazin-2-yl)piperazine-1 carboxylate was the same as that of (1-(4-(quinolin-3-yl)pyrimidin-2-yl)piperidin-4-yl)methylamine in **Example 1,** except that 2,4-dichloro-1,3,5-triazine was used instead of 3-(2-chloropyrimidin-4-yl)quinoline in **Example 1** and tert-butyl piperazine-1 carboxylate was used instead of piperidin-4-ylmethylamine. The yield was 46%.

MS-ESI: 300.3 [M+H] ⁺

### tert-butyl 4-(4-(quinolin-3-yl)-1,3,5-triazin-2-yl)piperazine-1 carboxylate

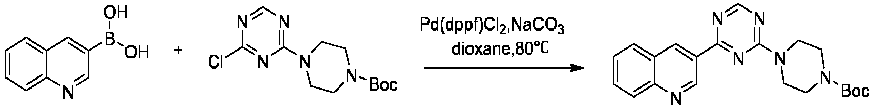

The synthesis of tert-butyl 4-(4-(quinolin-3-yl)-1,3,5-triazin-2-yl)piperazine-1 carboxylate was the same as that of 3-(2-chloropyrimidin-4-yl)quinoline in **Example 1,** except that tert-butyl 4-(4-(quinolin-3-yl)pyrimidin-2-yl)piperazine-1 carboxylate was used instead of quinoline-3-boronic acid in **Example 1.** The yield was 42%.

¹H NMR (400 MHz, *CDCl₃*) *δ* :9.92 (d, *J* = 4.0 Hz, 1H), 9.25 (d, *J* = 4.0 Hz, 1H), 8.78 (s, 1H), 8.25 (d, *J* = 8.0 Hz, 1H), 8.04 (d, *J* = 8.0 Hz, 1H), 7.87 (t, *J* = 8.0 Hz, 1H), 7.68 (t, *J* = 8.0 Hz, 1H), 4.07 (d, *J* = 44.0 Hz, 4H), 3.65 (d, *J* = 8.0 Hz, 4H), 1.58 (s, 9H).

MS-ESI: 393.4 [M+H] ⁺.

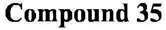

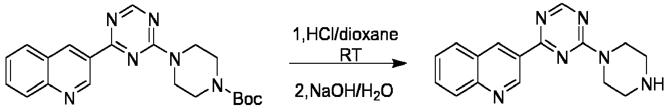

The synthesis of **compound 35** was the same as that of **compound 18** in **Example 18,** except that tert-butyl 4-(4-(quinolin-3-yl)-1,3,5-triazin-2-yl)piperazine-1 carboxylate was used instead of tert-butyl 4-(4-(quinolin-3-yl)pyrimidin-2-yl)piperazine-1 carboxylate in **Example 18.** The yield was 72%.

¹H NMR (400 MHz, *DMSO-d6*) *δ* :9.75 (d, *J* = 4.0 Hz, 1H), 9.30 (s, 1H), 8.74 (s, 1H), 8.22 (d, *J* = 8.0 Hz, 1H), 8.11 (d, *J* = 8.0 Hz, 1H), 7.88 (t, *J* = 8.0 Hz, 1H), 7.70 (t, *J* = 8.0 Hz, 1H), 3.89 (d, *J* = 28.0 Hz, 4H), 2.81 (d, *J* = 16.0 Hz, 4H), 1.23 (s, 1H).

MS-ESI: 293.4 [M+H]⁺.

### Example 36 Preparation of 2-(piperazin-1-yl)-4-(quinolin-3-yl)quinazoline (Compound 36)

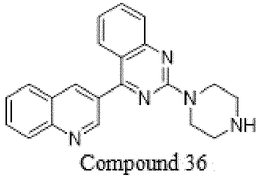

### 2-chloro-4-(quinolin-3-yl)quinazoline

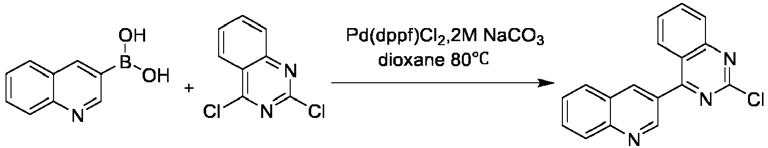

The synthesis of 2-chloro-4-(quinolin-3-yl)quinazoline was the same as that of 3-(2-chloropyrimidin-4-yl)quinoline in **Example 1,** except that 2,4-dichloroquinazoline was used instead of 2,4-dichloropyrimidine in **Example 4-1** and 1,4dioxane was used instead of acetonitrile. The yield was 73%.

¹H NMR (400 MHz, CDCl₃) *δ* 9.32 (d, *J* = 4.0 Hz, 1H), 8.67 (d, *J* = 2.0 Hz, 1H), 8.25 (d, *J* = 8.0 Hz, 1H), 8.19 - 8.10 (m, 2H), 8.05 - 7.97 (m, 2H), 7.88 (t, *J* = 8.0 Hz, 1H), 7.69 (t, *J* = 8.0 Hz, 2H).

MS-ESI: 292.4 [M+H]⁺.

### tert-butyl 4-(4-(quinolin-3-yl)quinazolin-2-yl)piperazine-1 carboxylate

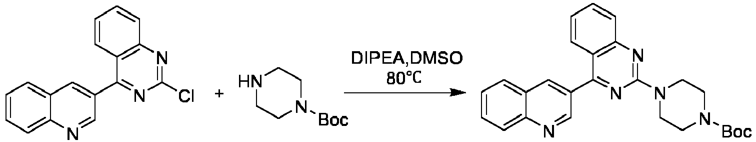

The synthesis of tert-butyl 4-(4-(quinolin-3-yl)quinazolin-2-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidin-4-ylmethylamine in **Example 1.** The yield was 84%.

¹H NMR (400 MHz, *CDCl₃*) *δ* 9.30 (d, *J* = 2.0 Hz, 1H), 8.54 (d, *J=* 2.0 Hz, 1H), 8.22 (d, *J* = 8.0 Hz, 1H), 7.96 (d, *J* = 8.0 Hz, 1H), 7.91 - 7.80 (m, 2H), 7.73 - 7.62 (m, 3H), 7.25 - 7.20 (m, 1H), 4.10 - 3.97 (m, 4H), 3.64 - 3.52 (m, 4H), 1.50 (s, 9H).

MS-ESI: 442.5 [M+H]⁺.

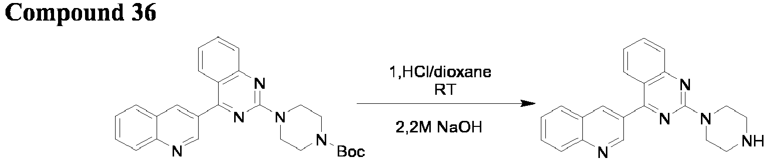

The synthesis of compound 36 was the same as that of **compound 18** in **Example 18,** except that tert-butyl 4-(4-(quinolin-3-yl)quinazolin-2-yl)piperazine-1 carboxylate was used instead of tert-butyl 4-(4-(quinolin-3-yl)pyrimidin-2-yl)piperazine-1 carboxylate in **Example 18.** The yield was 82%.

¹H NMR (400 MHz, *DMSO-d6*) *δ* 9.23 (d, *J* = 4.0 Hz, 1H), 8.80 (d, *J* = 2.0 Hz, 1H),8.20 - 8.13 (m, 2H), 7.95 - 7.85 (m, 2H), 7.80 - 7.70 (m, 2H), 7.62 (d, *J* = 8.0 Hz, 1H), 7.28 (t, *J* = 8.0 Hz, 1H), 3.96 - 3.84 (m, 4H), 2.92 - 2.81 (m, 4H).

MS-ESI: 342.3 [M+H]⁺.

### Example 37 Preparation of 2-(piperazin-1-yl)-4-(quinolin-3-yl)-5,6,7,8-tetrahydroquinazole (Compound 37)

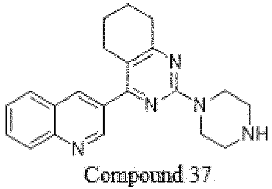

### 2-chloro-4-(quinolin-3-yl)-5,6,7,8-tetrahydroquinazole

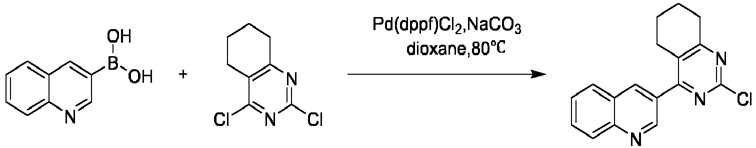

The synthesis of 2-chloro-4-(quinolin-3-yl)-5,6,7,8-tetrahydroquinazole was the same as that of 3-(2-chloropyrimidin-4-yl)quinoline in **Example 1,** except that 2,4-dichloro-5,6,7,8-tetrahydroquinazoline was used instead of 2,4-dichloropyrimidine. The yield was 92%.

¹H NMR (400 MHz, *CDCl₃*) *δ* :9.09 (d, *J* = 2.4 Hz, 1H), 8.39 (d, *J* = 2.4 Hz, 1H), 8.14 (d, *J* = 8.0 Hz, 1H), 7.87 (d, *J* = 8.0 Hz, 1H), 7.78 (t, *J* = 7.5 Hz, 1H), 7.59 (t, *J* = 7.5 Hz, 1H), 2.98 (t, *J* = 8.0 Hz, 2H), 2.81 (t, *J* = 8.0 Hz, 2H), 2.00-1.87 (m, 2H), 1.82-1.69 (m, 2H).

MS-ESI: 296.4 [M+H]⁺.

### tert-butyl 4-(4-(quinolin-3-yl)-5,6,7,8-tetrahydroquinazolin-2-yl)piperazine-1 carboxylate

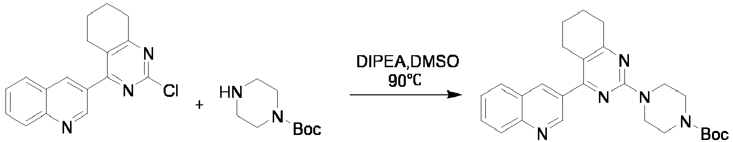

The synthesis of tert-butyl 4-(4-(quinolin-3-yl)-5,6,7,8-tetrahydroquinazolin-2-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidin-4-ylmethylamine in **Example 1** and 2-chloro-4-(quinolin-3-yl)-5,6,7,8-tetrahydroquinazole was used instead of 3-(2-chloropyrimidin-4-yl)quinoline in **Example 1.** The yield was 78%.

¹H NMR (400 MHz, CDCl₃) *δ* :9.14 (d, *J* = 4.0 Hz, 1H), 8.33 (d, *J* = 4.0 Hz, 1H), 8.15 (d, *J* = 8.0 Hz, 1H), 7.88 (d, *J* = 8.0 Hz, 1H), 7.76 (t, *J* = 8.0 Hz, 1H), 7.59 (t, *J* =8.0Hz, 1H), 3.88-3.77 (m, 4H), 3.57-3.42 (m, 4H), 2.81 (t, *J* = 8.0 Hz, 2H), 2.70 (t, *J* = 8.0Hz, 2H), 1.95-1.83 (m, 2H), 1.79-1.67 (m, 2H), 1.48 (s, 9H)_{∘}

MS-ESI: 446.6 [M+H]⁺.

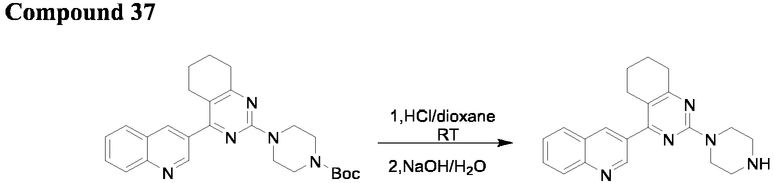

The synthesis of **compound 37** was the same as that of **compound 18** in **Example 18,** except that tert-butyl 4-(4-(quinolin-3-yl)-5,6,7,8-tetrahydroquinazolin-2-yl)piperazine-1 carboxylate was used instead of tert-butyl 4-(4-(quinolin-3-yl)pyrimidin-2-yl)piperazine-1 carboxylate in **Example 18.** The yield was 73%.

¹H NMR (400 MHz, *DMSO-d6*) *δ* :9.07 (d, *J* = 4.0 Hz, 1H), 8.58 (d, *J* = 2.4 Hz, 1H), 8.08 (t, *J* = 8.0 Hz, 2H), 7.83 (t, *J* = 8.0Hz, 1H), 7.67 (t, *J* = 8.0 Hz, 1H), 3.71-3.63 (m, 4H), 2.80-2.69 (m, 6H), 2.66 (t, *J* = 8.0 Hz, 2H), 1.86-1.77 (m, 2H), 1.71-1.62 (m, 2H), 1.23 (s, 1H).

MS-ESI: 346.5 [M+H] ⁺.

### Example 38 Preparation of 3-(5-amino-2-(piperazin-1-yl)pyrimidin-4-yl)quinoline (Compound 38)

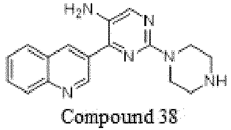

### 3-(2-chloro-6-methoxypyrimidin-4-yl)quinoline

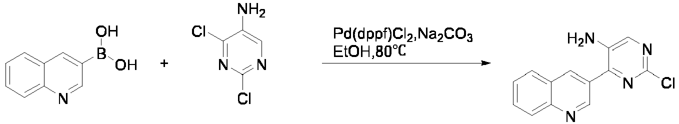

The synthesis of 3-(2-chloro-5-aminopyrimidin-4-yl)quinoline was the same as that of 3-(2-chloropyrimidin-4-yl)quinoline in **Example 1,** except that 2,4-dichloro-5-aminopyrimidine was used instead of 2,4-dichloropyrimidine in **Example 1.** The yield was 86%.

¹H NMR (400 MHz, *CDCl₃*) *δ:* 9.19 (d, *J* = 2.2 Hz, 1H), 8.76 (d, *J* = 2.2 Hz, 1H), 8.29 (s, 1H), 8.14 - 8.05 (m, 2H), 7.90 - 7.80 (m, 1H), 7.69 (t, *J* = 8.0 Hz, 1H), 5.95 (s, 2H).

MS-ESI: 257.3 [M+H]⁺.

### tert-butyl 4-(5-amino-6-(quinolin-3-yl)pyrimidin-2-yl)piperazine-1 carboxylate

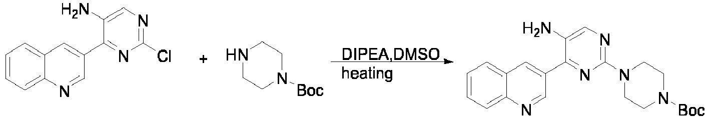

The synthesis of tert-butyl 4-(5-amino-6-(quinolin-3-yl)pyrimidin-2-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidin-4-ylmethylamine and 3-(2-chloro-5-aminopyrimidin-4-yl)quinoline was used instead of 3-(2-chloropyrimidin-4-yl)quinoline. The yield was 42%.

¹H NMR (400 MHz, DMSO-d₆) *δ:* 9.36 (d, *J* = 2.2 Hz, 1H), 8.85 (d, *J* = 2.2 Hz, 1H), 8.22 (s, 1H), 8.16 - 8.06 (m, 2H), 7.86 - 7.82 (m, 1H), 7.71 - 7.66 (m, 1H), 4.88 (s, 2H), 3.68 - 3.61 (m, 4H), 3.48 - 3.42 (m, 4H), 1.45 (s, 9H).

MS-ESI: 407.4 [M+H]⁺.

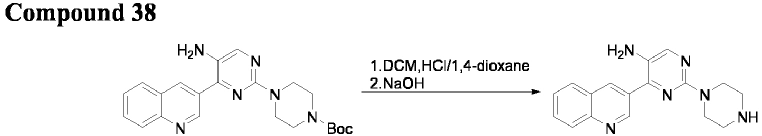

The synthesis of **compound 38** was the same as that of **compound 18** in **Example 18,** except that tert-butyl 4-(5-amino-6-(quinolin-3-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 63%.

¹H NMR (400 MHz, *DMSO*) *δ* : 9.32 (d, *J* = 2.2 Hz, 1H), 8.82 (d, *J* = 2.2 Hz, 1H), 8.18 (s, 1H), 8.09 - 8.03 (m, 2H), 7.81 (t, *J* = 7.6 Hz, 1H), 7.66 (t, *J* = 7.6 Hz, 1H), 4.80 (s, 2H), 3.59 (t, *J* = 5.0 Hz, 4H), 2.82 (t, *J* = 5.0 Hz, 4H).

MS-ESI:307.4 [M+H]⁺.

### Example 39 Preparation of 3-(6-methoxy-2-(piperazin-1-yl)pyrimidin-4-yl)quinoline (Compound 39)

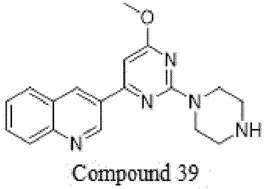

### 3-(2-chloro-6-methoxypyrimidin-4-yl)quinoline

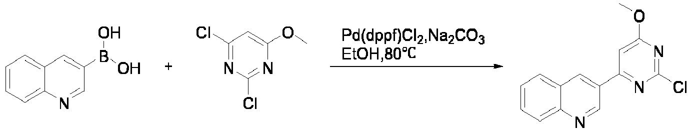

The synthesis of 3-(2-chloro-6-methoxypyrimidin-4-yl)quinoline was the same as that of 3-(2-chloropyrimidin-4-yl)quinoline in **Example 1,** except that 2,4-dichloro-6-methoxypyrimidine was used instead of 2,4-dichloropyrimidine in **Example 1.** The yield was 57%.

¹H NMR (400 MHz, *CDCl₃*) *δ* : 9.44 (d, *J* = 2.4 Hz, 1H), 8.90 (d, *J* = 2.4 Hz, 1H), 8.17 (d, *J* = 8.6 Hz, 1H), 8.00 - 7.93 (m, 1H), 7.81 (t, *J* = 8.6 Hz, 1H), 7.67 - 7.58 (m, 1H), 7.21 (s, 1H), 4.10 (s, 3H).

MS-ESI: 272.3 [M+H]⁺.

### tert-butyl 4-(4-methoxy-6-(quinolin-3-yl)pyrimidin-2-yl)piperazine-1 carboxylate

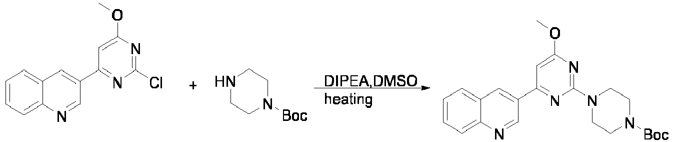

The synthesis of tert-butyl 4-(4-methoxy-6-(quinolin-3-yl)pyrimidin-2-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidin-4-ylmethylamine in **Example 1** and 3-(2-chloro-6-methoxypyrimidin-4-yl)quinoline was used instead of 3-(2-chloropyrimidin-4-yl)quinoline. The yield was 55%.

¹H NMR (400 MHz, DMSO-d₆) *δ* :9.58 (d, *J* = 2.2 Hz, 1H), 9.07 (d, *J* = 2.2 Hz, 1H), 8.12 (d*, J* = 8.0 Hz, 1H), 8.07 (d*, J* = 8.0 Hz, 1H), 7.90 - 7.78 (m, 1H), 7.68 (*t, J* = 7.4 Hz, 1H), 6.93 (s, 1H), 3.94 (s, 3H), 3.91 - 3.81 (m, 4H), 3.54 - 3.43 (m, 4H), 1.44 (s, 9H).

MS-ESI: 422.5 [M+H]⁺.

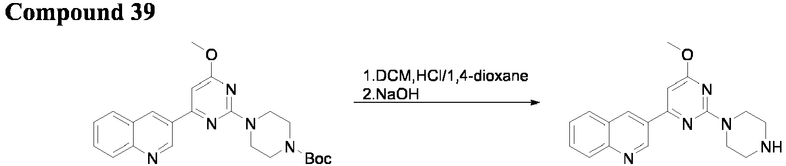

The synthesis of **compound 39** was the same as that of **compound 18** in **Example 18,** except that tert-butyl 4-(4-methoxy-6-(quinolin-3-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 60%.

¹H NMR (400 MHz, *DMSO*) *δ* : 9.56 (d, *J* = 2.2 Hz, 1H), 9.04 (d, *J* = 2.2 Hz, 1H), 8.12 (d, *J* = 8.4 Hz, 1H), 8.07 (d, *J* = 8.4 Hz, 1H), 7.82 (t, *J* = 8.4 Hz, 1H), 7.67 (t, *J* = 8.4 Hz, 1H), 6.87 (s, 1H), 3.92 (s, 3H), 3.82 (t, *J* = 5.0 Hz, 4H), 2.81 (t, *J* = 5.0 Hz, 4H).

MS-ESI:322.5 [M+H]⁺.

### Example 40 Preparation of 3-(2-(piperazin-1-yl)-6-(trifluoromethyl)pyrimidin-4-yl)quinoline (Compound 40)

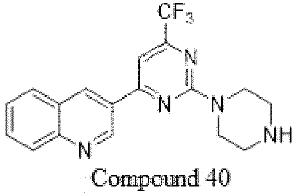

### tert-butyl 4-(4-chloro-6-(trifluoromethyl)pyrimidin-2-yl)piperazine-1 carboxylate

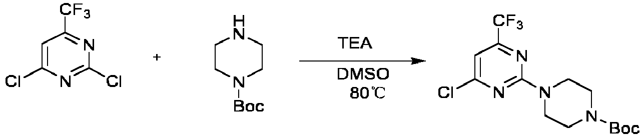

The synthesis of tert-butyl 4-(4-chloro-6-(trifluoromethyl)pyrimidin-2-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidine-4-ylmethylamine in **Example 1** and 2,4-dichloro-6-(trifluoromethyl)pyrimidine was used instead of 3-(2-chloropyrimidin-4-yl)quinoline. The yield was 40%.

¹H NMR (400 MHz, *CDCl₃*):δ 6.79 (s, 1H), 3.86- 3.83 (m, 4H), 3.52 -3.50 (m, 4H), 1.49 (s, 9H).

### tert-butyl 4-(4-(quinolin-3-yl)-6-(trifluoromethyl)pyrimidin-2-yl)piperazine-1 carboxylate

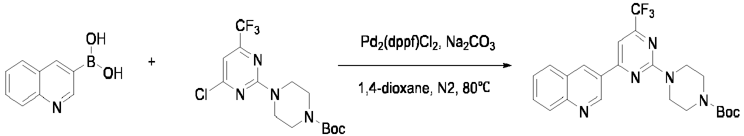

The synthesis of tert-butyl 4-(4-(quinolin-3-yl)-6-(trifluoromethyl)pyrimidin-2-yl)piperazine-1 carboxylate was the same as that of 3-(2-chloropyrimidin-4-yl)quinoline in **Example 1,** except that tert-butyl 4-(4-(quinolin-3-yl)-6-(trifluoromethyl)pyrimidin-2-yl)piperazine-1 carboxylate was used instead of 2,4-dichloropyrimidine and 1,4-dioxane was used instead of ethanol. The yield was 85%.

¹H NMR (400 *MHz,CDCl₃)* :δ 9.58 (d, *J* = 2.0 Hz, 1H), 8.79 (m, d, *J* = 2.0 Hz, 1H), 8.19 (d, *J* = 8.0 Hz, 1H), 7.96-7.95 (m, 1H), 7.84-7.80(m,1H), 7.66-7.64 (m, 1H), 7.37 (s, 1H), 4.00-3.99 (m,4H), 3.60-3.57(m,4H), 1.51 (s, 9H).

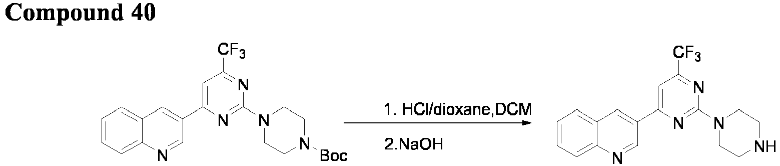

The synthesis of **compound 40** was the same as that of compound 18 in Example 18, except that tert-butyl 4-(4-(quinolin-3-yl)-6-(trifluoromethyl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 83%.

¹H NMR (400 MHz, *DMSO-d6)* :*δ* 9.67 (d, *J* = 4.0 Hz, 1H), 9.25 (d, *J* = 2.0 Hz, 1H), 8.17-8.15 (m, 1H), 8.10 (d, *J* = 8.0 Hz, 1H), 7.90 -7.86 (m, 1H), 7.82 (s, 1H), 7.73 -7.71 (m, 1H), 3.90-3.84(m, 4H), 2.85-2.83 (m, 4H).

MS-ESI: 360.3 [M+H]⁺.

### Example 41 Preparation of 3-(5-(piperazin-1-yl)imidazo[1,2-c]pyrimidin-7-yl)quinoline (Compound 41)

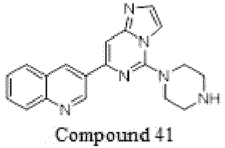

### tert-butyl 4-(7-chloroimidazo[1,2-c]pyrimidin-5-yl)piperazine-1 carboxylate

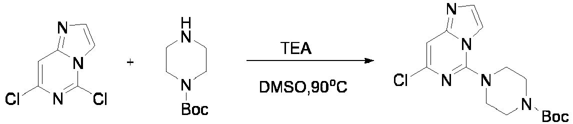

The synthesis of tert-butyl 4-(7-chloroimidazo[1,2-c]pyrimidin-5-yl)piperazine-1 carboxylate was the same as that of (1-(4-(quinolin-3-yl)pyrimidin-2-yl)piperidin-4-yl)methylamine in **Example 1,** except that 5,7-dichloroimidazo[1,2-c]pyrimidine was used instead of 3-(2-chloropyrimidin-4-yl)quinoline in **Example 1,** tert-butyl piperazine-1 carboxylate was used instead of piperidine-4-ylmethylamineand and TEA was used instead of DIPEA. The yield was 90%.

¹H NMR (400 MHz, *CDCl₃*) *δ:* 7.60 (s, 1H), 7.40 (s, 1H), 7.21 (s, 1H), 3.68 - 3.62 (m, 4H), 3.51 - 3.44 (m, 4H), 1.49 (s, 9H).

### tert-butyl 4-(7-(quinolin-3-yl)imidazo[1,2-c]pyrimidin-5-yl)piperazine-1 carboxylate

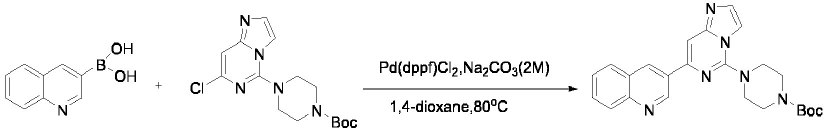

The synthesis of tert-butyl 4-(7-(quinolin-3-yl)imidazo[1,2-c]pyrimidin-5-yl)piperazine-1 carboxylate was the same as that of 3-(2-chloropyrimidin-4-yl)quinoline in **Example 1,** except that tert-butyl 4-(7-chloroimidazo[1,2-c]pyrimidin-5-yl)piperazine-1 carboxylate was used instead of 2,4-dichloropyrimidine and 1,4-dioxane was used instead of ethanol. The yield was 31%.

¹H NMR (400 MHz, *CDCl₃*) *δ:* 9.59 (s, 1H), 8.77 (s, 1H), 8.15 (d, *J* = 8.0 Hz, 1H), 7.95 (d, *J* = 8.0 Hz, 1H), 7.84 (s, 1H), 7.75 (t, *J* = 8.0 Hz, 1H), 7.70 (s, 1H), 7.60 (t, *J* = 8.0 Hz, 1H), 7.49 (s, 1H), 3.74 (m, 4H), 3.62 - 3.55 (m, 4H), 1.52 (s, 9H).

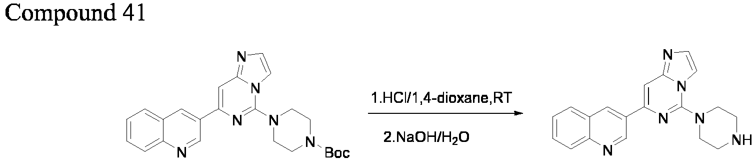

The synthesis of **compound 41** was the same as that of **compound 18** in **Example 18,** except that tert-butyl 4-(7-(quinolin-3-yl)imidazo[1,2-c]pyrimidin-5-yl)piperazine-1 carboxylate was used instead of tert-butyl 4-(4-(quinolin-3-yl)pyrimidin-2-yl)piperazine-1 carboxylate in **Example 18.** The yield was 53%.

¹H NMR (400 MHz, *DMSO*) *δ*: 9.68 (d, *J* = 2.0 Hz, 1H), 9.07 (d, *J* = 2.0 Hz, 1H), 8.12 - 8.04 (m, 3H), 7.89 (s, 1H), 7.83 - 7.76 (m, 1H), 7.71 - 7.63 (m, 2H), 3.60 - 3.54 (m, 4H), 3.12 - 3.06 (m, 4H).

MS-ESI: 331.5 [M+H]⁺.

### Example 42 Preparation of 3-(6-cyano-2-(piperazin-1-yl)pyrimidin-4-yl)quinoline (Compound 42)

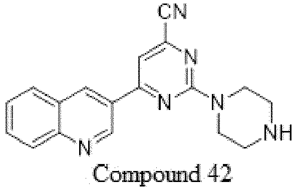

### tert-butyl 4-(4-chloro-6-cyanopyrimidin-2-yl)piperazine-1 carboxylate

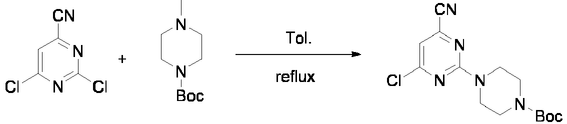

Compound 2,6-dichloropyrimidine-4-nitrile (125 mg, 0.72 mmol), tert-butyl 4-methylpiperazine-1 carboxylate (144 mg, 0.72 mmol) was dissolved in toluene (5 mL). The reaction was refluxed for 1.5 h, and TLC detected the reaction was completed. The solvent was evaporated under reduced pressure, and the residue was purified by flash column chromatography to obtain the target product (156 mg, 67%).

¹H NMR (400 MHz, *CDCl₃*) *δ* 6.79 (s, 1H), 3.93 - 3.72 (m, 4H), 3.58 - 3.43 (m, 4H), 1.49 (s, 9H).

MS-ESI: 224.3[M-100]⁺.

### tert-butyl 4-(4-cyano-6-(quinolin-3-yl)pyrimidin-2-yl)piperazine-1 carboxylate

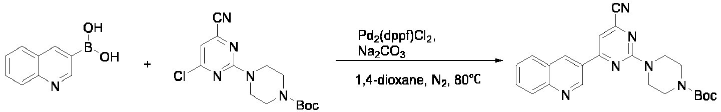

The synthesis of tert-butyl 4-(4-cyano-6-(quinolin-3-yl)pyrimidin-2-yl)piperazine-1 carboxylate was the same as that of 3-(2-chloropyrimidin-4-yl)quinoline in **Example 1,** except that tert-butyl 4-(4-chloro-6-cyanopyrimidin-2-yl)piperazine-1 carboxylate was used instead of 2,4-dichloropyrimidine in **Example 1.** The yield was 94%.

¹H NMR (400 MHz, *CDCl₃*) *δ* 9.53 (d, *J* = 2.2 Hz, 1H), 8.75 (d, *J* = 1.8 Hz, 1H), 8.18 (d, *J* = 8.6 Hz, 1H), 7.97 (d, *J* = 8.2 Hz, 1H), 7.86-7.80 (m, 1H), 7.64 (t, *J* = 7.6 Hz, 1H), 7.37 (s, 1H), 4.03 - 3.91 (m, 4H), 3.65 - 3.51 (m, 4H), 1.51 (s, 9H).

MS-ESI: 417.5[M+H]⁺.

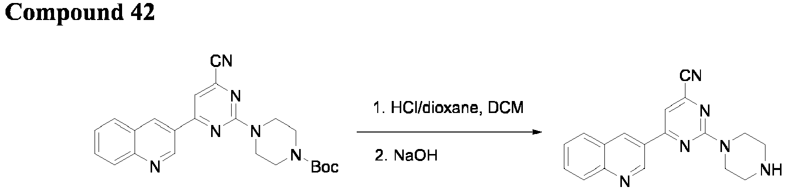

The synthesis of **compound 42** was the same as that of **compound 18** in **Example 18,** except that tert-butyl 4-(4-cyano-6-(quinolin-3-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 60%.

¹H NMR (400 MHz, *DMSO-d6*) *δ* 9.62 (d, J = 2.0 Hz, 1H), 9.19 (s, 1H), 8.13 (d, J = 8.2 Hz, 1H), 8.10 (d, J = 8.4 Hz, 1H), 7.99 (s, 1H), 7.88 (t, J = 7.4 Hz, 1H), 7.71 (t, J = 7.4 Hz, 1H), 3.87-3.75 (m, 4H), 2.88 - 2.76 (m, 4H).

MS-ESI: 317.4[M+H]⁺.

### Example 43 Preparation of 3-(5-methoxy-2-(piperazin-1-yl)pyrimidin-4-yl)quinoline (Compound 43)

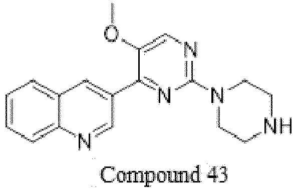

### 3-(2-chloro-5-methoxypyrimidin-4-yl)quinoline

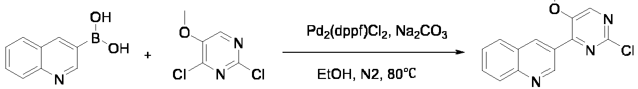

The synthesis of 3-(2-chloro-5-methoxypyrimidin-4-yl)quinoline was the same as that of 3-(2-chloropyrimidin-4-yl)quinoline in **Example 1,** except that 2,4-dichloro-5-methoxypyrimidine was used instead of 2,4-dichloropyrimidine in **Example 1.** The yield was 76%.

¹H NMR (400 MHz, *CDCl₃*) *δ* 9.62 (d, *J* = 2.2 Hz, 1H), 8.95 (d, *J* = 1.8 Hz, 1H), 8.39 (s, 1H), 8.15 (d, *J* = 8.4 Hz, 1H), 7.94 (d, *J* = 8.0 Hz, 1H), 7.85 - 7.75 (m, 1H), 7.65 - 7.58 (m, 1H), 4.07 (s, 3H).

MS-ESI: 272.3[M+H]⁺.

### tert-butyl 4-(5-methoxy-6-(quinolin-3-yl)pyrimidin-2-yl)piperazine-1 carboxylate

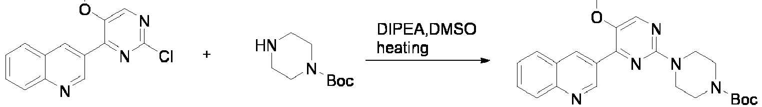

The synthesis of tert-butyl 4-(5-methoxy-6-(quinolin-3-yl)pyrimidin-2-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidine-4-ylmethylamine in **Example 1** and 3-(2-chloro-5-methoxypyrimidin-4-yl)quinoline was used instead of 3-(2-chloropyrimidin-4-yl)quinoline in **Example 1.** The yield was 68%.

1H NMR (400 MHz, CDCl₃) δ 9.63 (s, 1H), 8.91 (s, 1H), 8.26 (s, 1H), 8.14 (d, J = 8.8 Hz, 1H), 7.93 (d, J = 8.2 Hz, 1H), 7.79-7.73 (m, 1H), 7.62-7.56 (m, 1H), 3.90 (s, 3H), 3.86-3.80 (m, 4H), 3.58-3.51 (m, 4H), 1.50 (s, 9H).

MS-ESI: 422.5[M+H]⁺.

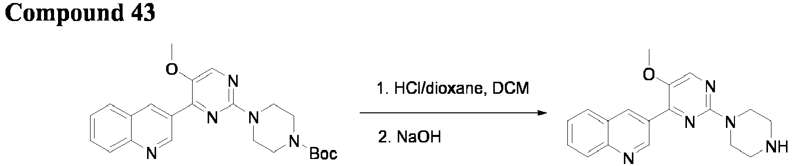

The synthesis of compound 43 was the same as that of **compound 18** in **Example 18,** except that tert-butyl 4-(5-methoxy-6-(quinolin-3-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 79%.

¹H NMR (400 MHz, DMSO-d₆) *δ* 9.48 (d, *J* = 2.2 Hz, 1H), 8.96 (d, *J* = 1.8 Hz, 1H), 8.44 (s, 1H), 8.11 (d, *J* = 7.8 Hz, 1H), 8.06 (d, *J* = 8.4 Hz, 1H), 7.86 - 7.79 (m, 1H), 7.70 - 7.62 (m, 1H), 3.88 (s, 3H), 3.72 - 3.63 (m, 4H), 2.85 - 2.75 (m, 4H).

MS-ESI: 322.5[M+H]⁺.

### Example 44 Preparation of 3-(6-(piperazin-1-yl)pyrimidin-4-yl)quinoline (Compound 44)

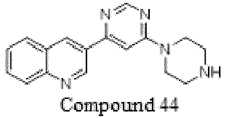

### 3-(6-chloropyrimidin-4-yl)quinoline

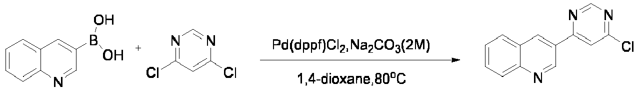

The synthesis of 3-(6-chloropyrimidin-4-yl)quinoline was the same as that of 3-(2-chloropyrimidin-4-yl)quinoline in **Example 1,** except that 4,6-dichloropyrimidine was used instead of 2,4-dichloropyrimidine in **Example 1.** The yield was 60%.

¹H NMR (400 MHz, *DMSO-d6) δ:* 9.68 (d, *J* = 4.0 Hz, 1H), 9.27 (d, *J* = 4.0 Hz, 1H), 9.20 (d, *J* = 4.0 Hz, 1H), 8.58 (d, *J* = 2.0 Hz, 1H), 8.13 (dd, *J* = 20.0, 8.0 Hz, 2H), 7.94 - 7.86 (m, 1H), 7.77 - 7.69 (m, 1H).

MS-ESI: 242.4 [M+H]⁺.

### tert-butyl 4-(6-(quinolin-3-yl)pyrimidin-4-yl)piperazine-1 carboxylate

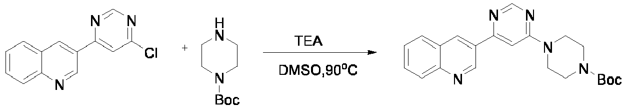

The synthesis of tert-butyl 4-(6-(quinolin-3-yl)pyrimidin-4-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidine-4-ylmethylamine in Example 1, 3-(6-chloropyrimidin-4-yl)quinoline was used instead of 3-(2-chloropyrimidin-4-yl)quinoline in **Example 1** and TEA was used instead of DIPEA. The yield was 95%.

¹H NMR (400 MHz, *DMSO-d6*) *δ:* 9.64 (d, *J* = 2.0 Hz, 1H), 9.12 (d, *J* = 2.0 Hz, 1H), 8.68 (s, 1H), 8.10 (t, *J* = 8.0 Hz, 2H), 7.84 (t, *J* = 8.0 Hz, 1H), 7.69 (t, *J* = 8.0 Hz, 1H), 7.60 (s, 1H), 3.82 - 3.75 (m, 4H), 3.50 - 3.44 (m, 4H), 1.44 (s, 9H).

MS-ESI: 392.4 [M+H]⁺.

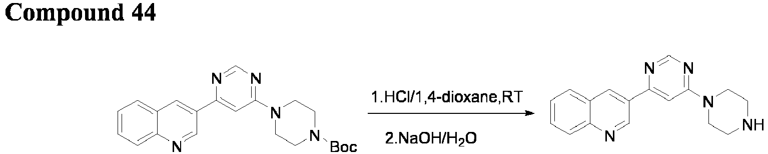

The synthesis of **compound 44** was the same as that of **compound 18** in **Example 18,** except that tert-butyl 4-(6-(quinolin-3-yl)pyrimidin-4-yl)piperazine-1 carboxylate was used instead of tert-butyl 4-(4-(quinolin-3-yl)pyrimidin-2-yl)piperazine-1 carboxylate in **Example 18.** The yield was 85%.

¹H NMR (400 MHz, *DMSO-d6*) *δ:* 9.62 (d, *J* = 2.0 Hz, 1H), 9.11 (d, *J* = 2.0 Hz, 1H), 8.63 (s, 1H), 8.09 (t, *J* = 8.0 Hz, 2H), 7.87 - 7.78 (m, 1H), 7.72 - 7.64 (m, 1H), 7.54 (s, 1H), 3.74 - 3.66 (m, 4H), 2.84 - 2.76 (m, 4H).

MS-ESI: 292.5 [M+H]⁺.

### Example 45 Preparation of 3-(9-methyl-2-(piperazin-1-yl)-9H-purin-6-yl)quinoline (Compound 45)

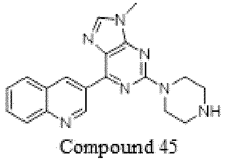

### 3-(2-chloro-9-methyl-9H-purin-6-yl)quinoline

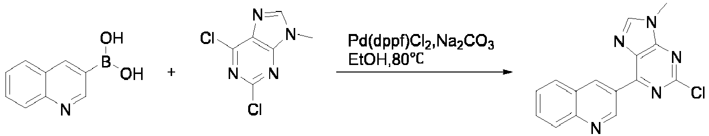

The synthesis of 3-(2-chloro-9-methyl-9H-purin-6-yl)quinoline was the same as that of 6-(2-chloropyrimidin-4-yl)quinoline in **Example 1,** except that 2,6-dichloro-9-methyl-9H-purine was used instead of 2,4-dichloropyrimidine in **Example 1.** The yield was 58%.

¹H NMR (400 MH z, DMSO-d₆) *δ:* 10.11 (d, *J* = 2.2 Hz, 1H), 9.63 (d, *J* = 2.2 Hz, 1H), 8.74 (s, 1H), 8.23 (d, *J* = 8.4 Hz, 1H), 8.12 (d, *J* = 8.4 Hz, 1H), 7.91 (t, *J* = 8.2, Hz, 1H), 7.72 (t, *J* = 8.2 Hz, 1H), 3.87 (s, 3H).

MS-ESI: 296.3 [M+H]⁺.

### tert-butyl 4-(9-methyl-6-(quinolin-3-yl)-9H-purin-2-yl)piperazine-1 carboxylate

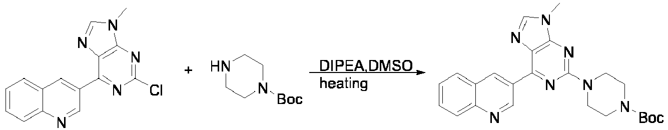

The synthesis of tert-butyl 4-(9-methyl-6-(quinolin-3-yl)-9H-purin-2-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidine-4-ylmethylamine in Example 1 and 3-(2-chloro-9-methyl-9H-purin-6-yl)quinoline was used instead of 3-(2-chloropyrimidin-4-yl)quinoline in **Example 1.** The yield was 84%.

¹H NMR (400 MHz, DMSO-d₆) *δ:* 10.14 (d, *J* = 2.1 Hz, 1H), 9.67 (d, *J* = 2.2 Hz, 1H), 8.29 (s, 1H), 8.18 (d, *J* = 8.0 Hz, 1H), 8.10 (d, *J* = 8.8 Hz, 1H), 7.86 (t, *J* = 8.6 Hz, 1H), 7.70 (t, *J* = 8.0 Hz, 1H), 3.96 - 3.89 (m, 4H), 3.73 (s, 3H), 3.55 - 3.47 (m, 4H).

MS-ESI: 446.5 [M+H]⁺.

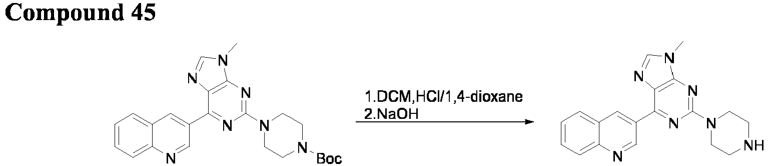

The synthesis of **compound 45** was the same as that of **compound 18** in **Example 18,** except that tert-butyl 4-(9-methyl-6-(quinolin-3-yl)-9H-purin-2-yl)piperazine-1 carboxylate was used instead of tert-butyl 4-(4-(quinolin-3-yl)pyrimidin-2-yl)piperazine-1 carboxylate in **Example 18.** The yield was 92%.

¹H NMR (400 MHz, DMSO-d₆) δ : 10.12 (d, *J* = 2.0 Hz, 1H), 9.64 (d, *J=* 2.0 Hz, 1H), 8.26 (s, 1H), 8.17 (d, *J* = 8.2 Hz, 1H), 8.10 (d, *J* = 8.4 Hz, 1H), 7.86 (t, *J* = 8.2 Hz, 1H), 7.69 (t, *J* = 8. 2 Hz, 1H), 3.86 (t, *J* = 5.0 Hz, 4H), 3.72 (s, 3H), 2.86 (t, *J* = 5.0 Hz, 4H).

MS-ESI:346.4 [M+H]⁺.

### Example 46 Preparation of 3-(5-fluoro-2-(piperazin-1-yl)pyrimidin-4-yl)quinoline (Compound 46)

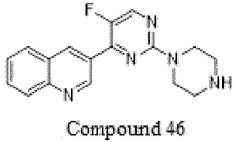

### 3-(2-chloro-5-fluoropyrimidin-4-yl)quinoline

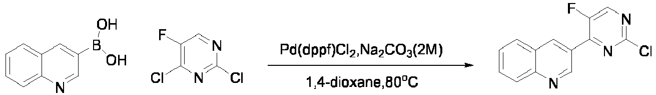

The synthesis of 3-(6-chloropyrimidin-4-yl)quinoline was the same as that of 3-(2-chloropyrimidin-4-yl)quinoline in **Example 1,** except that 2,4-dichloro-5-fluoropyrimidine was used instead of 2,4-dichloropyrimidine in **Example 1.** The yield was 84%.

MS-ESI: 260.7 [M+H]⁺.

### tert-butyl 4-(5-fluoro-4-(quinolin-3-yl)pyrimidin-2-yl)piperazine-1 carboxylate

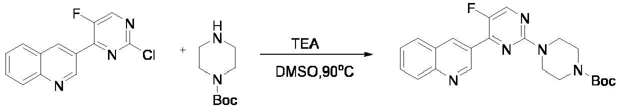

The synthesis of tert-butyl 4-(5-fluoro-4-(quinolin-3-yl)pyrimidin-2-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidine-4-ylmethylamine in **Example 1,** 3-(2-chloro-5-fluoropyrimidin-4-yl)quinoline was used instead of 3-(2-chloropyrimidin-4-yl)quinoline in **Example 1** and TEA was used instead of DIPEA. The yield was 95%.

¹H NMR (400 MHz, *DMSO-d6*) *δ*: 9.48 (d, *J* = 2.0 Hz, 1H), 9.00 (s, 1H), 8.63 (d, *J* = 4.0 Hz, 1H), 8.18 (d, *J* = 8.0 Hz, 1H), 8.10 (d, *J* = 8.0 Hz, 1H), 7.93 - 7.85 (m, 1H), 7.71 (m, 1H), 3.84 - 3.77 (m, 4H), 3.49 - 3.44 (m, 4H), 1.43 (s, 9H).

MS-ESI: 410.5[M+H]⁺.

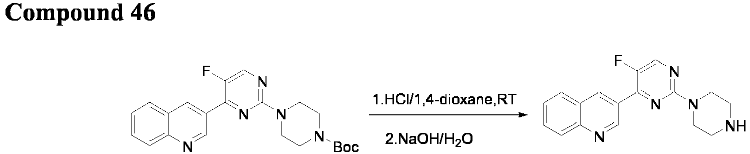

The synthesis of **compound 46** was the same as that of **compound 18** in **Example 18,** except that tert-butyl 4-(5-fluoro-4-(quinolin-3-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 75%.

¹H NMR (400 MHz, *DMSO-d6*) *δ:* 9.46 (s, 1H), 8.97 (s, 1H), 8.59 (d, *J* = 4.0 Hz, 1H), 8.18 (d, *J* = 8.0 Hz, 1H), 8.10 (d, *J* = 8.0 Hz, 1H), 7.92 - 7.84 (m, 1H), 7.70 (m, 1H), 3.77 - 3.70 (m, 4H), 2.84 - 2.76 (m, 4H).

MS-ESI: 310.4[M+H]⁺.

### Example 47 Preparation of 2-(piperazin-1-yl)-6-(quinolin-3-yl)pyrimidin-4-amine (Compound 47)

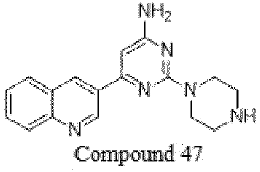

### tert-butyl 4-(4-((t-butyloxycarbonyl)amino)-6-chloropyrimidin-2-yl)piperazine-1 carboxylate

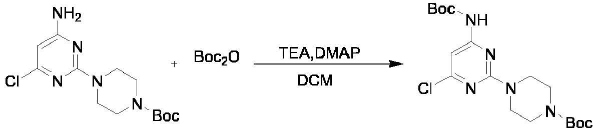

Compound tert-butyl 4-(4-amino-6-chloropyrimidin-2-yl)piperazine-1 carboxylate (395mg, 1.26mmol) was dissolved in dichloromethane (5mL), then triethylamine (254mg, 2.52mmol) was added. Di-tert-butyl dicarbonate (412 mg, 1.89 mmol) was slowly added dropwise under an ice bath, and the temperature was gradually raised to room temperature after the dropwise addition was completed. After 1h, TLC detected the reaction was basically completed. DMAP (40 mg, 0.33 mmol) was added, and the reaction was continued at room temperature. TLC detected the reaction was basically completed. The solvent was evaporated under reduced pressure, and the residue was purified by flash column chromatography to give the target product (487 mg, 93%).

¹H NMR (400 MHz, *CDCl₃*): *δ* 6.89 (s, 1H), 3.76-3.73 (m, 4H), 3.47-3.44 (m, 4H), 1.49 (s, 9H), 1.46 (s, 9H).

### tert-butyl 4-(4-((t-butyloxycarbonyl)amino)-6-(quinolin-3-yl)pyrimidin-2-yl)piperazine-1 carboxylate

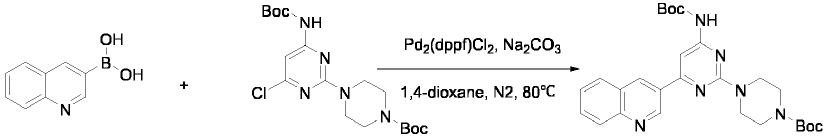

The synthesis of tert-butyl 4-(4-((t-butyloxycarbonyl)amino)-6-(quinolin-3-yl)pyrimidin-2-yl)piperazine-1 carboxylate was the same as that of 3-(2-chloropyrimidin-4-yl)quinoline in **Example 1,** except that tert-butyl 4-(4-((t-butyloxycarbonyl)amino)-6-chloropyrimidin-2-yl)piperazine-1 carboxylate was used instead of 2,4-dichloropyrimidine in **Example 1** and 1,4-dioxane was used instead of ethanol. The yield was 92%.

¹H NMR (400 MHz, *CDCl₃*): *δ* 9.62-9.57 (m, 1H), 8.77-8.75 (m, 1H), 8.17 -8.14 (m, 1H), 7.96-7.93 (m, 1H), 7.83 -7.75 (m, 1H), 7.61 -7.58 (m, 1H), 7.07- 7.06 (m, 1H), 3.90 -3.87 (m, 4H), 3.54-3.51 (m, 4H), 1.58 (s, 9H), 1.50 (s, 9H).

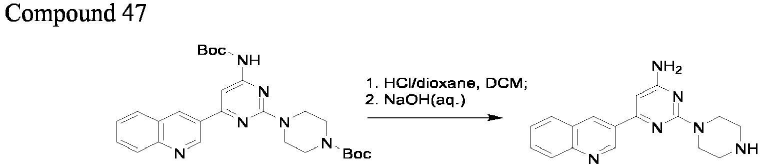

The synthesis of **compound 47** was the same as that of **compound 18** in **Example 18,** except that tert-butyl 4-(4-((t-butyloxycarbonyl)amino)-6-(quinolin-3-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 43%.

¹H NMR (400 MHz, *DMSO-d6*):δ 9.42 (d, *J* = 2.0Hz, 1H), 8.86 (d, *J* = 2.0 Hz, 1H), 8.12 (d, *J* = 8.0 Hz, 1H), 8.06 (d, *J* = 8.0 Hz, 1H), 7.82-7.79 (m, 1H), 7.67-7.64 (m, 1H), 6.61 (s, 2H), 6.46 (s, 1H), 3.78-3.75 (m, 4H), 2.82-2.80 (m, 4H).

MS-ESI: 307.4 [M+H]⁺.

### Example 48 Preparation of 6-(piperazin-1-yl)-2-(quinolin-3-yl)pyrimidin-4-amine (Compound 48)

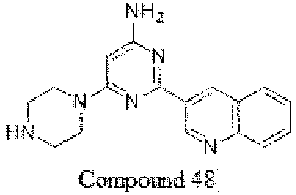

### tert-butyl 4-(4-amino-6-chloropyrimidin-2-yl)piperazine-1 carboxylate and tert-butyl 4-(6-amino-2-chloropyrimidin-4-yl)piperazine-1 carboxylate

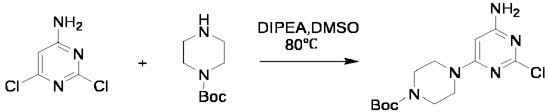

The synthesis of tert-butyl 4-(6-amino-2-chloropyrimidin-4-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidine-4-ylmethylamine in **Example 1** and 2,6-dichloropyrimidin-4-amine was used instead of 3-(2-chloropyrimidin-4-yl)quinoline in **Example 1.** The yield was 13%.

¹H NMR (400 MHz, *CDCl₃*): *δ* 5.41 (s, 1H), 3.56 -3.48 (m, 8H), 1.48 (s, 9H). tert-butyl 4-(6-amino-2-(quinolin-3-yl)pyrimidin-4-yl)piperazine-1 carboxylate

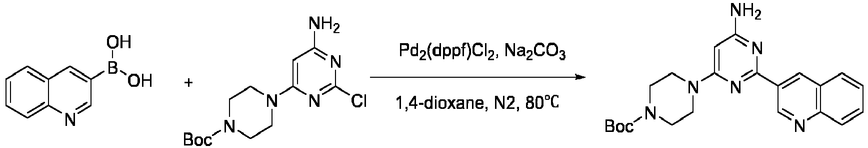

The synthesis oftert-butyl 4-(6-amino-2-(quinolin-3-yl)pyrimidin-4-yl)piperazine-1 carboxylate was the same as that of 3-(2-chloropyrimidin-4-yl)quinoline in **Example 1,** except that tert-butyl 4-(6-amino-2-chloropyrimidin-4-yl)piperazine-1 carboxylate was used instead of 2,4-dichloropyrimidine in **Example 1** and 1,4-dioxane was used instead of ethanol. The yield was 46%.

1H NMR (400 MHz, *CDCl₃*): *δ* 9.84 (d, *J* = 2.0 Hz, 1H), 9.06 (d, *J* = 2.0 Hz, 1H), 8.15 (d, *J* = 12.0Hz, 1H), 7.95 -7.93 (m, 1H), 7.76-7.72 (m, 1H), 7.58-7.54 (m, 1H), 5.59 (s, 1H), 4.70 (s, 2H), 3.72-3.69 (m, 4H), 3.59-3.56 (m, 4H), 1.50 (s, 9H).

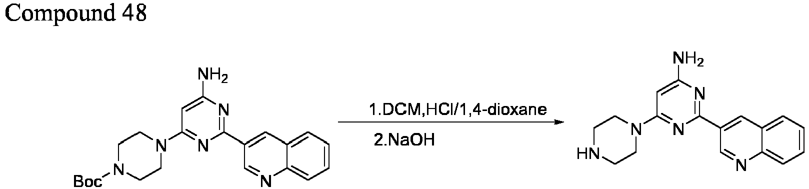

The synthesis of **compound 48** was the same as that of **compound 18** in **Example 18,** except that tert-butyl 4-(6-amino-2-(quinolin-3-yl)pyrimidin-4-yl)piperazine-1 carboxylate was used instead of tert-butyl 4-(4-(quinolin-3-yl)pyrimidin-2-yl)piperazine-1 carboxylate in **Example 18.** The yield was 53%.

MS-ESI: 307.3 [M+H]⁺.

### Example 49 Preparation of 4-(2-(piperazin-1-yl)-6-(quinolin-3-yl)pyrimidin-4-yl)morpholine (Compound 49)

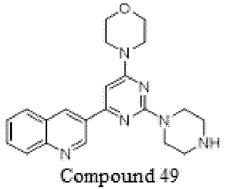

### tert-butyl 4-(4-chloro-6-morpholinopyrimidin-2-yl)piperazine-1 carboxylate

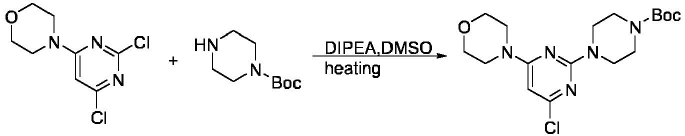

The synthesis of tert-butyl 4-(4-chloro-6-morpholinopyrimidin-2-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidine-4-ylmethylamine and 4-(2,6-dichloropyrimidin-4-yl)morpholine was used instead of 3-(2-chloropyrimidin-4-yl)quinoline. The yield was 89%.

¹H NMR (400 MHz, *CDCl₃*) *δ* 5.85 (s, 1H), 3.78 - 3.70 (m, 8H), 3.58 - 3.51 (m, 4H), 3.49 - 3.42 (m, 4H).

MS-ESI: 384.4[M+H]⁺.

### tert-butyl 4-(4-morpholin-6-(quinolin-3-yl)pyrimidin-2-yl)piperazine-1 carboxylate

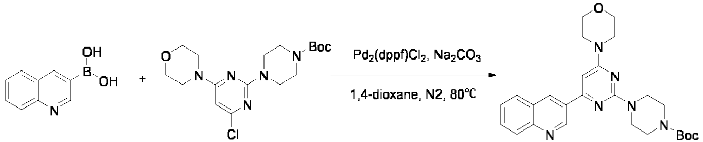

The synthesis of tert-butyl 4-(4-morpholin-6-(quinolin-3-yl)pyrimidin-2-yl)piperazine-1 carboxylate was the same as that of 3-(2-chloropyrimidin-4-yl)quinoline in **Example 1,** except that tert-butyl 4-(4-chloro-6-morpholinopyrimidin-2-yl)piperazine-1 carboxylate was used instead of 2,4-dichloropyrimidine in **Example 1** and 1,4-dioxane was used instead of ethanol. The yield was 74%.

¹H NMR (400 MHz, *CDCl₃*) *δ* 9.47 (d, *J* = 2.2 Hz, 1H), 8.73 (d, *J* = 1.6 Hz, 1H), 8.15 (d, *J* = 8.6 Hz, 1H), 7.93 (d, *J* = 8.2 Hz, 1H), 7.79 - 7.71 (m, 1H), 7.59 (t, *J* = 7.6 Hz, 1H), 6.44 (s, 1H), 3.94 - 3.86 (m, 4H), 3.86 - 3.80 (m, 4H), 3.72 - 3.66 (m, 4H), 3.57 - 3.51 (m, 4H), 1.50 (s, 9H).

MS-ESI: 477.5[M+H]⁺.

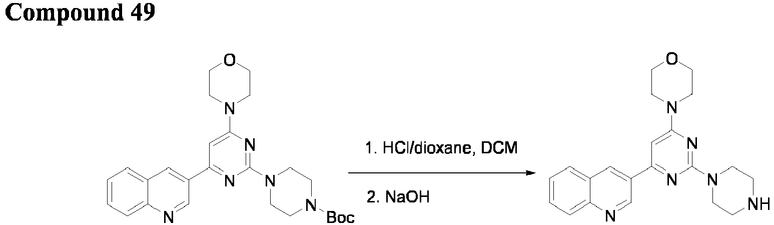

The synthesis of **compound 49** was the same as that of **compound 18** in **Example 18,** except that tert-butyl 4-(4-morpholin-6-(quinolin-3-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 81%.

¹H NMR (400 MHz, *DMSO-d₆*) *δ* 9.58 (d, *J* = 2.1 Hz, 1H), 9.03 (d, *J* = 1.8 Hz, 1H), 8.08 (t, *J* = 9.1 Hz, 2H), 7.86 - 7.76 (m, 1H), 7.66 (t, *J* = 7.1 Hz, 1H), 6.89 (s, 1H), 3.81 - 3.72 (m, 4H), 3.71-3.63 (m, 8H), 2.84 - 2.78 (m, 4H).

MS-ESI:377.5[M+H]⁺.

### Example 50 Preparation of 3-(5-cyclopropyl-2-(piperazin-1-yl)pyrimidin-4-yl)quinoline (Compound 50)

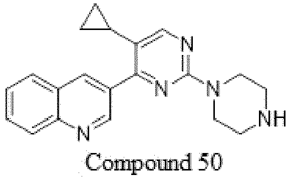

### 3-(2-chloro-5-cyclopropylpyrimidin-4-yl)quinoline

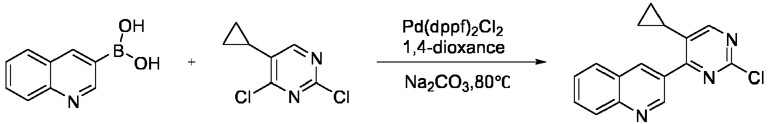

The synthesis of 3-(2-chloro-5-cyclopropylpyrimidin-4-yl)quinoline was the same as that of 3-(2-chloropyrimidin-4-yl)quinoline in **Example 1,** except that 2,4-dichloro-5-cyclopropylpyrimidine was used instead of 2,4-dichloropyrimidine in **Example 1** and 1,4-dioxane was used instead of ethanol. The yield was 67%.

¹H NMR (400 MHz, *CDCl₃*) *δ* 9.36 (d, *J* = 2.2 Hz, 1H), 8.66 (d, *J* = 1.7 Hz, 1H), 8.40 (s, 1H), 8.18 (d, *J* = 8.4 Hz, 1H), 7.94 (d, *J* = 8.1 Hz, 1H), 7.86 - 7.76 (m, 1H), 7.66 - 7.61 (m, 1H), 2.05 (tt, *J* = 8.5, 5.5 Hz, 1H), 1.15 - 1.08 (m, 2H), 0.80 (q, *J* = 5.2 Hz, 2H).

MS-ESI: 282.4 [M+H]⁺.

### tert-butyl 4-(5-cyclopropyl-4-(quinolin-3-yl)pyrimidin-2-yl)piperazine-1 carboxylate

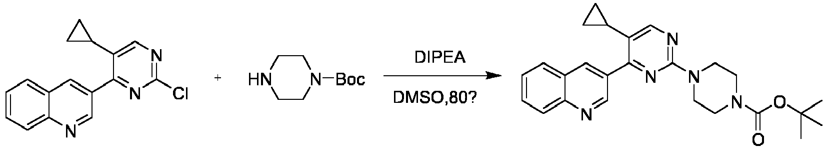

The synthesis of tert-butyl 4-(5-cyclopropyl-4-(quinolin-3-yl)pyrimidin-2-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidine-4-ylmethylamine in **Example 1** and 3-(2-chloro-5-cyclopropylpyrimidin-4-yl)quinoline was used instead of 3-(2-chloropyrimidin-4-yl)quinoline in **Example 1.** The yield was 79%.

¹H NMR (400 MHz, CDCl₃) *δ* 9.37 (d, *J* = 2.0 Hz, 1H), 8.62 (d, *J* = 1.8 Hz, 1H), 8.24 (s, 1H), 8.17 (d, *J* = 8.5 Hz, 1H), 7.92 (d, *J* = 8.1 Hz, 1H), 7.79 (t, *J* = 7.7 Hz, 1H), 7.59 (dd, *J* = 20.7, 13.5 Hz, 1H), 3.90 - 3.80 (m, 4H), 3.58 - 3.46 (m, 4H), 1.93 (dq, *J* = 8.4, 5.4 Hz, 1H), 1.49 (s, 10H), 0.90 (s, 2H), 0.57 (d, *J=* 5.4 Hz, 2H).

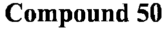

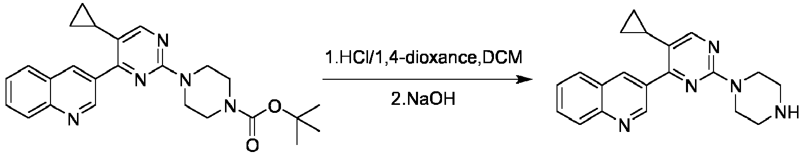

The synthesis of **compound 50** was the same as that of **compound 18** in **Example 18,** except that tert-butyl 4-(5-cyclopropyl-4-(quinolin-3-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 81%.

¹H NMR (400 MHz, *DMSO-d6) δ* 9.27 (d, J = 2.2 Hz, 1H), 8.79 (d, J = 1.9 Hz, 1H), 8.29 (s, 1H), 8.13 - 8.01 (m, 2H), 7.86 - 7.79 (m, 1H), 7.68 (t, J = 7.5 Hz, 1H), 3.79 - 3.66 (m, 4H), 2.84 - 2.70 (m, 4H), 2.06 - 1.94 (m, 2H), 0.87 - 0.71 (m, 3H), 0.53 (q, J = 5.9 Hz, 2H).

MS-ESI: 331.4 [M+H]⁺.

### Example 51 Preparation of 3-(6-(4-methoxyphenyl)-2-(piperazin-1-yl)pyrimidin-4-yl)quinoline (Compound 51)

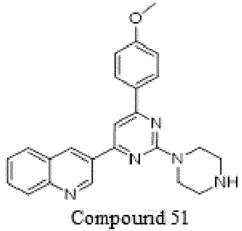

### 3-(2-chloro-6-(4-methoxyphenyl)pyrimidin-4-yl)quinoline

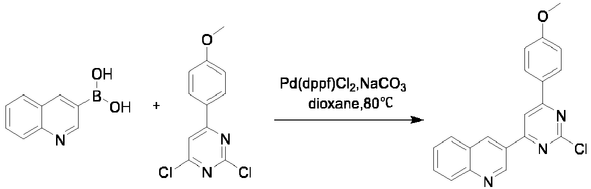

The synthesis of 3-(2-chloro-6-(4-methoxyphenyl)pyrimidin-4-yl)quinoline was the same as that of 3-(2-chloropyrimidin-4-yl)quinoline in **Example 1,** except that 2,4-dichloro-6-(4-methoxyphenyl)pyrimidine was used instead of 2,4-dichloropyrimidine in **Example 1.** The yield was 88%.

¹H NMR (400 MHz, *CDCl₃*) *δ* :9.57 (d, *J* = 4.0 Hz, 1H), 8.99 (d, *J* = 4.0 Hz, 1H), 8.26-8.13 (m, 3H), 8.11 (s, 1H), 8.00 (d, *J* = 8.0 Hz, 1H), 7.83 (t, *J* = 8.0 Hz, 1H), 7.65 (t, *J* = 8.0 Hz, 1H), 7.06 (d, *J* = 8.0 Hz, 2H), 3.92 (s, 3H).

MS-ESI: 348.4 [M+H]⁺.

### tert-butyl 4-(4-(4-methoxyphenyl)-6-(quinolin-3-yl)pyrimidin-2-yl)piperazine-1 carboxylate

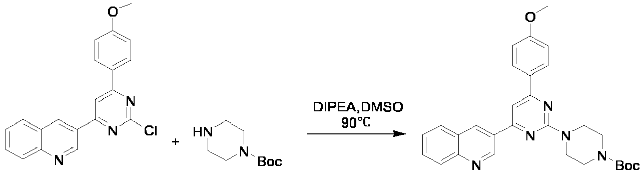

The synthesis of tert-butyl 4-(4-(4-methoxyphenyl)-6-(quinolin-3-yl)pyrimidin-2-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidine-4-ylmethylamine and 3-(2-chloro-6-(4-methoxyphenyl)pyrimidin-4-yl)quinoline was used instead of 3-(2-chloropyrimidin-4-yl)quinoline. The yield was 70%.

¹H NMR (400 MHz, *CDCl₃*) *δ* :9.61 (d, *J* = 4.0 Hz, 1H), 8.84 (d, *J* = 4.0 Hz, 1H), 8.19-8.13 (m, 3H), 7.97 (d, *J* = 8.0 Hz, 1H), 7.78 (t, *J* = 8.0 Hz, 1H), 7.62 (t, *J* = 8.0 Hz, 1H), 7.51 (s, 1H), 7.03 (d, *J* = 8.0 Hz, 2H), 4.15-3.99 (m, 4H), 3.90 (s, 3H), 3.66-3.55 (m, 4H), 1.52 (s, 9H).

MS-ESI: 498.4 [M+H]⁺.

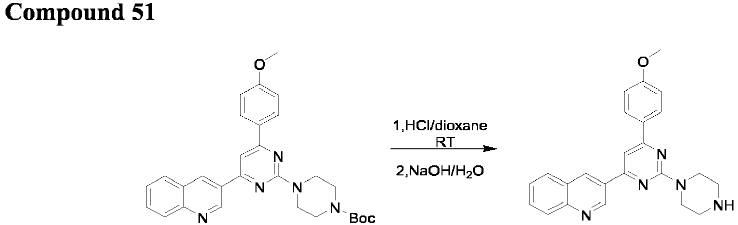

The synthesis of **compound 51** was the same as that of **compound 18** in **Example 18,** except that tert-butyl 4-(4-(4-methoxyphenyl)-6-(quinolin-3-yl)pyrimidin-2-yl)piperazine-1 carboxylate was used instead of tert-butyl 4-(4-(quinolin-3-yl)pyrimidin-2-yl)piperazine-1 carboxylate in **Example 18.** The yield was 74%.

¹H NMR (400 MHz, *DMSO-d6) δ* :9.73 (d, *J=* 4.0 Hz, 1H), 9.23 (d, *J* = 4.0Hz, 1H), 8.30 (d, *J* = 8.0 Hz, 2H), 8.13 (dd, *J* = 19.3, 8.1 Hz, 2H), 7.95 (s, 1H), 7.85 (t, *J* = 8.0 Hz, 1H), 7.70 (t, *J* = 8.0 Hz, 1H), 7.10 (d, *J* = 8.0 Hz, 2H), 3.96-3.87 (m, 4H), 3.86 (s, 3H), 2.89-2.82 (m, 4H), 1.23 (s, 1H).

MS-ESI: 398.4 [M+H]⁺.

### Example 52 Preparation of 3-(1-methyl-6-(piperazin-1-yl)-1H-pyrazolo[3,4-d]pyrimidin-4-yl)quinoline (Compound 52)

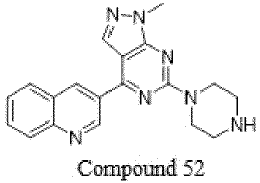

### 3-(6-chloro-1-methyl-1H-pyrazolo[3,4-d]pyrimidin-4-yl)quinoline

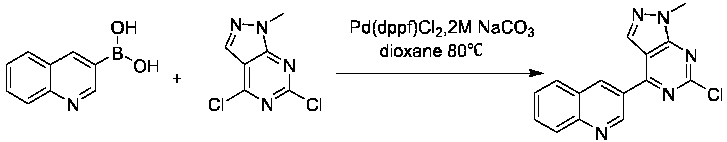

The synthesis of 3-(6-chloro-1-methyl-1H-pyrazolo[3,4-d]pyrimidin-4-yl)quinoline was the same as that of 3-(2-chloropyrimidin-4-yl)quinoline in **Example 1,** except that 4,6-dichloro-1-methyl-1H-pyrazole[3,4-d]pyrimidine was used instead of 2,4-dichloropyrimidine and 1,4dioxane was used instead of acetonitrile. The product was used directly without further purification.

MS-ESI: 296.2 [M+H]⁺.

### tert-butyl 4-(1-methyl-4-(quinolin-3-yl)-1H-pyrazole[3,4-d]pyrimidin-6-yl)piperazine-1 carboxylate

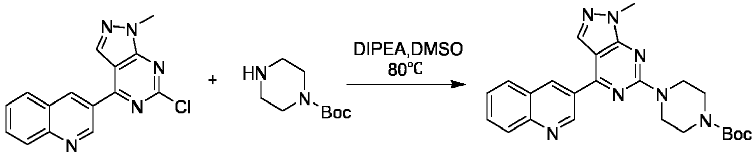

The synthesis of 3-(6-chloro-1-methyl-1H-pyrazolo[3,4-d]pyrimidin-4-yl)quinoline was the same as that of (1-(4-(quinolin-3-yl)pyrimidin-2-yl)piperidin-4-yl)methylamine in **Example 1,** except that tert-butyl piperazine-1 carboxylate was used instead of piperidine-4-ylmethylamine. The yield was 91%.

MS-ESI: 296.2 [M+H]⁺.

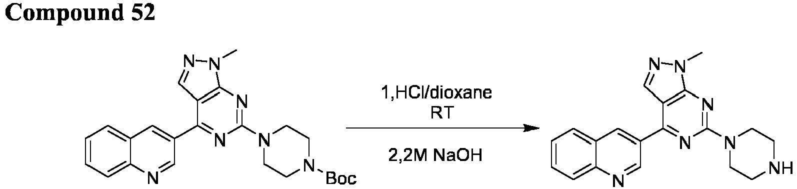

The synthesis of **compound 52** was the same as that of **compound 18** in **Example 18,** except that compound tert-butyl 4-(1-methyl-4-(quinolin-3-yl)-1H-pyrazole[3,4-d]pyrimidin-6-yl)piperazine-1 carboxylate was used instead of tert-butyl 4-(4-(quinolin-3-yl)pyrimidin-2-yl)piperazine-1 carboxylate in **Example 18.** The yield was 82%.

¹H NMR (400 MHz, *DMSO-d6*) δ 9.66 (d, *J* = 2.0 Hz, 1H), 9.18 (d, *J* = 2.0 Hz, 1H), 8.55 (s, 1H), 8.27 (d, *J* = 8.0 Hz, 1H), 8.11 (d, *J* = 8.0 Hz, 1H), 7.92 - 7.84 (m, 1H), 7.74 - 7.69 (m, 1H),3.93 - 23.89 (m, 4H) , 3.88 (s, 3H), 2.90 - 2.79 (m, 4H).

MS-ESI: 346.4 [M+H]⁺.

### Example 53 Preparation of 3-(5-methyl-6-(piperazin-1-yl)pyrimidin-4-yl)quinoline (Compound 53)

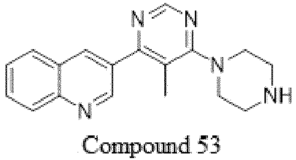

### 3-(6-chloro-5-methylpyrimidin-4-yl)quinoline

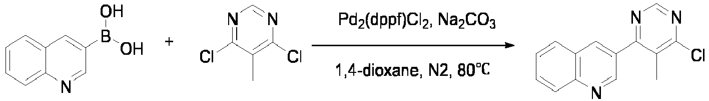

The synthesis of 3-(6-chloro-5-methylpyrimidin-4-yl)quinoline was the same as that of 3-(2-chloropyrimidin-4-yl)quinoline in **Example 1,** except that 4,6-dichloro-5-methylpyrimidine was used instead of 2,4-dichloropyrimidine. The yield was 49%.

¹H NMR (400 MHz, *CDCl₃*): *δ* 9.12 (d, *J* = 4.0Hz, 1H), 8.95 (s, 1H), 8.41 (d, *J* = 2.0Hz, 1H), 8.19 (dd, *J* = 16.0,4.0 Hz, 1H), 7.92 (dd, *J* = 8.0, 2.0Hz, 1H), 7.85-7.80 (m, 1H), 7.66-7.62 (m, 1H), 2.53 (s, 3H).

MS-ESI: 256.3 [M+H]⁺.

### tert-butyl 4-(5-methyl-6-(quinolin-3-yl)pyrimidin-4-yl)piperazine-1 carboxylate

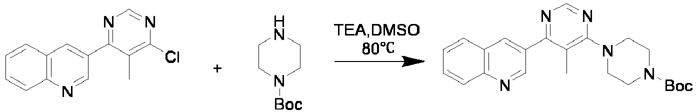

The synthesis of tert-butyl 4-(5-methyl-6-(quinolin-3-yl)pyrimidin-4-yl)piperazine-1 carboxylate was the same as that of (1-(4-(quinolin-3-yl)pyrimidin-2-yl)piperidin-4-yl)methylamine in **Example 1,** except that tert-butyl piperazine-1 carboxylate was used instead of piperidine-4-ylmethylamine, 3-(6-chloro-5-methylpyrimidin-4-yl)quinoline was used instead of 3-(2-chloropyrimidin-4-yl)quinoline and TEA was used instead of DIPEA. The yield was 89%.

1H NMR (400 MHz, *CDCl₃*): *δ* 9.15 (d, *J* =8.0Hz, 1H), 8.76 (s, 1H), 8.44-8.43 (m, 1H), 8.16 (d, *J* = 2.0 Hz, 1H), 7.91 (dd, *J* = 8.0, 4.0Hz, 1H), 7.81-7.77 (m, 1H), 7.63-7.59 (m, 1H), 3.62-3.59 (m, 4H), 3.48-3.45 (m, 4H), 2.31 (s, 3H), 1.50 (s, 9H).

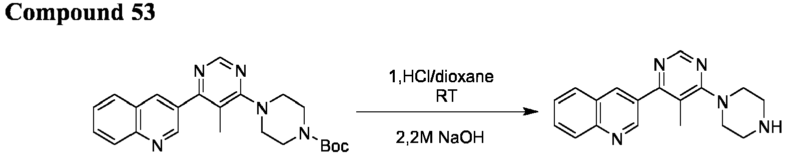

The synthesis of **compound 53** was the same as that of **compound 18** in **Example 18,** except that tert-butyl 4-(5-methyl-6-(quinolin-3-yl)pyrimidin-4-yl)piperazine-1 carboxylate was used instead of tert-butyl 4-(4-(quinolin-3-yl)pyrimidin-2-yl)piperazine-1 carboxylate in **Example 18.** The yield was 82%.

¹H NMR (400 MHz, *DMSO-d₆*): *δ* 9.17 (d, *J* = 4.0Hz, 1H), 8.69-8.66(m, 2H), 8.09 (d, *J* = 8.0Hz, 2H), 7.87-7.83 (m, 1H), 7.69 (t, *J* = 8.0 Hz, 1H), 3.42-3.35 (m, 4H), 2.85 (t, *J* =8.0 Hz, 4H), 2.27 (s, 3H).

MS-ESI: 306.3 [M+H]⁺.

### Example 54 Activity detection of CTLA-4 small molecule degradation agent (detection of protein-protein interaction reporting system, primary screening of CTLA-4 protein expression level)

HEK293 cells were spread on 96-well plates, and LRBA and CTLA-4 reporting system were co-transfected into HEK293 cells after 24h. After 24h, different compounds with final concentrations of 0.01, 0.033, 0.10, 0.33, 1.00, 3.33, 10.00, 33.33, and 100.00 µM were added. 24h after dosing, the cell culture medium was removed and the cells were washed with cold PBS. Then the activity of the compounds were detected by the dual luciferase assay kit, and IC₅₀ values were calculated according to the results.

For the compounds with strong activity (IC₅₀<200nM), the degradation of CTLA-4 protein was further detected by Western Blot.
IC₅₀: Concentration of compounds that inhibit 50% of reporting system activity
The size of IC₅₀ is divided into four levels:
++++, IC₅₀ < 200nM; +++, IC₅₀ is 200-1000nM; ++, IC₅₀ is 1000-2000nM; +, IC₅₀> 2000nM.

IC₅₀ values of the compounds of the present invention are shown below in Table 1.

**Table 1 Effects of compounds on the expression level of CTLA-4 protein (protein-protein interaction reporting system)**

| **Compound** | **Structure** | **In vitro activity** |
|---|---|---|
| Compound 1 | 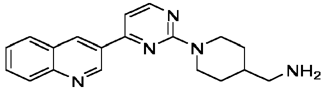 | ++++ |
| Compound 2 | 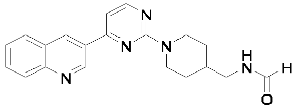 | + |
| Compound 3 | 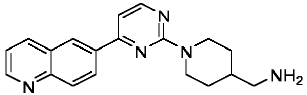 | + |
| Compound 4 | 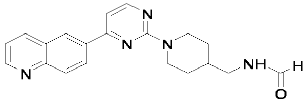 | + |
| Compound 5 | 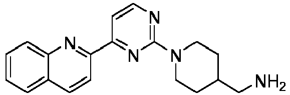 | + |
| Compound 6 | 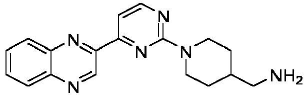 | + |
| Compound 7 | 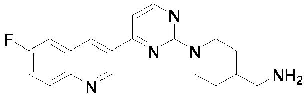 | +++ |
| Compound 8 | 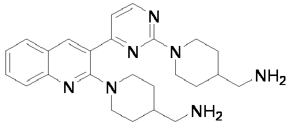 | ++ |
| Compound 9 | 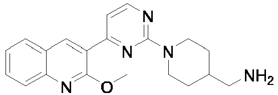 | ++++ |
| Compound 10 | 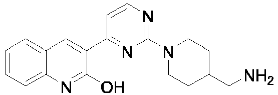 | ++++ |
| Compound 11 | 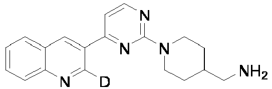 | ++++ |
| Compound 12 | 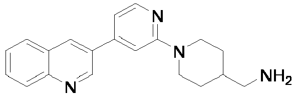 | + |
| Compound 13 | 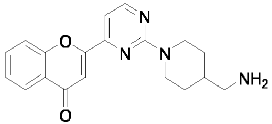 | + |
| Compound 14 | 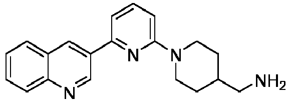 | + |
| Compound 15 | 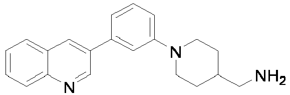 | + |
| Compound 16 | 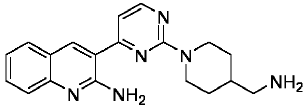 | + |
| Compound 17 | 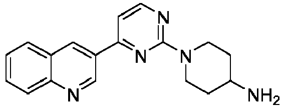 | +++ |
| Compound 18 | 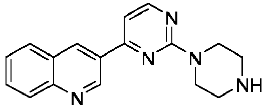 | ++++ |
| Compound 19 | 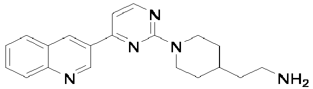 | +++ |
| Compound 20 | 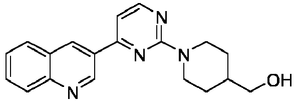 | + |
| Compound 21 | 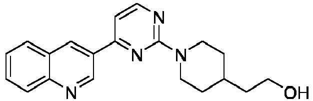 | + |
| Compound 22 | 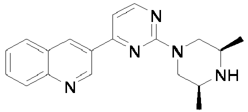 | + |
| Compound 23 | 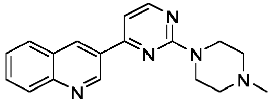 | + |
| Compound 24 | 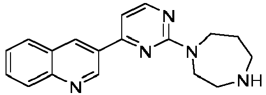 | + |
| Compound 25 | 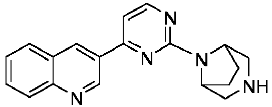 | + |
| Compound 26 | 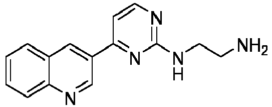 | ++++ |
| Compound 27 | 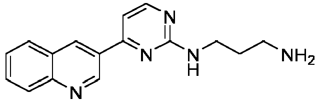 | ++ |
| Compound 28 | 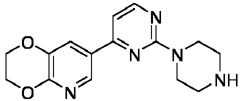 | + |
| Compound 29 | 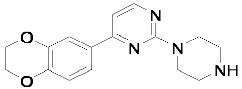 | + |
| Compound 30 | 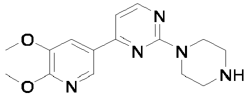 | + |
| Compound 31 | 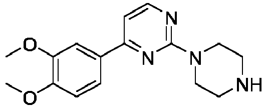 | + |
| Compound 32 | 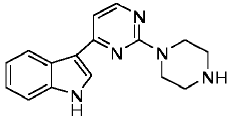 | +++ |
| Compound 33 | 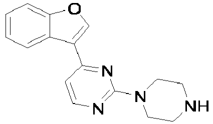 | +++ |
| Compound 34 | 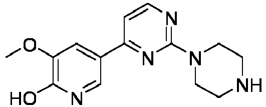 | + |
| Compound 35 | 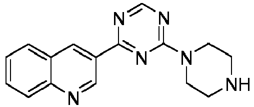 | ++++ |
| Compound 36 | 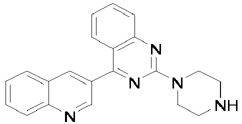 | +++ |
| Compound 37 | 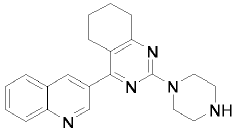 | +++ |
| Compound 38 | 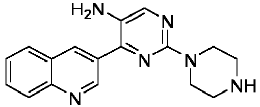 | ++++ |
| Compound 39 | 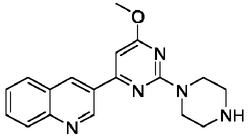 | ++++ |
| Compound 40 | 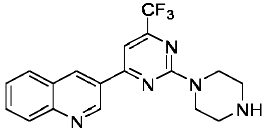 | ++++ |
| Compound 41 | 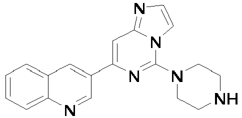 | +++ |
| Compound 42 | 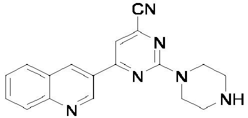 | ++++ |
| Compound 43 | 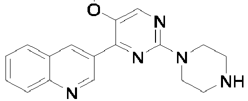 | ++++ |
| Compound 44 | 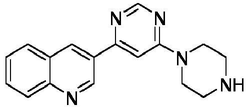 | ++++ |
| Compound 45 | 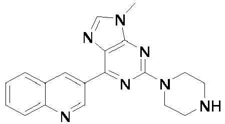 | +++ |
| Compound 46 | 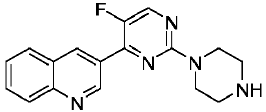 | ++++ |
| Compound 47 | 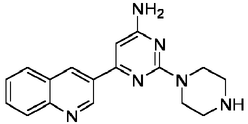 | ++++ |
| Compound 48 | 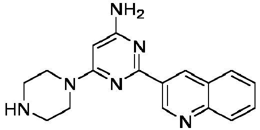 | ++ |
| Compound 49 | 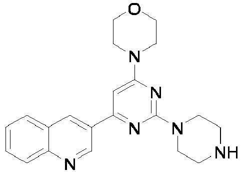 | +++ |
| Compound 50 | 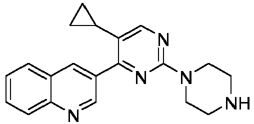 | ++++ |
| Compound 51 | 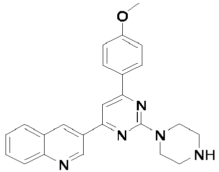 | ++++ |
| Compound 52 | 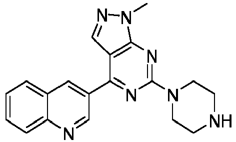 | ++ |
| Compound 53 | 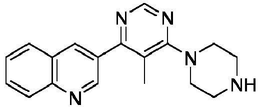 | ++++ |
| Comparing compound 1 | 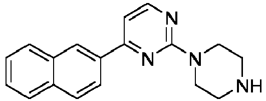 | + |

### Example 55 In vivo activity research of xenograft model (MC-38)

1. Cell culture: Mouse colon cancer MC-38 cells were adherently cultured in vitro. The culture conditions were RPMI-1640 medium with 10% fetal bovine serum, 100 U/mL penicillin and 100 µg/mL streptomycin, 37°C 5% CO₂ incubator culture. Passaged with routine processing two to three times a week. When the cell saturation was 80%-90% and the number reached the requirement, the cell count was collected, and the cell concentration was adjusted for experimental inoculation.
2. Animals: C57BL/6 mice, female, 7 weeks old, 18-20 grams in weight, 8 mice per group.
3. Tumor inoculation: 0.02mL (0.2×10⁶) MC-38 cells were subcutaneously inoculated into the right back of each mouse. On the 7th day after inoculation, the tumor volumes of the animals were measured, and then the groups were randomized according to tumor volume and administration was started.
4. Experimental indicators: The experimental indicators were used to examine whether the tumor growth is inhibited, delayed or cured. Tumor diameters were measured with vernier calipers two to three times a week. The calculation formula of tumor volume is: V = 0.5a × b², wherein a and b respectively represent the long and short diameters of tumors. The anti-tumor effect of the compounds was expressed by TGI (%). TGI (%), reflecting tumor growth inhibition rate. Calculation of TGI(%): TGI(%)=[(1-(average tumor volume at the end of administration of a treatment group - average tumor volume at the beginning of administration of the treatment group)) / (average tumor volume at the end of treatment of the solvent control group - average tumor volume at the beginning of treatment of the solvent control group)] × 100%.
5. Animal experiment grouping, administration program and experimental results:

| Group | Compound therapy | Dose (mg/kg) | Route of administration | Frequency of administration | TGI(%) |
|---|---|---|---|---|---|
| 1 | Solvent control | / | Oral | Once a day | / |
| 2 | Compound 18 | 30 | Oral | Once a day | 90% |
| 3 | Compound 44 | 30 | Oral | Once a day | 85% |

It should be understood that the above embodiments are all exemplary, and are not intended to include all possible embodiments contained in the claims. Without departing from the scope of the present disclosure, various modifications and changes may also be made on the basis of the above embodiments. Likewise, each technical feature of the above embodiments can also be arbitrarily combined to form additional embodiments of the present invention that may not be explicitly described. Therefore, the above embodiments only represent several embodiments of the present invention, and are not intended to limit the protection scope of the present invention.

## Claims

1. A compound with the structure of formula I or a pharmaceutically acceptable salt, an ester, a deuterated product, an isomer, a solvate, a prodrug, or an isotopic label thereof: 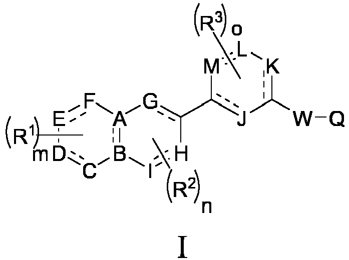 wherein,
A, B, C, D, E, F, G, H, I, J, K, L, and M are independently selected from a direct bond, CR⁶, CR⁶R¹⁴, N, NR⁷, O, S and C=O;
each of R¹, R², R³, R⁶, R⁷ and R¹⁴ is independently selected from hydrogen, deuterium, an unsubstituted or substituted alkyl group, an unsubstituted or substituted alkenyl group, an unsubstituted or substituted alkynyl group, an unsubstituted or substituted cycloalkyl group, an unsubstituted or substituted heterocycloalkyl group, halogen, -OH, an unsubstituted or substituted alkoxy group, an unsubstituted or substituted arylalkyl group, an unsubstituted or substituted heteroaryl alkyl group, an unsubstituted or substituted aryl ether group, an unsubstituted or substituted heteroaryl ether group, - CN, -N(R⁴R⁵), -NO₂, -N₃, a boronic acid group, an unsubstituted or substituted boronic ester group, a carboxyl group, an ester group, an unsubstituted or substituted carbamoyl group, an unsubstituted or substituted aryl group, an unsubstituted or substituted heteroaryl group, an unsubstituted or substituted thioether group, an unsubstituted or substituted sulfoxide group, an unsubstituted or substituted sulfone group, an unsubstituted or substituted sulfonamide group, 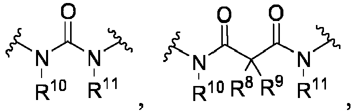 an unsubstituted or substituted phosphate ester group, wherein R⁴, R⁵, R⁸, R⁹, R¹⁰ and R¹¹ are independently selected from hydrogen, deuterium, a C₁₋₆ alkyl group, a C₃₋₇ cycloalkyl group, an unsubstituted or substituted aryl group, an unsubstituted or substituted heteroaryl group, R⁴ and R⁵, R⁸ and R⁹ can be connected with adjacent nitrogen atoms or carbon atoms to form a ring;
or two adjacent R¹ and/or two adjacent R³ can also be connected to form an unsubstituted or substituted cycloalkyl group, an unsubstituted or substituted heterocycloalkyl group, an unsubstituted or substituted aryl group or an unsubstituted or substituted heteroaryl group;
m, n and o are independently selected from integers from 0 to 4;
W is selected from the direct bond, an unsubstituted or substituted aryl group, an unsubstituted or substituted heteroaryl group, an unsubstituted or substituted cycloalkyl group, an unsubstituted or substituted heterocycloalkyl group, an unsubstituted or substituted bridged cycloalkyl group, an unsubstituted or substituted bridged heterocycloalkyl group, an unsubstituted or substituted spiro cycloalkyl group, an unsubstituted or substituted spiro heterocycloalkyl group, an unsubstituted or substituted alkyl group, an unsubstituted or substituted heteroalkyl group, an unsubstituted or substituted alkenyl group, an unsubstituted or substituted heteroalkenyl group, an unsubstituted or substituted alkynyl group, an unsubstituted or substituted heteroalkynyl group, an unsubstituted or substituted -N(R¹²R¹³), an unsubstituted or substituted aminoalkyl group, an unsubstituted or substituted aminoalkylamino group,
an unsubstituted or substituted 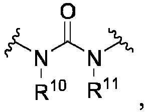 an unsubstituted or substituted 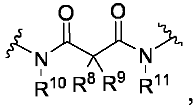 wherein R¹² and R¹³ are independently selected from hydrogen, deuterium, an unsubstituted or substituted C₁₋₆ alkyl group, a C₃₋₇ cycloalkyl group, an unsubstituted or substituted alkylamino group, an unsubstituted or substituted aryl group, an unsubstituted or substituted heteroaryl, or R¹² and R¹³ can be connected to form a ring;
Q is -H, -NH₂, -OH, -alkyl-NHC(=O)H, a cycloalkyl group, an unsubstituted or substituted alkylacyl group, an unsubstituted or substituted alkylhydroxy group, an unsubstituted or substituted alkenylhydroxy group, an unsubstituted or substituted alkynylhydroxy group, an unsubstituted or substituted alkylamino group, an unsubstituted or substituted sulfonamide group, an unsubstituted or substituted alkylsulfonamide group, an amino acid residue, 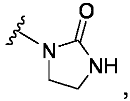 a sulfonamide group, a sulfonyl hydrazine group, an unsubstituted or substituted aryl group, an unsubstituted or substituted heteroaryl group, an unsubstituted or substituted 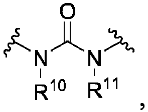 an unsubstituted or substituted 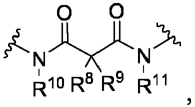 an unsubstituted or substituted -(CH₂ )ₙ₁ -(M)ₙ₂ -(CH₂ )ₙ₃ -(M)ₙ₄ -(CH₂)ₙ₅ -(M)ₙ₆, wherein each M is independently selected from O, OH, S, SO, SO₂ and an unsubstituted or substituted amino group, each n1, n2, n3, n4, n5, and n6 are independently selected from integers from 0 to 6;
or W and Q can be connected or fused to form a substituted or unsubstituted cycloalkyl group, a heterocycloalkyl group, an aryl group, or a heteroaryl group.

2. The compound with the structure of formula I or a pharmaceutically acceptable salt, an ester, a deuterated product, an isomer, a solvate, a prodrug, or an isotopic label thereof according to claim 1, **characterized in that** at least one of C, G, and I is an N atom.

3. The compound with the structure of formula I or a pharmaceutically acceptable salt, an ester, a deuterated product, an isomer, a solvate, a prodrug, or an isotopic label thereof according to claim 1, **characterized in that** at least one of J, K, and M is an N atom.

4. The compound with the structure of formula I or a pharmaceutically acceptable salt, an ester, a deuterated product, an isomer, a solvate, a prodrug, or an isotopic label thereof according to claim 1, **characterized in that** I, J, and K are all N atoms, or I, M and K are all N atoms.

5. The compound with the structure of formula I or a pharmaceutically acceptable salt, an ester, a deuterated product, an isomer, a solvate, a prodrug, or an isotopic label thereof according to claim 1, **characterized in that** each of R¹, R², R³, R⁶, R⁷ and R¹⁴ is independently selected from hydrogen, deuterium, an unsubstituted or substituted C₁₋₆ alkyl group, an unsubstituted or substituted C₂₋₆ alkenyl group, an unsubstituted or substituted C₂₋₆ alkynyl group, an unsubstituted or substituted C₃₋₇ cycloalkyl group, an unsubstituted or substituted 3-7 membered heterocycloalkyl group, halogen, -OH, an unsubstituted or substituted C₁₋₆ alkoxyl group, an unsubstituted or substituted C₆₋₁₀ arylethyl group, an unsubstituted or substituted 5-10 membered heteroaryl ethyl group, an unsubstituted or substituted C₆₋₁₀ aryl ether group, an unsubstituted or substituted 5-10 membered heteroaryl ether group, -CN, -NH₂, -NO₂, -N₃, a boronic acid group, an unsubstituted or substituted boronic ester group, a carboxyl group, an ester group, an unsubstituted or substituted carbamoyl group, an unsubstituted or substituted C₆₋₁₀ aryl group, an unsubstituted or substituted 5-10 membered heteroaryl group, an unsubstituted or substituted thioether group, an unsubstituted or substituted sulfoxide group, an unsubstituted or substituted sulfone group, an unsubstituted or substituted sulfonamide group, 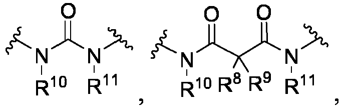 an unsubstituted or substituted phosphate ester group, the substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

6. The compound with the structure of formula I or a pharmaceutically acceptable salt, an ester, a deuterated product, an isomer, a solvate, a prodrug, or an isotopic label thereof according to claim 1, **characterized in that** W is selected from the direct bond, an unsubstituted or substituted aryl group, a heteroaryl group, a cycloalkyl group, a heterocycloalkyl group, a bridged cycloalkyl group, a bridged heterocycloalkyl group, a spiro cycloalkyl group, a spiro heterocycloalkyl group, an alkyl group, a heteroalkyl group, an alkenyl group, a heteroalkenyl group, an alkynyl group, a heteroalkynyl group, -N(R¹²R¹³), an aminoalkyl group, an aminoalkylamino group, an unsubstituted or substituted 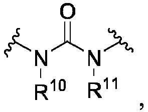 an unsubstituted or substituted 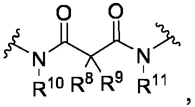 the substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

7. The compound with the structure of formula I or a pharmaceutically acceptable salt, an ester, a deuterated product, an isomer, a solvate, a prodrug, or an isotopic label thereof according to claim 6, **characterized in that** W is selected from an unsubstituted or substituted 5-7 membered heterocycloalkyl group, an unsubstituted or substituted amino-C₁₋₆ alkyl group, the substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

8. The compound with the structure of formula I or a pharmaceutically acceptable salt, an ester, a deuterated product, an isomer, a solvate, a prodrug, or an isotopic label thereof according to claim 7, **characterized in that** an atom in W connected to the ring containing J and K is N.

9. The compound with the structure of formula I or a pharmaceutically acceptable salt, an ester, a deuterated product, an isomer, a solvate, a prodrug, or an isotopic label thereof according to claim 7, **characterized in that** W is selected from a substituted or unsubstituted 5-7 membered heterocycloalkyl group, the 5-7 membered heterocycloalkyl group contains at least one nitrogen atom, preferably, the 5-7 membered heterocycloalkyl group is piperidinyl or piperazinyl.

10. The compound with the structure of formula I or a pharmaceutically acceptable salt, an ester, a deuterated product, an isomer, a solvate, a prodrug, or an isotopic label thereof according to claim 1, **characterized in that** Q is -H, -NH₂, -OH, -C₁₋₆ alkyl-HNC(=O)H, an unsubstituted or substituted C₁₋₆ alkylhydroxy group, an unsubstituted or substituted C₂₋₆ alkenylhydroxy group, an unsubstituted or substituted C₂₋₆ alkynylhydroxy group, an unsubstituted or substituted alkylamino group, a sulfonamide group, and a sulfonyl hydrazine group, the substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

11. The compound with the structure of formula I or a pharmaceutically acceptable salt, an ester, a deuterated product, an isomer, a solvate, a prodrug, or an isotopic label thereof according to claim 1, **characterized in that** W and Q can be connected or fused to form a ring, and the ring is a substituted or unsubstituted 5-7 membered cycloalkyl group, a substituted or unsubstituted 5-7 membered heterocycloalkyl group, a substituted or unsubstituted C₆₋₁₀ aryl group, and a substituted or unsubstituted 5-10 membered heteroaryl group.

12. The compound with the structure of formula I or a pharmaceutically acceptable salt, an ester, a deuterated product, an isomer, a solvate, a prodrug, or an isotopic label thereof according to claim 11, **characterized in that** the substitution is replaced by a substituent selected from hydrogen, deuterium, halogen, a C₁₋₆ alkyl group, a C₂₋₆ alkenyl group, a C₂₋₆ alkynyl group, a C₁₋₆ haloalkyl group, a C₁₋₆ haloalkenyl group, a C₁₋₆ haloalkynyl group, a C₃₋₇ cycloalkyl group, a 3-7 membered heterocycloalkyl group, halogen, -OH, a C₁₋₆ alkoxyl group, a C₁₋₆ haloalkoxyl group, -CN, -NH₂, -NO₂, N₃, a boronic acid group, a carboxyl group, an ester group, a formamide group, a C₁₋₆ alkylamide group, a C₆₋₁₀ aryl group, a 5-10 membered heteroaryl group, and an alkylamino group.

13. The compound with the structure of formula I or a pharmaceutically acceptable salt, an ester, a deuterated product, an isomer, a solvate, a prodrug, or an isotopic label thereof according to claim 1, **characterized in that** the compound with the structure of formula I is: 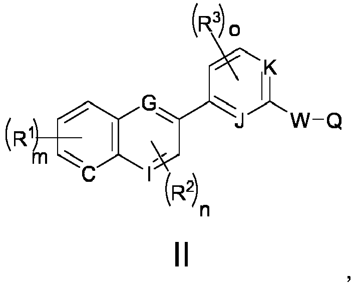 wherein, C, G, I, J, and K are independently selected from CR⁶ and N, and at least one of C, G, and I is N, and at least one of J and K is N; the definitions of R¹, R², R³, R⁶, o, m, n, W and Q are the same as those of claim 1.

14. The compound with the structure of formula I or a pharmaceutically acceptable salt, an ester, a deuterated product, an isomer, a solvate, a prodrug, or an isotopic label thereof according to claim 1, **characterized in that** the compound with the structure of formula I is: 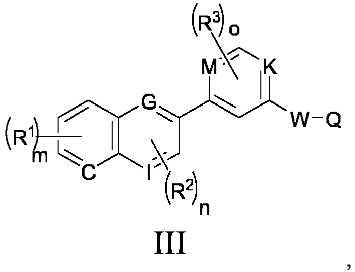 wherein, C, G, I, K, and M are independently selected from CR⁶ and N, and at least one of C, G, and I is N, and at least one of K and M is N; the definitions of R¹, R², R³, R⁶, o, m, n, W and Q are the same as those of claim 1.

15. A compound or a pharmaceutically acceptable salt, an ester, an isomer, a solvate, a prodrug, or an isotopic label thereof, **characterized in that** the compound is selected from:

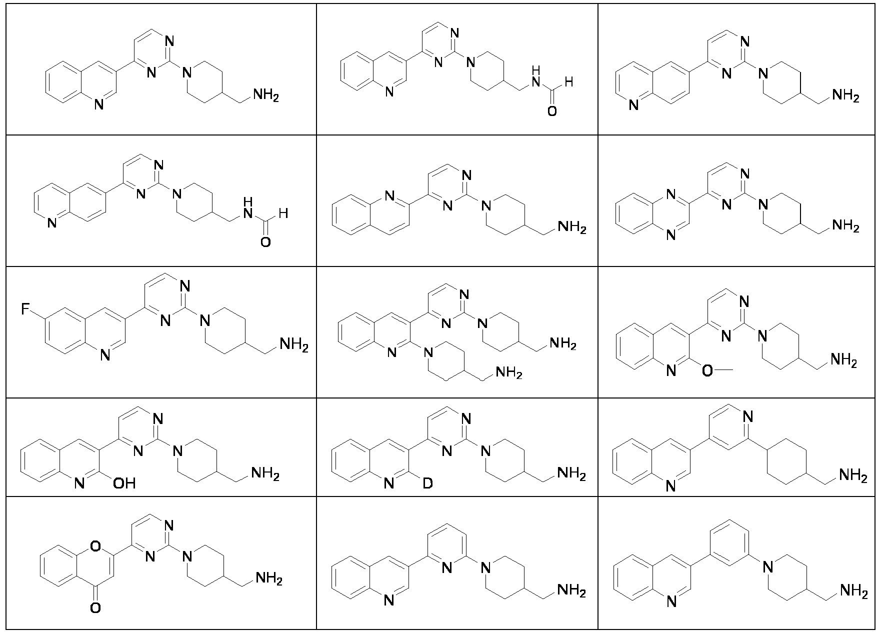

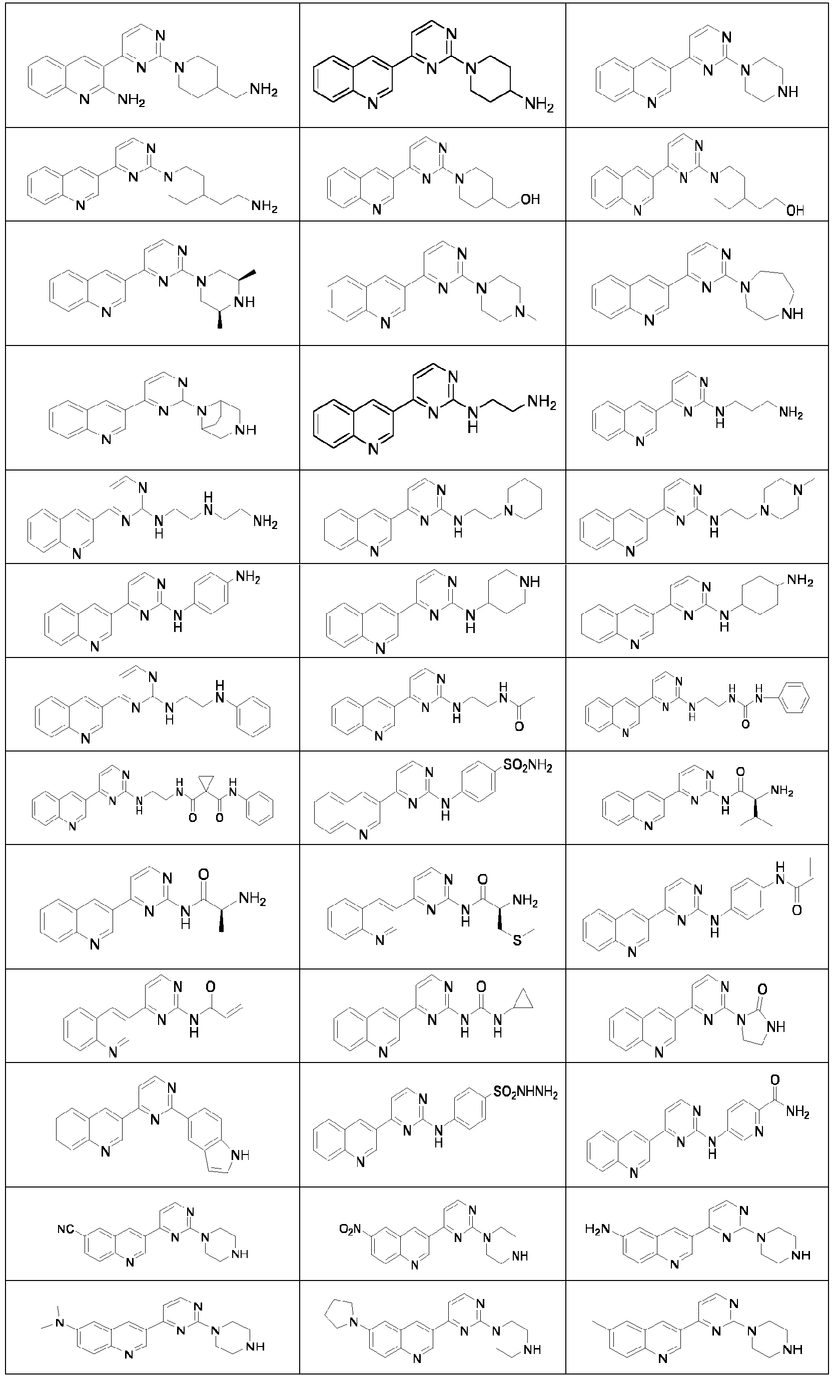

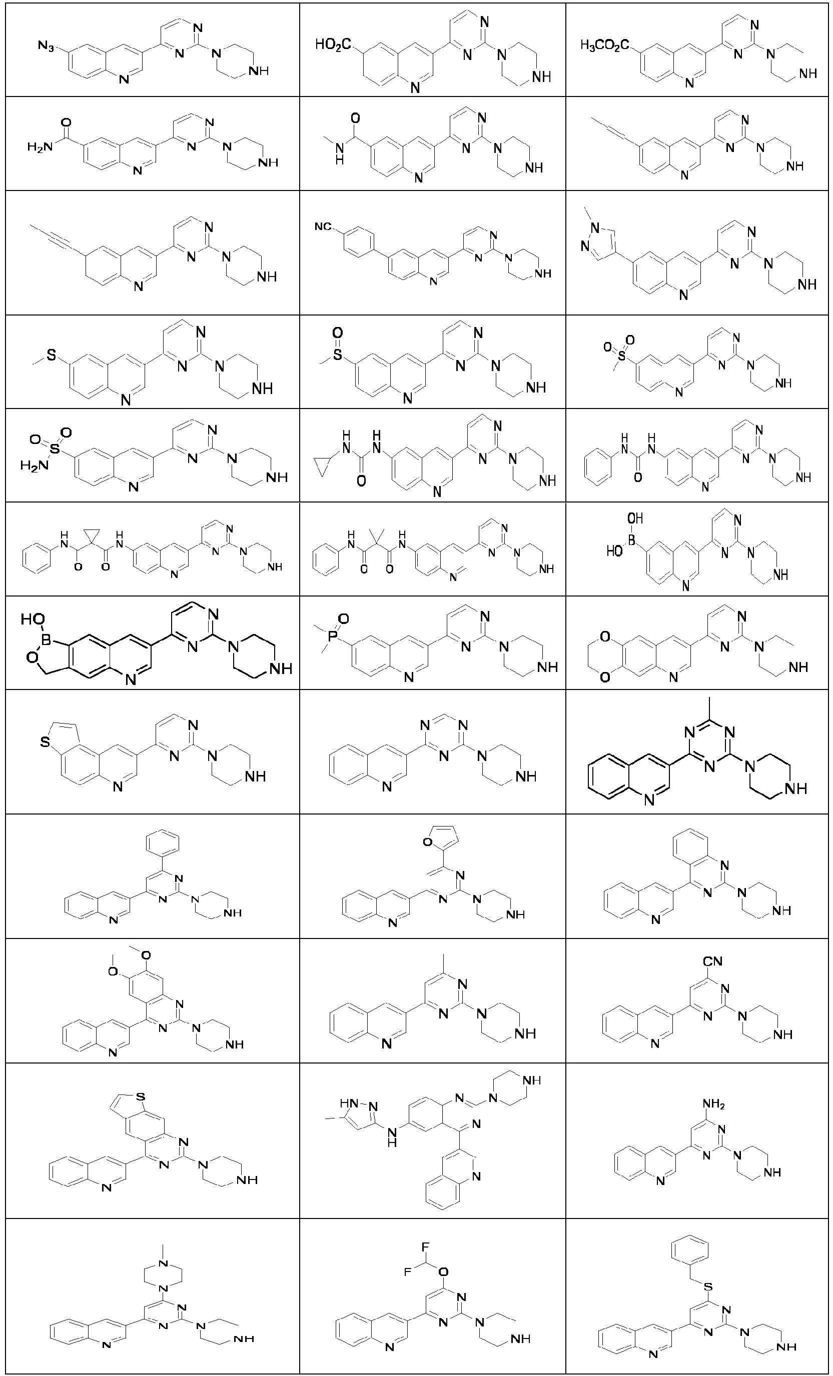

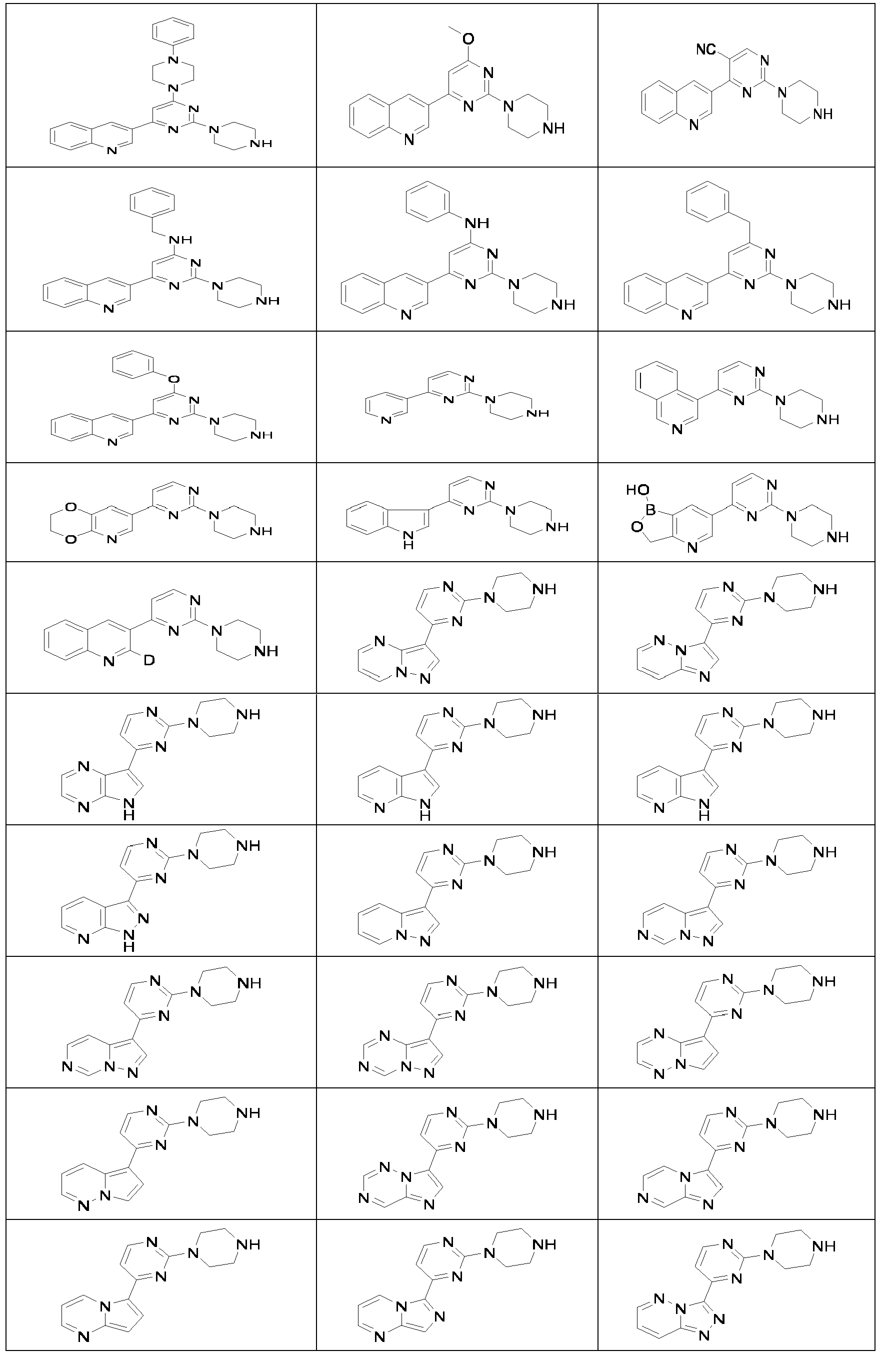

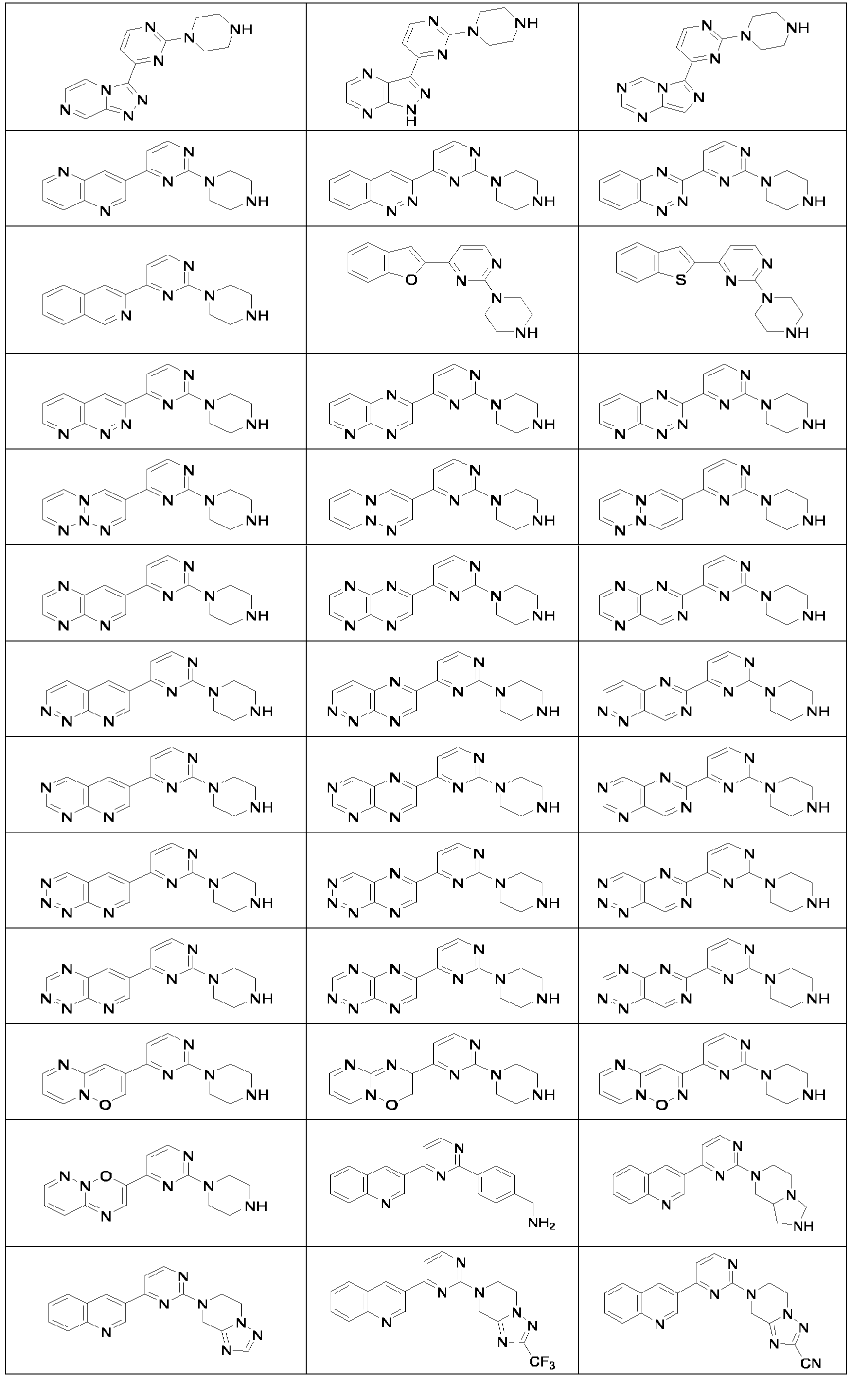

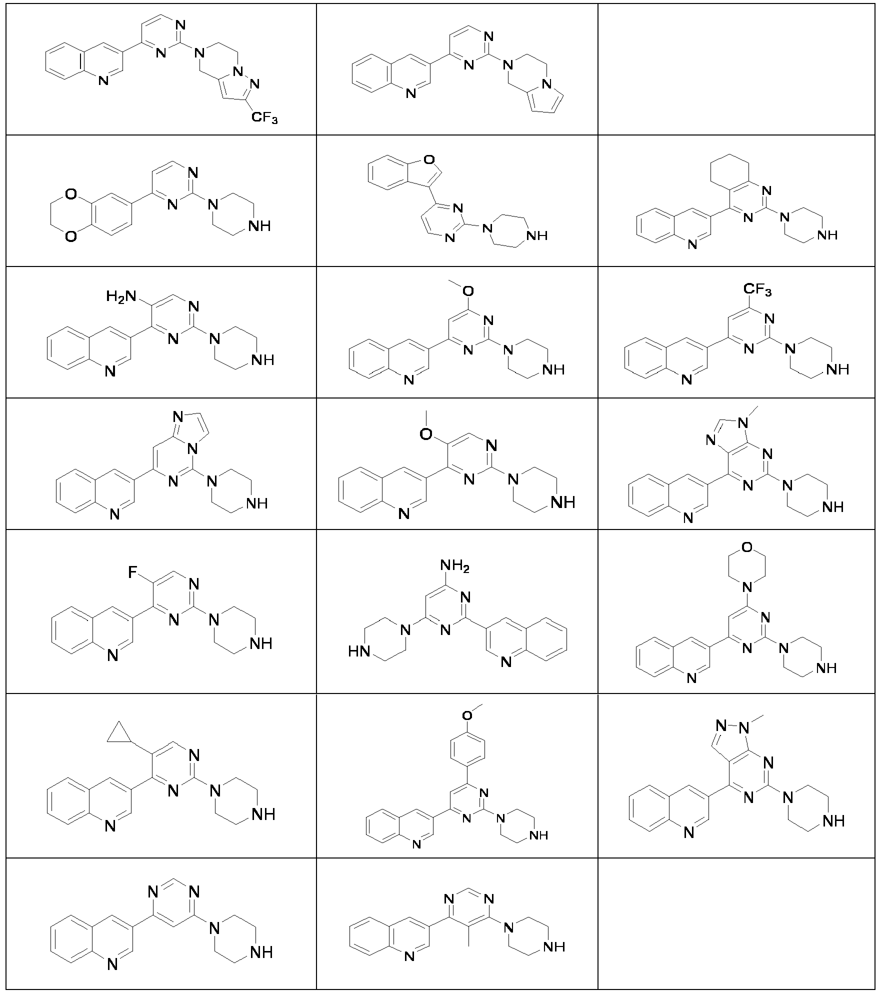

16. A pharmaceutical composition, comprising the compound or a pharmaceutically acceptable salt, an ester, a deuterated product, an isomer, a solvate, a prodrug, or an isotopic label thereof according to any one of claims 1-15, and pharmaceutically acceptable excipients.

17. The pharmaceutical composition according to claim 16, wherein the form of the pharmaceutical composition is any one of aqueous dispersions, liquid, gels, syrups, elixirs, slurries, suspensions, sprays, controlled-release formulations, instantizing agents, effervescing agents, lyophilized agents, tablets, powders, pills, dragees, capsules, relayed release preparations, sustained-release dosages, pulsed release tablets, microgranules, or immediate release agents.

18. Application of the compound or a pharmaceutically acceptable salt, an ester, an isomer, a solvate, a prodrug, or an isotopic label thereof according to any one of claims 1-15, or the pharmaceutical composition according to any one of claims 16-17 in the preparation of drugs for treating CTLA-4 related diseases.

19. The application according to claim 18, **characterized in that** the CTLA-4 related diseases include cancers, autoimmune diseases, immunodeficiency diseases, viral infections, and organ transplant rejections.

20. The application according to claim 19, **characterized in that** the cancer is selected from skin cancer, bladder cancer, breast cancer, pancreatic cancer, bone cancer, brain cancer, neurocytoma, esophageal cancer, labial cancer, laryngeal carcinoma, hypopharynx carcinoma, tongue carcinoma, salivary gland cancer, adenocarcinoma, medullary thyroid cancer, papillary thyroid cancer, choriocarcinoma, pancreatic cancer, urinary cancer, brain tumors such as glioblastoma, astrocytoma, meningioma, medulloblastoma and peripheral neuroectodermal tumors, Hodgkin's lymphoma, non-Hodgkin's lymphoma, Burkitt's lymphoma, adult T-cell leukemia lymphoma, diffuse large B-cell lymphoma (DLBCL), gallbladder cancer, bronchogenic carcinoma, multiple myeloma, basal cell tumor, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing's sarcoma or plasmacytoma, papilloma, blasto glioma, sarcoma (including but not limited to chondrosarcoma, histosarcoma, malignant fibrous histiocytoma, lymphosarcoma and rhabdomyosarcoma), melanoma, hemangioma, keloid, squamous cell carcinoma, astrocytoma, lymphoma (including but not limited to non-Hodgkin's lymphoma, AIDS related lymphoma, cutaneous T-cell lymphoma, Hodgkin's disease, and central nervous system lymphoma), respiratory cancer (including but not limited to lung cancer, such as small cell and non-small cell lung cancer, as well as bronchial adenoma and pleuropulmonary blastoma), head and neck cancer (including but not limited to head cancer, neck cancer, laryngeal carcinoma, hypopharyngeal cancer, nasopharyngeal cancer and/or oropharyngeal cancer, lip and oral cancer), bladder cancer, breast cancer (including but not limited to invasive ductal carcinoma, invasive lobular carcinoma, ductal carcinoma in situ and lobular carcinoma in situ), tumors of the digestive tract (including but not limited to anal cancer, colon cancer, colorectal cancer, esophageal cancer, gallbladder cancer, rectal cancer, gastric cancer, small bowel cancer and salivary gland cancer), thyroid cancer, parathyroid cancer and its remote metastasis, pancreatic cancer, liver cancer (including but not limited to hepatocellular carcinoma, hepatocellular carcinoma with or without fibrolamellar form, cholangiocarcinoma, and mixed hepatocellular cholangiocarcinoma), leukemia (including but not limited to acute lymphoblastic leukemia, chronic lymphoblastic leukemia, acute myeloid leukemia, chronic myeloid leukemia, and villous cell leukemia), brain cancer (including but not limited to brain stem and pituitary glioma, medulloblastoma, cerebellar and cerebral astrocytoma, ependymoma, and neuroectodermal tumor and pineal adenoma), reproductive organ cancer (including but not limited to prostate cancer, testicular cancer, ovarian cancer, endometrial cancer, cervical cancer, endometrial cancer, vaginal cancer and vulval cancer, and uterine sarcoma), urethral cancer, eye cancer (including but not limited to intraocular melanoma and retinoblastoma), skin cancer (including but not limited to Kaposi's sarcoma, squamous cell tumor, malignant melanoma, Merkel cell skin cancer and non-melanoma skin cancer), renal parenchymal carcinoma, renal carcinoma (also known as renal cell cancer and renal adenocarcinoma) and other related cancers.
